# EUROPEAN PATENT APPLICATION

(11) **EP 4 592 319 A1**
(43) Date of publication of application: **30.07.2025**
(21) Application number: 25153913.6
(22) Date of filing: 24.01.2025
(51) Int. Cl.: C07K 16/28, A61K 39/395

(54) **ANTIBODIES CAPABLE OF SPECIFICALLY BINDING TNF-LIKE LIGAND 1A AND USE THEREOF**

(30) Priority: 26.01.2024 CN 202410115198
(71) Applicant: Futuregen Biopharmaceutical (Beijing) Co., Ltd., Beijing 102629 (CN)
(72) Inventor: LI, Yun, Beijing, 102629 (CN); JIN, Zhaoyu, Beijing, 102629 (CN); YANG, Yaping, Beijing, 102629 (CN); LI, Yue, Beijing, 102629 (CN); LI, Feng, Beijing, 102629 (CN); ZHANG, Lina, Beijing, 102629 (CN); ZHAO, An, Beijing, 102629 (CN); HUO, Naifan, Beijing, 102629 (CN); CAO, An, Beijing, 102629 (CN)
(74) Representative: Sagittarius IP

(57) **Abstract**

The present disclosure relates to antibodies that specifically binding to TL1A and uses thereof, in particular to antibodies that specifically bind to TL1A or antigen-binding fragments thereof, nucleic acids encoding the antibodies or antigen-binding fragments thereof, vectors containing the nucleic acids, and host cells containing the nucleic acids or the vectors. Pharmaceutical compositions comprising the antibodies as well as methods of treatment using the antibodies are also provided.

## Description

### REFERENCE TO RELATED APPLICATIONS

This application claims the benfit of the filing date and priority to the Chinese Patent Application No. CN202410115198.1, filed on January 26, 2024, the entire contents of which, including any drawings and sequence listings, are incorporated herein by reference.

### REFERENCE TO A SEQUENCE LISTING XML

This application contains a Sequence Listing XML file which has been submitted electronically in XML format. The Sequence Listing XML file is incorporated herein by reference. Said Sequence Listing XML file, created on January 15, 2025, is named 140255-00102_SL.xml, and is 68,100 bytes in size.

### TECHNICAL FIELD

The present invention relates to the field of biotechnology, and more specifically to anti-TL1A antibodies and use thereof.

### BACKGROUND OF THE INVENTION

TL1A (TNF-like ligand 1A) is a member of the tumor necrosis factor superfamily (TNFSF), encoded by the TNFSF15 gene, and exists in two forms: membrane-bound and soluble forms. TL1A initially exists in a membrane-bound form, and is subsequently cleaved by metalloproteinases to release the soluble form of TL1A and form a trimer to function (Kokkotis, Georgios and Giorgos Bamias. Expert review of clinical immunology vol. 18,6 (2022): 551-555; Migone, Thi Sau et al. Immunity vol. 16,3 (2002): 479-92). In the resting state, TL1A is expressed at low levels in various types of cells. Under the induction of pro-inflammatory stimulations, TL1A expression levels increase sharply and are mainly expressed in endothelial cells, monocytes, dendritic cells, macrophages and some lymphocyte subsets (Richard, Arianne C et al. Journal of leukocyte biology vol. 98,3 (2015): 333-45; Prehn, John et al. Clinical immunology (Orlando, Fla.) vol. 112,1 (2004): 66-77). TL1A has two receptors, namely DR3 (Death receptor 3, TNFRSF25) and DcR3 (Decoy receptor 3, TNFRSF6B), which are members of the tumor necrosis factor receptor superfamily (TNFRSF). DR3 is expressed on T cells, NK cells and innate lymphocytes (ILC), especially activated T cells. DcR3 lacks the intracellular signal transduction structure and plays a biological regulatory role mainly by competing with the DR3 receptor for binding ligands (Zhan, Chenyang et al. Biochemistry vol. 48,32 (2009): 7636-45; Papadakis, Konstantinos A et al. Journal of immunology (Baltimore, Md.: 1950) vol. 172,11 (2004): 7002-7; Bamias, Giorgos et al. Journal of immunology (Baltimore, Md.: 1950) vol. 171,9 (2003)): 4868-74).

After TL1A binds to DR3, it can regulate a series of downstream signaling pathways including NF-κB. As an initial T cell activation cofactor, TL1A can provide co-stimulatory signals for T cell activation and proliferation, amplifying the effector responses of Th1, Th17, Th2 and other cells (Jin, S et al. Mucosal immunology vol. 6,5 (2013): 886-99; Holmkvist, P et al. Mucosal immunology vol. 8,3 (2015): 545-58; Kamada, Nobuhiko et al. Inflammatory bowel diseases vol. 16,4 (2010): 568-75; Takedatsu, Hidetoshi et al. Gastroenterology vol. 135,2 (2008): 552-67), including but not limited to increasing IL-2 and IL-2 receptor expression to upregulate IL-2 signaling; co-stimulating secretion of IFN-y and other Th1-dependent pro-inflammatory cytokines with IL-12 and IL-18; binding to DR3 on Th17 to enhance the production of IL-17; regulating the differentiation, proliferation and function of Treg cells, *etc.* In addition to co-stimulating T cells and affecting T cell-mediated inflammatory responses, TL1A can also co-stimulate innate lymphoid cells (ILCs) and play an important role in maintaining tissue homeostasis and defending against pathogens (especially in mucosal tissues). In addition, TL1A binds to DR3 on fibroblasts and can promote the expression of collagen and IL-31R, thereby promoting the occurrence and development of fibrosis. As a result, the TL1A/DR3 signaling pathway is considered to be related to a series of autoimmune diseases, inflammatory diseases, and fibrotic diseases (Kokkotis, Georgios, and Giorgos Bamias, supra).

Inflammatory bowel disease (IBD) is a chronic non-specific intestinal inflammatory disease, including ulcerative colitis (UC) and Crohn's disease (CD). Targeting TL1A is considered as a powerful strategy for the treatment of IBD and has been initially verified in clinical trials. The representative TL1A-targeting drugs currently under clinical development are TL1A monoclonal antibody RVT-3101 of Pfizer/Roivant and TL1A monoclonal antibody PRA023 of Merck/Prometheus. While the effectiveness of TL1A antibodies is encouraging, the issue of anti-drug antibodies (ADAs) raises concerns. In the phase 1 clinical trial of RVT-3101, among the 68 subjects, except for 11 subjects whose test results were inconclusive, 56/57 (98.2%) of the remaining subjects had confirmed ADA, and 24/68 (35.3%) of the subjects were NAb positive (Banfield, Christopher, et al. British journal of clinical pharmacology vol. 86,4 (2020): 812-824). RVT-3101 also showed strong immunogenicity in the phase 2a TUSCANY study in UC patients, with 82% of 50 subjects being ADA positive and 10% being NAb positive (Danese, Silvio et al. Clinical gastroenterology and hepatology: the official clinical practice journal of the American Gastroenterological Association vol. 19,11 (2021): 2324-2332.e6.). Prometheus disclosed in patent WO2022178158A1 that in the phase 1 clinical trial of PRA023, the incidence of ADA did not exceed 20% at clinically relevant doses (high doses). However, the high concentrations of PRA023 present in clinically relevant dose groups may interfere with ADA detection, and indeed, Prometheus reported that ADA positivity only occurred at low PRA023 concentrations, and that ADA titers were inversely proportional to PRA023 exposure (less drug in serum, less interference). Therefore, it is reasonable to speculate that the true ADA positive rate of PRA023 may be higher than 20%, and the incidence of ADA may increase significantly after repeated administration. In addition, current TL1A antibodies also have the problems of high dosage and high frequency of administration.

There is a need in this field to develop novel TL1A-targeting antibodies that can overcome the immunogenicity problem of current TL1A antibodies while having better biological activity and pharmacokinetic properties.

### SUMMARY OF THE INVENTION

The present invention provides specific TL1A binding epitopes and a series of novel TL1A antibodies specific for said TL1A binding epitopes. The novel TL1A antibody of the present invention binds to TL1A at said specific epitope, forms a smaller immune complex with TL1A, is not easily recognized by the body, has lower immunogenicity; has better biological activity; and/or has pH-dependent TL1A binding properties, which can reduce the level of TL1A in the body by accelerating the clearance of TL1A in lysosomes; in addition, by optionally changing the binding properties of the antibody and FcRn through Fc mutation, the half-life of the antibody is increased, and the frequency of administration can be reduced.

In a first aspect the invention provides an antigen-binding protein (such as an antibody) or antigen-binding fragment thereof that binds to TL1A, wherein the antigen-binding protein or antigen-binding fragment thereof binds to an epitope in TL1A comprising one or more (*e.g.,* all or substantially all) amino acid residues selected from the group consisting of: R103 , G124, M196, Q193, Y238, T239, K240, E241, H118, E120, H121, E122, L123, V102 and T105, wherein the amino acid positions are numbered according to the amino acid sequence of TL1A as set forth in SEQ ID NO: 68.

In some embodiments, the epitope comprises R103, G124, Y238, T239, E120, and V102. In a preferred embodiment, the epitope further comprises one or more (*e.g.,* all or substantially all) amino acid residues selected from the group consisting of M196, K240, E241, H118, H121, E122, L123 and T105.

In some embodiments, the epitope comprises amino acid residues R103, G124, M196, Y238, T239, K240, E241, H118, E120, H121, E122, L123, V102, and T105.

In some embodiments, the epitope consists of amino acid residues R103, G124, M196, Y238, T239, K240, E241, H118, E120, H121, E122, L123, V102, and T105.

In some embodiments, the epitope consists of amino acid residues R103, G124, M196, Y238, T239, E120, and V102.

In some embodiments, the epitope consists of amino acid residues G124, M196, Y238, T239, and E120.

In some embodiments of the TL1A-binding antigen-binding protein (such as an antibody) or antigen-binding fragment thereof disclosed herein, the antigen-binding protein comprises a heavy chain variable region (VH) and a light chain variable region (VL), wherein
(1) The VH comprises heavy chain CDR1, heavy chain CDR2 and heavy chain CDR3 of the heavy chain variable region having the amino acid sequence as set forth in SEQ ID NO: 2, and the VL comprises light chain CDR1, light chain CDR2 and light chain CDR3 of the light chain variable region having the amino acid sequence as set forth in SEQ ID NO: 1;
(2) The VH comprises heavy chain CDR1, heavy chain CDR2 and heavy chain CDR3 of the heavy chain variable region having the amino acid sequence as set forth in SEQ ID NO: 4, and the VL comprises light chain CDR1, light chain CDR2 and light chain CDR3 of the light chain variable region having the amino acid sequence as set forth in SEQ ID NO: 3;
(3) The VH comprises heavy chain CDR1, heavy chain CDR2 and heavy chain CDR3 of the heavy chain variable region having the amino acid sequence as set forth in SEQ ID NO: 6, and the VL comprises light chain CDR1, light chain CDR2 and light chain CDR3 of the light chain variable region having the amino acid sequence as set forth in SEQ ID NO: 5;
(4) The VH comprises heavy chain CDR1, heavy chain CDR2 and heavy chain CDR3 of the heavy chain variable region having the amino acid sequence as set forth in SEQ ID NO: 8, and the VL comprises light chain CDR1, light chain CDR2 and light chain CDR3 of the light chain variable region having the amino acid sequence as set forth in SEQ ID NO: 7;
(5) The VH comprises heavy chain CDR1, heavy chain CDR2 and heavy chain CDR3 of the heavy chain variable region having the amino acid sequence as set forth in SEQ ID NO: 10, and the VL comprises light chain CDR1, light chain CDR2 and light chain CDR3 of the light chain variable region having the amino acid sequence as set forth in SEQ ID NO: 9; or
(6) The VH comprises heavy chain CDR1, heavy chain CDR2 and heavy chain CDR3 of the heavy chain variable region having the amino acid sequence as set forth in SEQ ID NO: 12, and the VL comprises light chain CDR1, light chain CDR2 and light chain CDR3 of the light chain variable region having the amino acid sequence as set forth in SEQ ID NO: 11.

In certain embodiments, the heavy chain variable region has the amino acid sequence as set forth in SEQ ID NO: 10, and the light chain variable region has the amino acid sequence as set forth in SEQ ID NO: 9.

In some embodiments, the VH comprises heavy chain CDRs 1-3 having the amino acid sequence as set forth in SEQ ID NO: 16-18 respectively, and the VL comprises light chain CDRs 1-3 having the amino acid sequence as set forth in SEQ ID NO: 13-15 respectively. In some embodiments, the VH comprises heavy chain CDRs 1-3 having the amino acid sequence as set forth in SEQ ID NO: 22-24 respectively, and the VL comprises light chain CDRs 1-3 having the amino acid sequence as set forth in SEQ ID NO: 19-21 respectively. In some embodiments, the VH comprises heavy chain CDRs 1-3 having the amino acid sequence as set forth in SEQ ID NO: 28-30 respectively, and the VL comprises light chain CDRs 1-3 having the amino acid sequence as set forth in SEQ ID NO: 25-27 respectively. In some embodiments, the VH comprises heavy chain CDRs 1-3 having the amino acid sequence as set forth in SEQ ID NO: 34-36 respectively, and the VL comprises light chain CDRs 1-3 having the amino acid sequence as set forth in SEQ ID NO: 31-33 respectively. In some embodiments, the VH comprises heavy chain CDRs 1-3 having the amino acid sequence as set forth in SEQ ID NO: 40-42 respectively, and the VL comprises light chain CDRs 1-3 having the amino acid sequence as set forth in SEQ ID NO: 37-39 respectively. In some embodiments, the VH comprises heavy chain CDRs 1-3 having the amino acid sequence as set forth in SEQ ID NO: 46-48 respectively, and the VL comprises light chain CDRs 1-3 having the amino acid sequence as set forth in SEQ ID NO: 43-45 respectively.

In certain embodiments, the VH CDRs 1-3 have the amino acid sequences as set forth in SEQ ID NO: 40-42, respectively, and the VL CDRs have the amino acid sequences as set forth in SEQ ID NO: 37-39, respectively.

In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 2 and the VL comprises an amino acid sequence having at least 80%, at least 85%, at least 90%, at least 95%, at least 96%, at least 97%, at least 98%, at least 99%, or 100% sequence identity to SEQ ID NO: 1. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 4 and the VL comprises an amino acid sequence having at least 80%, at least 85%, at least 90%, at least 95%, at least 96%, at least 97%, at least 98%, at least 99%, or 100% sequence identity to SEQ ID NO: 3. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 6 and the VL comprises an amino acid sequence having at least 80%, at least 85%, at least 90%, at least 95%, at least 96%, at least 97%, at least 98%, at least 99%, or 100% sequence identity to SEQ ID NO: 5. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 8 and the VL comprises an amino acid sequence having at least 80%, at least 85%, at least 90%, at least 95%, at least 96%, at least 97%, at least 98%, at least 99%, or 100% sequence identity to SEQ ID NO: 7. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 10 and the VL comprises an amino acid sequence having at least 80%, at least 85%, at least 90%, at least 95%, at least 96%, at least 97%, at least 98%, at least 99%, or 100% sequence identity to SEQ ID NO: 9. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 12 and the VL comprises an amino acid sequence having at least 80%, at least 85%, at least 90%, at least 95%, at least 96%, at least 97%, at least 98%, at least 99%, or 100% sequence identity to SEQ ID NO: 11.

In certain embodiments, the VH comprises an amino acid sequence having at least 80%, at least 85%, at least 90%, at least 95%, at least 96%, at least 97%, at least 98%, at least 99%, or 100% sequence identity to SEQ ID NO: 10, and the VL comprises an amino acid sequence having at least 80%, at least 85%, at least 90%, at least 95%, at least 96%, at least 97%, at least 98%, at least 99%, or 100% sequence identity to SEQ ID NO: 9.

In some embodiments, the VH comprises an amino acid sequence as set forth in SEQ ID NO: 2 and the VL comprises an amino acid sequence as set forth in SEQ ID NO: 1. In some embodiments, the VH comprises an amino acid sequence as set forth in SEQ ID NO: 4 and the VL comprises an amino acid sequence as set forth in SEQ ID NO: 3. In some embodiments, the VH comprises an amino acid sequence as set forth in SEQ ID NO: 6 and the VL comprises an amino acid sequence as set forth in SEQ ID NO: 5. In some embodiments, the VH comprises an amino acid sequence as set forth in SEQ ID NO: 8 and the VL comprises an amino acid sequence as set forth in SEQ ID NO: 7. In some embodiments, the VH comprises an amino acid sequence as set forth in SEQ ID NO: 10 and the VL comprises an amino acid sequence as set forth in SEQ ID NO: 9. In some embodiments, the VH comprises an amino acid sequence as set forth in SEQ ID NO: 12 and the VL comprises an amino acid sequence as set forth in SEQ ID NO: 11.

In certain embodiments, the VH comprises an amino acid sequence as set forth in SEQ ID NO: 10, and the VL comprises an amino acid sequence as set forth in SEQ ID NO: 9.

In some embodiments of the presently disclosed antigen-binding protein or antigen-binding fragment thereof that binds to TL1A, the antigen-binding protein is an antibody.

In some embodiments, the antibody is a monoclonal antibody, a bispecific antibody, or a multispecific antibody.

In some embodiments, the antibody is selected from the group consisting of a murine antibody, a chimeric antibody, a humanized antibody, and a fully human antibody.

In some embodiments, the antibody comprises an Fc region.

In some embodiments, the antibody is of an isotype selected from IgG, IgA, IgM, IgE, and IgD.

In some embodiments, the antibody is of a subtype selected from IgG1, IgG2, IgG3, and IgG4.

In some embodiments of the presently disclosed antigen-binding protein or antigen-binding fragment thereof that binds to TL1A, the Fc region of the antibody contains a modification capable of increasing the binding to FcRn.

In some embodiments, the antibody comprises a heavy chain (HC) and a light chain (LC), wherein:
(1) The heavy chain comprises an amino acid sequence having at least 80%, at least 85%, at least 90%, at least 95%, at least 96%, at least 97%, at least 98%, at least 99%, or 100% sequence identity to SEQ ID NO: 50 and the light chain comprises an amino acid sequence having at least 80%, at least 85%, at least 90%, at least 95%, at least 96%, at least 97%, at least 98%, at least 99%, or 100% sequence identity to SEQ ID NO: 49;
(2) The heavy chain comprises an amino acid sequence having at least 80%, at least 85%, at least 90%, at least 95%, at least 96%, at least 97%, at least 98%, at least 99%, or 100% sequence identity to SEQ ID NO: 52 and the light chain comprises an amino acid sequence having at least 80%, at least 85%, at least 90%, at least 95%, at least 96%, at least 97%, at least 98%, at least 99%, or 100% sequence identity to SEQ ID NO: 51;
(3) The heavy chain comprises an amino acid sequence having at least 80%, at least 85%, at least 90%, at least 95%, at least 96%, at least 97%, at least 98%, at least 99%, or 100% sequence identity to SEQ ID NO: 54 and the light chain comprises an amino acid sequence having at least 80%, at least 85%, at least 90%, at least 95%, at least 96%, at least 97%, at least 98%, at least 99%, or 100% sequence identity to SEQ ID NO: 53;
(4) The heavy chain comprises an amino acid sequence having at least 80%, at least 85%, at least 90%, at least 95%, at least 96%, at least 97%, at least 98%, at least 99%, or 100% sequence identity to SEQ ID NO: 56 and the light chain comprises an amino acid sequence having at least 80%, at least 85%, at least 90%, at least 95%, at least 96%, at least 97%, at least 98%, at least 99%, or 100% sequence identity to SEQ ID NO: 55;
(5) The heavy chain comprises an amino acid sequence having at least 80%, at least 85%, at least 90%, at least 95%, at least 96%, at least 97%, at least 98%, at least 99%, or 100% sequence identity to SEQ ID NO: 58 and the light chain comprises an amino acid sequence having at least 80%, at least 85%, at least 90%, at least 95%, at least 96%, at least 97%, at least 98%, at least 99%, or 100% sequence identity to SEQ ID NO: 57; or
(6) The heavy chain comprises an amino acid sequence having at least 80%, at least 85%, at least 90%, at least 95%, at least 96%, at least 97%, at least 98%, at least 99%, or 100% sequence identity to SEQ ID NO: 60 and the light chain comprises an amino acid sequence having at least 80%, at least 85%, at least 90%, at least 95%, at least 96%, at least 97%, at least 98%, at least 99%, or 100% sequence identity to SEQ ID NO: 59.

In certain embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 58, and the light chain comprises an amino acid sequence having at least 80%, at least 85%, at least 90%, at least 95%, at least 96%, at least 97%, at least 98%, at least 99%, or 100% sequence identity to SEQ ID NO: 57.

In some embodiments, the heavy chain comprises an amino acid sequence as set forth in SEQ ID NO: 50 and the light chain comprises an amino acid sequence as set forth in SEQ ID NO: 49. In some embodiments, the heavy chain comprises an amino acid sequence as set forth in SEQ ID NO: 52 and the light chain comprises an amino acid sequence as set forth in SEQ ID NO: 51. In some embodiments, the heavy chain comprises an amino acid sequence as set forth in SEQ ID NO: 54 and the light chain comprises an amino acid sequence as set forth in SEQ ID NO: 53. In some embodiments, the heavy chain comprises an amino acid sequence as set forth in SEQ ID NO: 56 and the light chain comprises an amino acid sequence as set forth in SEQ ID NO: 55. In some embodiments, the heavy chain comprises an amino acid sequence as set forth in SEQ ID NO: 58 and the light chain comprises an amino acid sequence as set forth in SEQ ID NO: 57. In some embodiments, the heavy chain comprises an amino acid sequence as set forth in SEQ ID NO: 60 and the light chain comprises an amino acid sequence as set forth in SEQ ID NO: 59.

In certain embodiments, the heavy chain comprises an amino acid sequence as set forth in SEQ ID NO: 58, and the light chain comprises an amino acid sequence as set forth in SEQ ID NO: 57.

In some embodiments of the presently disclosed antigen-binding protein or antigen-binding fragment thereof that binds to TL1A, the antigen-binding fragment is selected from the group consisting of Fab, Fab', F(ab')₂, Fv, scFv, and ds-scFv.

In some embodiments of the presently disclosed antigen-binding protein or antigen-binding fragment thereof that binds to TL1A, compared to an anti-TL1A antibody that binds to other epitopes than said epitope in TL1A, the antibody forms a complex with a smaller size when binding to TL1A. In some embodiments, the particle size of the complex formed by the antibody and TL1A is less than 33 nm. In a preferred embodiment, the particle size of the complex formed by the antibody and TL1A is less than 30 nm. In a preferred embodiment, the particle size of the complex formed by the antibody and TL1A is less than 25 nm.

In some embodiments of the presently disclosed antigen-binding protein or antigen-binding fragment thereof that binds to TL1A, the antibody binds to TL1A in a 1:1 ratio to form an antigen-antibody complex. In some embodiments, the Fc valence of the complex formed by the antibody and TL1A is <5. In a preferred embodiment, the Fc valence of the complex formed by the antibody and TL1A is 2.

In some embodiments of the presently disclosed antigen-binding protein or antigen-binding fragment thereof that binds to TL1A, the binding of the antibody to TL1A under neutral pH conditions is stronger than the binding to TL1A under acidic conditions. In a preferred embodiment, the bingding of the antibody to TL1A at pH 7.4 is stronger than the binding to TL1A at pH ≤ 6.0 (*e.g.,* pH 6.0, 5.8, 5.6). In some embodiments of the presently disclosed antigen-binding protein or antigen-binding fragment thereof that binds to TL1A, the antibody has a binding affinity constant (KD) of 10⁻⁹M to TL1A at pH 7.4 and does not bind to TL1A at pH 5.6.

In a related additional first aspect of the invention, the invention provides an isolated antibody or an antigen-binding fragment thereof that specifically binds to TL1A, wherein the isolated antibody comprises an immunoglobulin heavy chain (HC) comprising a heavy chain variable region (VH) comprising a heavy chain CDR1, a heavy chain CDR2, and a heavy chain CDR3 (VH CDRs 1-3), and an immunoglobulin light chain (LC) comprising a variable region (VL) comprising a light chain CDR1, a light chain CDR2, and a light chain CDR3 (VL CDRs 1-3), wherein the VH CDRs 1-3 have the amino acid sequences as set forth in SEQ ID NO: 40-42, respectively, and the VL CDRs 1-3 have the amino acid sequences as set forth in SEQ ID NO: 37-39, respectively.

In certain embodiments, the VH comprises an amino acid sequence having at least 80%, at least 85%, at least 90%, at least 95%, at least 96%, at least 97%, at least 98%, at least 99%, or 100% amino acid sequence identity to SEQ ID NO: 10, and the VL comprises an amino acid sequence having at least 80%, at least 85%, at least 90%, at least 95%, at least 96%, at least 97%, at least 98%, at least 99%, or 100% amino acid sequence identity to SEQ ID NO: 9.

In certain embodiments, the VH comprises an amino acid sequence as set forth in SEQ ID NO: 10, and the VL comprises an amino acid sequence as set forth in SEQ ID NO: 9.

In certain embodiments, the heavy chain comprises an amino acid sequence having at least 80%, at least 85%, at least 90%, at least 95%, at least 96%, at least 97%, at least 98%, at least 99%, or 100% amino acid sequence identity to SEQ ID NO: 58, and the light chain comprises an amino acid sequence having at least 80%, at least 85%, at least 90%, at least 95%, at least 96%, at least 97%, at least 98%, at least 99%, or 100% amino acid sequence identity to SEQ ID NO: 57.

In certain embodiments, the heavy chain comprises an amino acid sequence set forth in SEQ ID NO: 58, and the light chain comprises an amino acid sequence set forth in SEQ ID NO: 57.

In certain embodiments, the isolated antibody is a monoclonal antibody, a bispecific antibody or a multispecific antibody.

In certain embodiments, the isolated antibody is a murine antibody, a chimeric antibody, a humanized antibody, or a fully human antibody.

In certain embodiments, the isolated antibody or antigen-binding fragment thereof binds to an epitope in TL1A, wherein the epitope comprises one or more amino acid residues selected from the group consisting of: R103 , G124, M196, Q193, Y238, T239, K240, E241, H118, E120, H121, E122, L123, V102 and T105, and wherein the amino acid positions are numbered according to the amino acid sequence of TL1A as set forth in SEQ ID NO: 68.

In certain embodiments, the epitope consists of amino acid residues R103, G124, M196, Y238, T239, K240, E241, H118, E120, H121, E122, L123, V102 and T105.

In certain embodiments, the epitope consists of amino acid residues R103, G124, M196, Y238, T239, E120, and V102.

In certain embodiments, the epitope consists of amino acid residues G124, M196, Y238, T239, and E120.

In certain embodiments, the isolated antibody comprises an Fc region.

In certain embodiments, the antibody is of an isotype selected from the group consisting of IgG, IgA, IgM, IgE and IgD.

In certain embodiments, the antibody is of a subtype selected from the group consisting of IgG1, IgG2, IgG3 and IgG4.

In certain embodiments, the Fc region comprises a modification capable of increasing the binding to FcRn.

In certain embodiments, the antigen-binding fragment is selected from the group consisting of Fab, Fab', F(ab')2, Fv, scFv and ds-scFv.

In certain embodiments, when compared to a control anti-TL1A antibody that binds to an epitope other than said epitope in TL1A to form a control complex, said isolated antibody binds to TL1A to form a complex smaller in size compared to the control complex.

In certain embodiments, the size of the complex formed by the isolated antibody and TL1A is less than 33 nm, preferably less than 30 nm, more preferably less than 25 nm.

In certain embodiments, the antibody binds to TL1A at a 1:1 molar ratio to form an antigen-antibody complex.

In certain embodiments, the Fc valence of the complex formed by the antibody and TL1A is <5, preferably 2.

In certain embodiments, the binding of the antibody to TL1A under neutral pH conditions is stronger than the binding to TL1A under acidic conditions, preferably, the bingding of the antibody to TL1A at pH 7.4 is stronger than the binding to TL1A at pH ≤ 6.0 (*e.g.,* pH 6.0, 5.8, 5.6).

In certain embodiments, the antibody has a binding affinity constant (KD) of 10⁻⁹M to TL1A at pH 7.4 and does not bind to TL1A at pH 5.6.

In a second aspect, the invention provides a nucleic acid encoding the antigen-binding protein (such as an antibody) or the antigen-binding fragment thereof according to the first aspect of the invention, including the related first aspect of the invention.

In a third aspect, the invention provides a vector comprising the nucleic acid according to the second aspect of the invention.

In a fourth aspect, the invention provides a host cell comprising the nucleic acid according to the second aspect of the invention or the vector according to the third aspect of the invention.

In a fifth aspect, the invention provides a method for preparing the antigen-binding protein (such as an antibody) or antigen-binding fragment thereof according to the first aspect of the invention, including the related first aspect of the invention, the method comprising:
a) culturing the host cell according to the fourth aspect of the invention under conditions suitable for expression of the antigen-binding protein (such as an antibody) or antigen-binding fragment thereof; and
b) isolating the antigen-binding protein (such as an antibody) or antigen-binding fragment thereof from the culture and/or culture supernatant of the host cell.

In a sixth aspect, the invention provides a pharmaceutical composition comprising antibody or an antigen-binding fragment thereof according to the first aspect of the invention (including the related first aspect of the invention), the nucleic acid according to the second aspect of the invention, the vector according to the third aspect of the invention, or the host cell according to the fourth aspect of the invention, and optionally a pharmaceutically acceptable carrier or excipient.

In some embodiments, the pharmaceutical composition further comprises a second therapeutic agent, optionally the second therapeutic agent is selected from the group consisting of an antibody, a chemotherapeutic agent, a radiotherapeutic agent, an antisense oligonucleotide (such as siRNA), a peptide and a small molecule drug.

In a seventh aspect, the invention provides a method for treating a disease in a subject, comprising administering to the subject an effective amount of the antibody or the antigen-binding fragment thereof according to the first aspect of the invention (including the related first aspect of the invention), the nucleic acid according to the second aspect of the invention, the vector according to the third aspect of the invention, the host cell according to the fourth aspect of the invention, or the pharmaceutical composition according to the sixth aspect of the invention.

In some embodiments, the disease is selected from an autoimmune disease, an inflammatory disease, and a fibrotic diseases.

In some embodiments, the disease is selected from the group consisting of inflammatory bowel disease (*e.g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

In some embodiments, the method further comprises a second therapeutic agent, optionally the second therapeutic agent is selected from the group consisting of an antibody, a chemotherapeutic agent, a radiotherapeutic agent, an antisense oligonucleotide (such as siRNA), a peptide and a small molecule drug.

In an eighth aspect, the invention provides use of the antibody or the antigen-binding fragment thereof according to the first aspect of the invention (including the related first aspect of the invention), the nucleic acid according to the second aspect of the invention, the vector according to the third aspect of the invention, the host cell according to the fourth aspect of the invention, or the pharmaceutical composition according to the sixth aspect of the invention in the manufacture of a medicament for treating a disease in a subject.

In some embodiments, the disease is selected from an autoimmune disease, an inflammatory disease, and a fibrotic diseases.

In some embodiments, the disease is selected from the group consisting of inflammatory bowel disease (*e.g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

In some embodiments, the medicament is used for combined administration with a second therapeutic agent, optionally the second therapeutic agent is selected from the group consisting of an antibody, a chemotherapeutic agent, a radiotherapeutic agent, an antisense oligonucleotide (such as siRNA), a peptide and a small molecule drug.

In a ninth aspect, the invention provides the antibody or the antigen-binding fragment thereof according to the first aspect of the invention (including the related first aspect of the invention), the nucleic acid according to the second aspect of the invention, the vector according to the third aspect of the invention, the host cell according to the fourth aspect of the invention, or the pharmaceutical composition according to the sixth aspect of the invention for use in treating a disease in a subject.

In some embodiments, the disease is selected from an autoimmune disease, an inflammatory disease, and a fibrotic diseases.

In some embodiments, the disease is selected from the group consisting of inflammatory bowel disease (*e.g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

In some embodiments, the antibody or antigen-binding fragment thereof, nucleic acid, vector, host cell, conjugate or pharmaceutical composition is used for combined administration with a second therapeutic agent, optionally the second therapeutic agent is selected from the group consisting of an antibody, a chemotherapeutic agent, a radiotherapeutic agent, an antisense oligonucleotide (such as siRNA), a peptide and a small molecule drug.

The invention described herein is also described in the numbered paragraphs below
1. An antigen-binding protein or antigen-binding fragment thereof that binds to TL1A, wherein the antigen-binding protein or antigen-binding fragment thereof binds to an epitope in TL1A, the epitope comprising one or more amino acid residues selected from the group consisting of: R103 , G124, M196, Q193, Y238, T239, K240, E241, H118, E120, H121, E122, L123, V102 and T105, wherein the amino acid positions are numbered according to the amino acid sequence of TL1A as set forth in SEQ ID NO: 68.
2. The antigen-binding protein or antigen-binding fragment thereof according to paragraph 1, wherein the epitope comprises R103, G124, Y238, T239, E120, V102; preferably, the epitope further comprises one or more amino acid residues selected from M196, K240, E241, H118, H121, E122, L123 and T105.
3. The antigen-binding protein or antigen-binding fragment thereof according to paragraph 1 or 2, wherein the epitope comprises amino acid residues R103, G124, M196, Y238, T239, K240, E241, H118, E120, H121, E122, L123, V102 and T105.
4. The antigen-binding protein or antigen-binding fragment thereof according to any one of paragraphs 1-3, wherein the epitope consists of amino acid residues R103, G124, M196, Y238, T239, K240, E241, H118, E120, H121, E122, L123, V102 and T105.
5. The antigen-binding protein or antigen-binding fragment thereof according to any one of paragraphs 1-3, wherein the epitope consists of amino acid residues R103, G124, M196, Y238, T239, E120, and V102.
6. The antigen-binding protein or antigen-binding fragment thereof according to any one of paragraphs 1-3, wherein the epitope consists of amino acid residues G124, M196, Y238, T239, and E120.
7. The antigen-binding protein or antigen-binding fragment thereof according to any one of paragraphs 1-4, the antigen-binding protein comprising an immunoglobulin heavy chain variable region (VH) and light chain variable region (VL), wherein
   (1) the VH comprises heavy chain CDR1, heavy chain CDR2 and heavy chain CDR3 of the heavy chain variable region having the amino acid sequence as set forth in SEQ ID NO: 10, and the VL comprises light chain CDR1, light chain CDR2 and light chain CDR3 of the light chain variable region having the amino acid sequence as set forth in SEQ ID NO: 9;
   (2) the VH comprises heavy chain CDR1, heavy chain CDR2 and heavy chain CDR3 of the heavy chain variable region having the amino acid sequence as set forth in SEQ ID NO: 2, and the VL comprises light chain CDR1, light chain CDR2 and light chain CDR3 of the light chain variable region having the amino acid sequence as set forth in SEQ ID NO: 1;
   (3) the VH comprises heavy chain CDR1, heavy chain CDR2 and heavy chain CDR3 of the heavy chain variable region having the amino acid sequence as set forth in SEQ ID NO: 4, and the VL comprises light chain CDR1, light chain CDR2 and light chain CDR3 of the light chain variable region having the amino acid sequence as set forth in SEQ ID NO: 3;
   (4) the VH comprises heavy chain CDR1, heavy chain CDR2 and heavy chain CDR3 of the heavy chain variable region having the amino acid sequence as set forth in SEQ ID NO: 6, and the VL comprises light chain CDR1, light chain CDR2 and light chain CDR3 of the light chain variable region having the amino acid sequence as set forth in SEQ ID NO: 5;
   (5) the VH comprises heavy chain CDR1, heavy chain CDR2 and heavy chain CDR3 of the heavy chain variable region having the amino acid sequence as set forth in SEQ ID NO: 8, and the VL comprises light chain CDR1, light chain CDR2 and light chain CDR3 of the light chain variable region having the amino acid sequence as set forth in SEQ ID NO: 7; or,
   (6) the VH comprises heavy chain CDR1, heavy chain CDR2 and heavy chain CDR3 of the heavy chain variable region having the amino acid sequence as set forth in SEQ ID NO: 12, and the VL comprises light chain CDR1, light chain CDR2 and light chain CDR3 of the light chain variable region having the amino acid sequence as set forth in SEQ ID NO: 11.
8. The antigen-binding protein or antigen-binding fragment thereof according to paragraph 7, wherein the heavy chain variable region has the amino acid sequence as set forth in SEQ ID NO: 10, and the light chain variable region has the amino acid sequence as set forth in SEQ ID NO: 9.
9. The antigen-binding protein or antigen-binding fragment thereof according to any one of paragraphs 1-8, wherein
   (1) the VH comprises heavy chain CDRs 1-3 having the amino acid sequence as set forth in SEQ ID NO: 40-42 respectively, and the VL comprises light chain CDRs having the amino acid sequence as set forth in SEQ ID NO: 37-39 respectively;
   (2) the VH comprises heavy chain CDRs 1-3 having the amino acid sequence as set forth in SEQ ID NO: 16-18 respectively, and the VL comprises light chain CDRs having the amino acid sequence as set forth in SEQ ID NO: 13-15 respectively;
   (3) the VH comprises heavy chain CDRs 1-3 having the amino acid sequence as set forth in SEQ ID NO: 22-24 respectively, and the VL comprises light chain CDRs having the amino acid sequence as set forth in SEQ ID NO: 19-21 respectively;
   (4) the VH comprises heavy chain CDRs 1-3 having the amino acid sequence as set forth in SEQ ID NO: 28-30 respectively, and the VL comprises light chain CDRs having the amino acid sequence as set forth in SEQ ID NO: 25-27 respectively;
   (5) the VH comprises heavy chain CDRs 1-3 having the amino acid sequence as set forth in SEQ ID NO: 34-36 respectively, and the VL comprises light chain CDRs having the amino acid sequence as set forth in SEQ ID NO: 31-33 respectively; or,
   (6) the VH comprises heavy chain CDRs 1-3 having the amino acid sequence as set forth in SEQ ID NO: 46-48 respectively, and the VL comprises light chain CDRs having the amino acid sequence as set forth in SEQ ID NO: 43-45 respectively.
10. The antigen-binding protein or antigen-binding fragment thereof according to paragraph 9, wherein the VH CDRs 1-3 have the amino acid sequences as set forth in SEQ ID NO: 40-42, respectively, and the VL CDRs have the amino acid sequences as set forth in SEQ ID NO: 37-39, respectively.
11. The antigen-binding protein or antigen-binding fragment thereof according to any one of paragraphs 1-10, wherein
   (1) the VH comprises an amino acid sequence having at least 80%, at least 85%, at least 90%, at least 95%, at least 96%, at least 97%, at least 98%, at least 99%, or 100% sequence identity to SEQ ID NO: 10 and the VL comprises an amino acid sequence having at least 80%, at least 85%, at least 90%, at least 95%, at least 96%, at least 97%, at least 98%, at least 99%, or 100% sequence identity to SEQ ID NO: 9;
   (2) the VH comprises an amino acid sequence having at least 80%, at least 85%, at least 90%, at least 95%, at least 96%, at least 97%, at least 98%, at least 99%, or 100% sequence identity to SEQ ID NO: 2 and the VL comprises an amino acid sequence having at least 80%, at least 85%, at least 90%, at least 95%, at least 96%, at least 97%, at least 98%, at least 99%, or 100% sequence identity to SEQ ID NO: 1;
   (3) the VH comprises an amino acid sequence having at least 80%, at least 85%, at least 90%, at least 95%, at least 96%, at least 97%, at least 98%, at least 99%, or 100% sequence identity to SEQ ID NO: 4 and the VL comprises an amino acid sequence having at least 80%, at least 85%, at least 90%, at least 95%, at least 96%, at least 97%, at least 98%, at least 99%, or 100% sequence identity to SEQ ID NO: 3;
   (4) the VH comprises an amino acid sequence having at least 80%, at least 85%, at least 90%, at least 95%, at least 96%, at least 97%, at least 98%, at least 99%, or 100% sequence identity to SEQ ID NO: 6 and the VL comprises an amino acid sequence having at least 80%, at least 85%, at least 90%, at least 95%, at least 96%, at least 97%, at least 98%, at least 99%, or 100% sequence identity to SEQ ID NO: 5;
   (5) the VH comprises an amino acid sequence having at least 80%, at least 85%, at least 90%, at least 95%, at least 96%, at least 97%, at least 98%, at least 99%, or 100% sequence identity to SEQ ID NO: 8 and the VL comprises an amino acid sequence having at least 80%, at least 85%, at least 90%, at least 95%, at least 96%, at least 97%, at least 98%, at least 99%, or 100% sequence identity to SEQ ID NO: 7; or,
   (6) the VH comprises an amino acid sequence having at least 80%, at least 85%, at least 90%, at least 95%, at least 96%, at least 97%, at least 98%, at least 99%, or 100% sequence identity to SEQ ID NO: 12 and the VL comprises an amino acid sequence having at least 80%, at least 85%, at least 90%, at least 95%, at least 96%, at least 97%, at least 98%, at least 99%, or 100% sequence identity to SEQ ID NO: 11.
12. The antigen-binding protein or antigen-binding fragment thereof according to paragraph 11, wherein the VH comprises an amino acid sequence having at least 80%, at least 85%, at least 90%, at least 95%, at least 96%, at least 97%, at least 98%, at least 99%, or 100% sequence identity to SEQ ID NO: 10, and the VL comprises an amino acid sequence having at least 80%, at least 85%, at least 90%, at least 95%, at least 96%, at least 97%, at least 98%, at least 99%, or 100% sequence identity to SEQ ID NO: 9.
13. The antigen-binding protein or antigen-binding fragment thereof according to paragraph 12, wherein
   (1) the VH comprises an amino acid sequence as set forth in SEQ ID NO: 10, and the VL comprises an amino acid sequence as set forth in SEQ ID NO: 9;
   (2) the VH comprises an amino acid sequence as set forth in SEQ ID NO: 2, and the VL comprises an amino acid sequence as set forth in SEQ ID NO: 1;
   (3) the VH comprises an amino acid sequence as set forth in SEQ ID NO: 4, and the VL comprises an amino acid sequence as set forth in SEQ ID NO: 3;
   (4) the VH comprises an amino acid sequence as set forth in SEQ ID NO: 6, and the VL comprises an amino acid sequence as set forth in SEQ ID NO: 5;
   (5) the VH comprises an amino acid sequence as set forth in SEQ ID NO: 8, and the VL comprises an amino acid sequence as set forth in SEQ ID NO: 7; or,
   (6) the VH comprises an amino acid sequence as set forth in SEQ ID NO: 12, and the VL comprises an amino acid sequence as set forth in SEQ ID NO: 11.
14. The antigen-binding protein or antigen-binding fragment thereof according to paragraph 13, wherein the VH comprises an amino acid sequence as set forth in SEQ ID NO: 10, and the VL comprises an amino acid sequence as set forth in SEQ ID NO: 9.
15. The antigen-binding protein or antigen-binding fragment thereof according to any one of paragraphs 1-14, wherein the antigen-binding protein is an antibody.
16. The antigen-binding protein or antigen-binding fragment thereof according to paragraph 15, wherein the antibody is a monoclonal antibody, a bispecific antibody or a multispecific antibody.
17. The antigen-binding protein or antigen-binding fragment thereof according to paragraph 15 or 16, wherein the antibody is selected from the group consisting of a murine antibody, a chimeric antibody, a humanized antibody and a fully human antibody.
18. The antigen-binding protein or antigen-binding fragment thereof according to any one of paragraphs 15-17, wherein the antibody comprises an Fc region.
19. The antigen-binding protein or antigen-binding fragment thereof according to any one of paragraphs 15-18, wherein the antibody is of an isotype selected from the group consisting of IgG, IgA, IgM, IgE and IgD.
20. The antigen-binding protein or antigen-binding fragment thereof according to any one of paragraphs 15-19, wherein the antibody is of a subtype selected from the group consisting of IgG1, IgG2, IgG3 and IgG4.
21. The antigen-binding protein or antigen-binding fragment thereof according to any one of paragraphs 15-20, wherein the Fc region comprises a modification capable of increasing the binding to FcRn.
22. The antigen-binding protein or antigen-binding fragment thereof according to any one of paragraphs 15-21, wherein the antibody comprises a heavy chain (HC) and a light chain (LC), and wherein:
   (1) the heavy chain comprises an amino acid sequence having at least 80%, at least 85%, at least 90%, at least 95%, at least 96%, at least 97%, at least 98%, at least 99%, or 100% sequence identity to SEQ ID NO: 58 and the light chain comprises an amino acid sequence having at least 80%, at least 85%, at least 90%, at least 95%, at least 96%, at least 97%, at least 98%, at least 99%, or 100% sequence identity to SEQ ID NO: 57;
   (2) the heavy chain comprises an amino acid sequence having at least 80%, at least 85%, at least 90%, at least 95%, at least 96%, at least 97%, at least 98%, at least 99%, or 100% sequence identity to SEQ ID NO: 50 and the light chain comprises an amino acid sequence having at least 80%, at least 85%, at least 90%, at least 95%, at least 96%, at least 97%, at least 98%, at least 99%, or 100% sequence identity to SEQ ID NO: 49;
   (3) the heavy chain comprises an amino acid sequence having at least 80%, at least 85%, at least 90%, at least 95%, at least 96%, at least 97%, at least 98%, at least 99%, or 100% sequence identity to SEQ ID NO: 52 and the light chain comprises an amino acid sequence having at least 80%, at least 85%, at least 90%, at least 95%, at least 96%, at least 97%, at least 98%, at least 99%, or 100% sequence identity to SEQ ID NO: 51;
   (4) the heavy chain comprises an amino acid sequence having at least 80%, at least 85%, at least 90%, at least 95%, at least 96%, at least 97%, at least 98%, at least 99%, or 100% sequence identity to SEQ ID NO: 54 and the light chain comprises an amino acid sequence having at least 80%, at least 85%, at least 90%, at least 95%, at least 96%, at least 97%, at least 98%, at least 99%, or 100% sequence identity to SEQ ID NO: 53;
   (5) the heavy chain comprises an amino acid sequence having at least 80%, at least 85%, at least 90%, at least 95%, at least 96%, at least 97%, at least 98%, at least 99%, or 100% sequence identity to SEQ ID NO: 56 and the light chain comprises an amino acid sequence having at least 80%, at least 85%, at least 90%, at least 95%, at least 96%, at least 97%, at least 98%, at least 99%, or 100% sequence identity to SEQ ID NO: 55; or,
   (6) the heavy chain comprises an amino acid sequence having at least 80%, at least 85%, at least 90%, at least 95%, at least 96%, at least 97%, at least 98%, at least 99%, or 100% sequence identity to SEQ ID NO: 60 and the light chain comprises an amino acid sequence having at least 80%, at least 85%, at least 90%, at least 95%, at least 96%, at least 97%, at least 98%, at least 99%, or 100% sequence identity to SEQ ID NO: 59.
23. The antigen-binding protein or antigen-binding fragment thereof according to paragraph 22, wherein the heavy chain comprises an amino acid sequence having at least 80%, at least 85%, at least 90%, at least 95%, at least 96%, at least 97%, at least 98%, at least 99%, or 100% sequence identity to SEQ ID NO: 58, and the light chain comprises an amino acid sequence having at least 80%, at least 85%, at least 90%, at least 95%, at least 96%, at least 97%, at least 98%, at least 99%, or 100% sequence identity to SEQ ID NO: 57.
24. The antigen-binding protein or antigen-binding fragment thereof according to paragraph 22 or 23, wherein:
   (1) the heavy chain comprises an amino acid sequence as set forth in SEQ ID NO: 58, and the light chain comprises an amino acid sequence as set forth in SEQ ID NO: 57;
   (2) the heavy chain comprises an amino acid sequence as set forth in SEQ ID NO: 50, and the light chain comprises an amino acid sequence as set forth in SEQ ID NO: 49;
   (3) the heavy chain comprises an amino acid sequence as set forth in SEQ ID NO: 52, and the light chain comprises an amino acid sequence as set forth in SEQ ID NO: 51;
   (4) the heavy chain comprises an amino acid sequence as set forth in SEQ ID NO: 54, and the light chain comprises an amino acid sequence as set forth in SEQ ID NO: 53;
   (5) the heavy chain comprises an amino acid sequence as set forth in SEQ ID NO: 56, and the light chain comprises an amino acid sequence as set forth in SEQ ID NO: 55; or,
   (6) the heavy chain comprises an amino acid sequence as set forth in SEQ ID NO: 60, and the light chain comprises an amino acid sequence as set forth in SEQ ID NO: 59.
25. The antigen-binding protein or antigen-binding fragment thereof according to paragraph 24, wherein the heavy chain comprises an amino acid sequence as set forth in SEQ ID NO: 58, and the light chain comprises an amino acid sequence as set forth in SEQ ID NO: 57.
26. The antigen-binding protein or antigen-binding fragment thereof according to any one of paragraphs 1-25, wherein the antigen-binding fragment is selected from the group consisting of Fab, Fab', F(ab')₂, Fv, scFv and ds-scFv.
27. The antigen-binding protein or antigen-binding fragment thereof according to any one of paragraphs 15-26, wherein compared to an anti-TL1A antibody that binds to an epitope other than said epitope in TL1A, the antibody forms a complex with a smaller size when binding to TL1A.
28. The antigen-binding protein or antigen-binding fragment thereof according to paragraph 27, wherein the particle size of the complex formed by the antibody and TL1A is less than 33 nm, preferably less than 30 nm, more preferably less than 25 nm.
29. The antigen-binding protein or antigen-binding fragment thereof according to any one of paragraphs 15-28, wherein the antibody binds to TL1A at a 1:1 ratio to form an antigen-antibody complex.
30. The antigen-binding protein or antigen-binding fragment thereof according to any one of paragraphs 15-29, wherein the Fc valence of the complex formed by the antibody and TL1A is <5, preferably 2.
31. The antigen-binding protein or antigen-binding fragment thereof according to any one of paragraphs 15-30, wherein the binding of the antibody to TL1A under neutral pH conditions is stronger than the binding to TL1A under acidic conditions, preferably, the bingding of the antibody to TL1A at pH 7.4 is stronger than the binding to TL1A at pH ≤ 6.0 (e.g., pH 6.0, 5.8, 5.6).
32. The antigen-binding protein or antigen-binding fragment thereof according to paragraph 31, wherein the antibody has a binding affinity constant (KD) of 10⁻⁹M to TL1A at pH 7.4 and does not bind to TL1A at pH 5.6.
33. An isolated antibody that specifically binds to TL1A, wherein the isolated antibody comprises an immunoglobulin heavy chain (HC) comprising a heavy chain variable region (VH) comprising a heavy chain CDR1, a heavy chain CDR2, and a heavy chain CDR3 (VH CDRs 1-3), and an immunoglobulin light chain (LC) comprising a variable region (VL) comprising a light chain CDR1, a light chain CDR2, and a light chain CDR3 (VL CDRs 1-3), wherein the VH CDRs 1-3 have the amino acid sequences as set forth in SEQ ID NO: 40-42, respectively, and the VL CDRs 1-3 have the amino acid sequences as set forth in SEQ ID NO: 37-39, respectively.
34. The isolated antibody or antigen-binding fragment thereof of paragraph 33, wherein the VH comprises an amino acid sequence having at least 80%, at least 85%, at least 90%, at least 95%, at least 96%, at least 97%, at least 98%, at least 99%, or 100% amino acid sequence identity to SEQ ID NO: 10, and the VL comprises an amino acid sequence having at least 80%, at least 85%, at least 90%, at least 95%, at least 96%, at least 97%, at least 98%, at least 99%, or 100% amino acid sequence identity to SEQ ID NO: 9.
35. The isolated antibody or antigen-binding fragment thereof of paragraph 33 or 34, wherein the VH comprises an amino acid sequence as set forth in SEQ ID NO: 10, and the VL comprises an amino acid sequence as set forth in SEQ ID NO: 9.
36. The isolated antibody or antigen-binding fragment thereof of any one of paragraphs 33-35, wherein the heavy chain comprises an amino acid sequence having at least 80%, at least 85%, at least 90%, at least 95%, at least 96%, at least 97%, at least 98%, at least 99%, or 100% amino acid sequence identity to SEQ ID NO: 58, and the light chain comprises an amino acid sequence having at least 80%, at least 85%, at least 90%, at least 95%, at least 96%, at least 97%, at least 98%, at least 99%, or 100% amino acid sequence identity to SEQ ID NO: 57.
36. The isolated antibody or antigen-binding fragment thereof of paragraph 36, wherein the heavy chain comprises an amino acid sequence set forth in SEQ ID NO: 58, and the light chain comprises an amino acid sequence set forth in SEQ ID NO: 57.
37. The isolated antibody or antigen-binding fragment thereof of any one of paragraphs 33-36, wherein the isolated antibody is a monoclonal antibody, a bispecific antibody or a multispecific antibody.
38. The isolated antibody or antigen-binding fragment thereof of any one of paragraphs 33-37, wherein the isolated antibody is a murine antibody, a chimeric antibody, a humanized antibody, or a fully human antibody.
39. The isolated antibody or antigen-binding fragment thereof of any one of paragraphs 33-38, wherein the isolated antibody or antigen-binding fragment thereof binds to an epitope in TL1A, wherein the epitope comprises one or more amino acid residues selected from the group consisting of: R103 , G124, M196, Q193, Y238, T239, K240, E241, H118, E120, H121, E122, L123, V102 and T105, and wherein the amino acid positions are numbered according to the amino acid sequence of TL1A as set forth in SEQ ID NO: 68.
40. The isolated antibody or antigen-binding fragment thereof of paragraph 39, wherein the epitope consists of amino acid residues R103, G124, M196, Y238, T239, K240, E241, H118, E120, H121, E122, L123, V102 and T105.
41. The isolated antibody or antigen-binding fragment thereof of any one of paragraphs 33-40, wherein the isolated antibody comprises an Fc region.
42. The isolated antibody or antigen-binding fragment thereof of any one of paragraphs 33-41, wherein the antibody is of an isotype selected from the group consisting of IgG, IgA, IgM, IgE and IgD.
43. The isolated antibody or antigen-binding fragment thereof according to any one of paragraphs 33-42, wherein the antibody is of a subtype selected from the group consisting of IgG1, IgG2, IgG3 and IgG4.
44. The isolated antibody or antigen-binding fragment thereof according to any one of paragraphs 41-43, wherein the Fc region comprises a modification capable of increasing the binding to FcRn.
45. The isolated antibody or antigen-binding fragment thereof according to any one of paragraphs 33-44, wherein the antigen-binding fragment is selected from the group consisting of Fab, Fab', F(ab')2, Fv, scFv and ds-scFv.
46. The isolated antibody or antigen-binding fragment thereof according to any one of paragraphs 39-45, wherein compared to a control anti-TL1A antibody that binds to an epitope other than said epitope in TL1A to form a control complex, said isolated antibody binds to TL1A to form a complex smaller in size compared to the control complex.
47. The isolated antibody or antigen-binding fragment thereof according to paragraph 46, wherein the size of the complex formed by the isolated antibody and TL1A is less than 33 nm, preferably less than 30 nm, more preferably less than 25 nm.
48. The isolated antibody or antigen-binding fragment thereof according to any one of paragraphs 33-47, wherein the antibody binds to TL1A at a 1:1 molar ratio to form an antigen-antibody complex.
49. The isolated antibody or antigen-binding fragment thereof according to any one of paragraphs 33-48, wherein the Fc valence of the complex formed by the antibody and TL1A is <5, preferably 2.
50. The isolated antibody or antigen-binding fragment thereof according to any one of paragraphs 33-49, wherein the binding of the antibody to TL1A under neutral pH conditions is stronger than the binding to TL1A under acidic conditions, preferably, the bingding of the antibody to TL1A at pH 7.4 is stronger than the binding to TL1A at pH ≤ 6.0 (*e.g*., pH 6.0, 5.8, 5.6).
51. The isolated antibody or antigen-binding fragment thereof according to paragraph 50, wherein the antibody has a binding affinity constant (KD) of 10⁻⁹M to TL1A at pH 7.4 and does not bind to TL1A at pH 5.6.
52. A nucleic acid comprising a nucleotide sequence encoding the antigen-binding protein or antigen-binding fragment thereof according to any one of paragraphs 1-51.
53. A vector comprising the nucleic acid according to paragraph 52.
54. A host cell comprising the nucleic acid according to paragraph 52 or the vector according to paragraph 53.
55. A method for preparing the antigen-binding protein or antigen-binding fragment thereof according to any one of paragraphs 1-51, wherein the method comprises:
   a) culturing the host cell according to paragraph 54 under conditions suitable for expression of the antigen-binding protein or antigen-binding fragment thereof; and
   b) isolating the antigen-binding protein or antigen-binding fragment thereof from the culture and/or culture supernatant of the host cell.
56. A pharmaceutical composition comprising the antigen-binding protein or antigen-binding fragment thereof according to any one of paragraphs 1-51, the nucleic acid according to paragraph 52, the vector according to paragraph 53, or the host cell according to paragraph 54, and optionally a pharmaceutically acceptable carrier or excipient.
57. The pharmaceutical composition according to paragraph 56, wherein the pharmaceutical composition further comprises a second therapeutic agent, optionally the second therapeutic agent is selected from the group consisting of an antibody, a chemotherapeutic agent, a radiotherapeutic agent, an antisense oligonucleotide (such as siRNA), a peptide and a small molecule drug.
58. A method of treating a disease in a subject (such as a disease treatable by inhbiting or antagonizing TL1A activity, and/or TL1A binding to a receptor of TL1A such as DR3), comprising administering to the subject an effective amount of the antigen-binding protein or antigen-binding fragment thereof according to any one of paragraphs 1-51, the nucleic acid according to paragraph 52, the vector according to paragraph 53, the host cell according to paragraph 54, or the pharmaceutical composition according to paragraph 56 or 57, wherein the disease is optionally selected from an autoimmune disease, an inflammatory disease and a fibrotic disease.
59. The method of paragraph 58, wherein the disease is selected from the group consisting of inflammatory bowel disease (*e.g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive block fibrosis, and arthrofibrosis.
60. The method of paragraph 58 or 59, further comprising administering a second therapeutic agent to the subject, preferably the second therapeutic agent is selected from the group consisting of an antibody, a chemotherapeutic agent, a radiotherapeutic agent, an antisense oligonucleotide (such as siRNA), a peptide and a small molecule drug.
61. Use of the antigen-binding protein or antigen-binding fragment thereof according to any one of paragraphs 1-51, the nucleic acid according to paragraph 52, the vector according to paragraph 53, the host cells according to paragraph 54, or a pharmaceutical composition according to paragraph 56 or 57, in the manufacture of a medicament for treating a disease in a subject (such as a disease treatable by inhbiting or antagonizing TL1A activity, and/or TL1A binding to a receptor of TL1A such as DR3), wherein the disease is optionally selected from an autoimmune disease, an inflammatory disease and a fibrotic disease.
62. The use of paragraph 61, wherein the disease is selected from the group consisting of inflammatory bowel disease (*e.g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.
63. The use according to paragraph 61 or 62, wherein the medicament is for use in combined administration with a second therapeutic agent, preferably, the second therapeutic agent is selected from the group consisting of an antibody, a chemotherapeutic agent, a radiotherapeutic agent, an antisense oligonucleotide (such as siRNA), a peptide and a small molecule drug.
64. An isolated antibody that specifically binds to TL1A, wherein the isolated antibody comprises an immunoglobulin heavy chain (HC) comprising a heavy chain variable region (VH) comprising a heavy chain CDR1, a heavy chain CDR2, and a heavy chain CDR3 (VH CDRs 1-3), and an immunoglobulin light chain (LC) comprising a variable region (VL) comprising a light chain CDR1, a light chain CDR2, and a light chain CDR3 (VL CDRs 1-3), wherein the VH CDRs 1-3 have the amino acid sequences as set forth in SEQ ID NO: 40-42, respectively, and the VL CDRs 1-3 have the amino acid sequences as set forth in SEQ ID NO: 37-39, respectively.
65. The isolated antibody of paragraph 64, wherein the VH comprises an amino acid sequence as set forth in SEQ ID NO: 10, and the VL comprises an amino acid sequence as set forth in SEQ ID NO: 9.
66. The isolated antibody of paragraph 64 or 65, wherein the heavy chain comprises an amino acid sequence set forth in SEQ ID NO: 58, and the light chain comprises an amino acid sequence set forth in SEQ ID NO: 57.
67. The isolated antibody of any one of paragraphs 64-66, wherein the isolated antibody comprises an Fc region.
68. A nucleic acid comprising a nucleotide sequence encoding the isolated antibody according to any one of paragraphs 64-67.
69. The nucleic acid of paragraph 68, comprising the nucleotide sequence encoding the isolated antibody according to paragraph 66.
70. A vector comprising the nucleic acid according to paragraph 68 or 69.
71. The vector of paragraph 70, comprising the nucleic acid according to paragraph 69.
72. A host cell comprising the nucleic acid according to paragraph 68 or 69, or the vector according to paragraph 70 or 71.
73. The host cell of paragraph 72, comprising the nucleic acid according to paragraph 69, or the vector according to paragraph 71.
74. A method for preparing the isolated antibody according to any one of paragraphs 64-67, wherein the method comprises:
   a) culturing the host cell according to paragraph 72 or 73 under conditions suitable for expression of the isolated antibody; and
   b) isolating the isolated antibody from the culture and/or culture supernatant of the host cell.
75. A pharmaceutical composition comprising the isolated antibody according to any one of paragraphs 64-67, and optionally a pharmaceutically acceptable carrier or excipient.
76. The pharmaceutical composition of paragraph 75, comprising the isolated antibody according to paragraph 66, and a pharmaceutically acceptable carrier or excipient.
77. A method of treating a disease in a subject, comprising administering to the subject an effective amount of the isolated antibody according to any one of paragraphs 64-67.
78. The method of paragraph 77, wherein the disease is an autoimmune disease, an inflammatory disease, or a fibrotic disease.
79. The method of paragraph 77 or 78, comprising administering to the subj ect the effective amount of the isolated antibody according to paragraph 66, and a pharmaceutically acceptable carrier or excipient.
80. Use of the isolated antibody according to any one of paragraphs 64-67 in the manufacture of a medicament for treating a disease in a subject.
81. The use of paragraph 80, wherein the disease is an autoimmune disease, an inflammatory disease, or a fibrotic disease.
82. The use of paragraph 80 or 81, wherein the isolated antibody is the isolated antibody according to paragraph 66.

### DESCRIPTION OF THE DRAWINGS

The specific features of the invention to which this application relates are set forth in the appended claims. An understanding of the features and advantages of the invention to which this application relates will be achieved by reference to the following detailed description that sets forth illustrative embodiments and the accompanying drawings. A brief description of the drawings is as follows:
Figure 1. ELISA binding curves of M701 antibodies and control antibodies to human TL1A at pH7.4.
Figure 2. ELISA binding curves of M701 antibodies and control antibodies to human TL1A at pH7.4 and pH6.0.
Figure 3. ELISA binding curves of M701 antibodies and control antibodies to human TL1A at pH7.4, pH5.8 and pH5.6.
Figure 4. Binding and dissociation curves of M701 antibodies and control antibodies with human TL1A at pH7.4 and pH5.6.
Figure 5. Spatial location of epitopes of M701 antibodies and control antibodies.
Figure 6. Design (A) and results (B) of the Ouchterlony double immunodiffusion experiment of M701 antibodies and control antibodies.
Figure 7. SEC spectra of mixtures of M701 antibodies and control antibodies with human TL1A.
Figure 8. Negative staining electron microscopy images of M701 antibodies and control antibodies binding to human TL1A.
Figure 9. Conformation of the complex of M701-4007 antibody bound to human TL1A.
Figure 10. SEC spectra of mixtures of M701-4007 Fab and human TL1A at different ratios.
Figure 11. Soluble TL1A neutralizing activity of M701 and control antibodies measured by reporter gene assay.
Figure 12. Membrane-bound TL1A neutralizing activity of M701 and control antibodies measured by reporter gene assay.
Figure 13. Effect of M701 antibodies and control antibodies on DcR3/TL1A interaction.
Figure 14. Inhibition of exogenous TL1A activated T cells by M701 antibodies and control antibodies.
Figure 15. Inhibition of endogenous TL1A activated immune cells by M701 antibodies and control antibodies.
Figure 16. Pharmacokinetic study of M701 antibodies and control antibodies in SCID mice: (A) serum concentration-time curve of antibodies, (B) serum concentration-time curves of human TL1A.
Figure 17. Serum concentration-time curves of M701 antibodies and control antibodies in rhesus monkeys.

### DETAILED DESCRIPTION OF THE INVENTION

The aforementioned features and advantages of the invention as well as additional features and advantages thereof will be more clearly understood hereafter by detailed description of the following embodiments when taken in conjunction with the drawings.

The embodiments described herein with reference to drawings are explanatory, illustrative, and used to generally understand the present invention. The embodiments shall not be construed to limit the scope of the present invention. The same or similar elements and the elements having same or similar functions are denoted by like reference numerals throughout the descriptions.

### Term definitions

Unless indicated or defined otherwise, all terms used have their usual meaning in the art, which will be clear to the skilled person. Reference is for example made to the standard handbooks, such as Leuenberger, H.G.W, Nagel, B. and Klbl, H. eds., "A multilingual glossary of biotechnological terms: (IUPAC Recommendations)", Helvetica Chimica Acta (1995), CH-4010 Basel, Switzerland; Sambrook et al, "Molecular Cloning: A Laboratory Manual" (2nd Ed.), Vols. 1-3, Cold Spring Harbor Laboratory Press (1989); F. Ausubel et al, eds., "Current protocols in molecular biology", Green Publishing and Wiley InterScience, New York (1987); Roitt et al., "Immunology (6th Ed.), Mosby/Elsevier, Edinburgh (2001); and Janeway et al., "Immunobiology" (6th Ed.), Garland Science Publishing/Churchill Livingstone, New York (2005), as well as the general background art cited above.

As used herein, "antigen" broadly refers to a molecule or part of a molecule capable of being bound by an antibody, which is additionally capable of inducing in an animal the production of an antibody capable of binding to an epitope of the antigen. An antigen may have one epitope or more than one epitope.

As used herein, the term "epitope" refers to the site on an antigen to which an antigen-binding protein binds. Epitopes can be formed from contiguous amino acids or from non-contiguous amino acids juxtaposed by the tertiary fold of one or more proteins. Epitopes formed by contiguous amino acids (also called linear epitopes) are generally retained upon exposure to denaturing solvents, whereas epitopes formed by tertiary folding (also called conformational epitopes) are usually lost upon treatment with denaturing solvents. Epitopes typically include at least 2, more typically at least 5 or 8-10 amino acids in a unique spatial conformation. An epitope defines the minimal binding site of an antigen-binding protein and is therefore a specific target for the antigen-binding protein or antigen-binding fragment thereof.

As used herein, an "antigen-binding protein" is a protein comprising a moiety that binds to an antigen, particularly a protein or polypeptide comprising the heavy chain variable region (VH) and/or the light chain variable region (VL) of an anti-TL1A antibody as disclosed herein and capable of binding to TL1A. Examples of antigen-binding proteins include antibodies, antibody fragments (*e.g*., the antigen-binding portion of an antibody), antibody derivatives, antibody analogs, T cell receptors (TCRs), chimeric antigen receptors (CARs), and the like.

As used herein, the term "antibody" refers to an immunoglobulin molecule which has the ability to specifically bind to a specific antigen. Such molecule typically contains two heavy (H) chains and two light (L) chains interconnected by disulfide bonds. Each heavy chain consists of a heavy chain variable region (or domain) (herein abbreviated as VH) and a heavy chain constant region. The heavy chain constant region consists of three domains, CH1, CH2 and CH3. Each light chain consists of a light chain variable region (or domain) (herein abbreviated as VL) and a light chain constant region. The light chain constant region consists of one domain, *i.e.* CL. The variable regions of the antibody heavy and light chain contain binding domains that interact with the antigen. The constant region of an antibody can mediate the binding of immunoglobulins to host tissues or factors, including various cells of the immune system (such as effector cells) and components of the complement system such as C1q (the first component in the classical pathway of complement activation).

The heavy chain of immunoglobulins can be divided into three functional regions: Fd region, hinge region and Fc region (fragment crystallizable). The Fd region contains VH and CH1 domains and combines with the light chain to form Fab (fragment antigen-binding). The Fc fragment is responsible for immunoglobulin effector functions, including, for example, complement fixation and binding to cognate Fc receptors on effector cells. The hinge region found in the IgG, IgA and IgD immunoglobulin classes acts as a flexible spacer, allowing the Fab portion to move freely in space relative to the Fc region. Hinge domains are structurally diverse, varying in sequence and length between immunoglobulin classes and subclasses.

A "light chain variable region" (VL) or "heavy chain variable region" (VH) consists of a "framework" region interrupted by three "complementarity determining regions" or "CDRs". The framework regions serve to align the CDRs for specific binding to an epitope of an antigen. The CDRs include the amino acid residues of an antibody that are primarily responsible for antigen binding. From amino-terminus to carboxyl-terminus, both VL and VH domains comprise the following framework (FR) and CDR regions: FR1, CDR1, FR2, CDR2, FR3, CDR3, and FR4. CDRs 1, 2, and 3 of a VL domain are also referred to herein, respectively, as LCDR1, LCDR2, and LCDR3; CDRs 1, 2, and 3 of a VH domain are also referred to herein, respectively, as HCDR1, HCDR2, and HCDR3.

The assignment of amino acids to each VL and VH domain is in accordance with any conventional definition of CDRs. Conventional definitions include, the Kabat definition (Kabat, Sequences of Proteins of Immunological Interest (National Institutes of Health, Bethesda, MD, 1987 and 1991), the Chothia definition (Chothia & Lesk, J. Mol. Biol. 196:901-917, 1987; Chothia et al., Nature 342:878-883, 1989); a composite of Chothia Kabat CDR in which CDR-H1 is a composite of Chothia and Kabat CDRs; the AbM definition used by Oxford Molecular's antibody modelling software; and the CONTACT definition of Martin et al. (world wide web bioinfo.org.uk/abs).

In the technical solutions of the present invention, Kabat definition, Chothia definition, Combined definition rules including Kabat definition and Chothia definition, AbM definition, CONTACT definition, *etc.* can be used to determine the amino acid residues in the variable domain sequences, see table below for details. It will be understood by those skilled in the art that, unless otherwise specified, the "CDRs" and "complementarity determining regions" of an antibody or a region thereof (*e.g.,* a variable region) should be understood to encompass complementarity determining regions as defined by any of the above known schemes described by the present invention. Although in the preferred embodiments, the amino acid sequences of the CDRs are shown based on the Kabat definition rules, the corresponding amino acid sequences according to other CDR definition rules should also be within the protection scope of the present invention.

| | Kabat | Chothia | Combined | AbM | CONTACT |
|---|---|---|---|---|---|
| LCDR1 | L24--L34 | L24--L34 | L24-L34 | L24--L34 | L30--L36 |
| LCDR2 | L50--L56 | L50--L56 | L50-L56 | L50--L56 | L46--L55 |
| LCDR3 | L89--L97 | L89--L97 | L89--L97 | L89--L97 | L89--L96 |
| HCDR1 | H31--H35 | H26--H32 | H26--H35 | H26--H35 | H30--H35 |
| HCDR2 | H50--H65 | H52--H56 | H50--H65 | H50--H58 | H47--H58 |
| HCDR3 | H95--H102 | H95--H102 | H95--H102 | H95--H102 | H93--H101 |

The term "antibody" as used herein should be understood in its broadest meaning, and includes monoclonal antibodies (including full-length monoclonal antibodies), polyclonal antibodies, antibody fragments, single-arm antibodies, and multi-specific antibodies containing at least two different antigen binding regions (*e.g*., bispecific antibodies). The antibody may contain additional modifications, such as non-naturally occurring amino acids, mutations in Fc regions, and mutations in glycosylation sites. Antibodies also include post-translation modified antibodies, fusion proteins containing the antigenic determinants of the antibody, and immunoglobulin molecules containing any other modifications to antigen recognition sites, as long as these antibodies exhibit desired biological activity (*e.g*., single-arm antibodies).

As used herein, the term "antigen binding fragment" of an antigen binding protein refers to one or more fragments of an antigen binding protein that retain the ability to specifically bind to an antigen (*e.g*., an TL1A protein). For example, the antigen binding function of an antibody can be performed by fragments of a full-length antibody.

Examples of "antigen binding fragment" include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fab' fragment, which is essentially an Fab with part of the hinge region; (iv) a Fd fragment consisting of the VH and CH1 domains; (v) a Fd' fragment having VH and CH1 domains and one or more cysteine residues at the C-terminus of the CH1 domain; (vi) a Fv fragment consisting of the VL and VH domains of a single arm of an antibody, (vii) a dAb fragment, which consists of a VH domain; (viii) an isolated complementarity determining region (CDR); and (ix) a nanobody, a heavy chain variable region containing a single variable domain and two constant domains. Furthermore, although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv)). Such single chain antibodies are also intended to be encompassed within the term "antigen binding fragment" of an antibody. Furthermore, the term also includes a "linear antibody" comprising a pair of tandem Fd segments (VH-CH1-VH-CH1), which forms an antigen binding region together with a complementary light chain polypeptide, and a modified version of any of the foregoing fragments, which retains antigen binding activity.

These antigen binding fragments can be obtained using conventional techniques known to those with skill in the art, and the fragments are screened for utility in the same manner as are intact antibodies.

As used herein, the term "Fc region" generally refers to a dimeric complex comprising an immunoglobulin heavy chain C-terminal polypeptide sequence obtainable by papain digestion of an intact antibody. The Fc region may comprise native or variant Fc sequences. Although the boundaries of the Fc sequence of an immunoglobulin heavy chain can vary, the human IgG heavy chain Fc sequence is generally defined as the segment of the Fc sequence from the amino acid residue at approximately position Cys226, or from approximately position Pro230, to the carboxyl terminus. However, the C-terminal lysine (Lys447) of the Fc sequence may or may not be present. The Fc sequence of an immunoglobulin generally contains two constant domains, a CH2 domain and a CH3 domain, and optionally a CH4 domain.

"Fc receptor" or "FcR" refers to a receptor that binds the Fc region of an antibody. For example, the FcR may be an FcR (gamma receptor) that binds an IgG antibody, including receptors of the FcyRI, FcyRII, and FcyRIII subclasses. Fc receptors or FcR also include the neonatal receptor FcRn, which is responsible for the transfer of maternal IgG to the fetus (Guyer et al., J. Immunol. 117:587 (1976); and Kim et al., J. Immunol. 24:249 (1994)) and the regulation of immunoglobulin homeostasis. Studies have found that FcRn receptors play a key role in the *in vivo* half-life of antibodies and Fc fusion proteins. The presence of FcRn receptors makes circulating IgG and Fc fusion proteins have a longer serum half-life. The FcRn receptor can specifically bind to the Fc region of monoclonal antibodies and Fc fusion proteins, but this binding ability is regulated by pH value. Fc fusion proteins and antibody molecules can be taken up by cells through pinocytosis. Once the protein is endocytosed, the FcRn receptor binds to the antibody or Fc fusion protein with high affinity under acidic pH (pH6.0) conditions, and then the antibody or Fc fusion protein is released into the blood circulation system through exocytosis. The pH during exocytosis is consistent with the blood circulation system (pH7.4). At this time, the affinity of the antibody or Fc fusion protein to FcRn is greatly reduced, so it can be successfully released from the cell membrane. During the endocytosis process, some antibodies or Fc fusion proteins fail to bind to FcRn or fall off during the binding process, and are then taken into lysosomes and degraded.

As used herein, the term "binding" or "specifically binding" refers to a non-random binding reaction between two molecules, such as between an antibody and its target antigen. The binding specificity of an antibody can be determined based on affinity and/or avidity. The affinity, represented by the equilibrium constant for the dissociation of an antigen with an antibody (KD), is a measure for the binding strength between an antigenic determinant and an antigen-binding site on the antibody: the lesser the value of the KD, the stronger the binding strength between an antigenic determinant and the antibody. Alternatively, the affinity can also be expressed as the affinity constant (KA), which is 1/KD. Avidity is the measure of the strength of binding between an antibody and the pertinent antigen. Avidity is related to both the affinity between an antigenic determinant and its antigen binding site on the antibody and the number of pertinent binding sites present on the antibody. Typically, an antibody will bind to an antigen with a dissociation constant (KD) of 10⁻⁵ to 10⁻¹² M or less, and preferably 10⁻⁷ to 10⁻¹² M or less and more preferably 10⁻⁸ to 10⁻¹² M, and/or with a binding affinity of at least 10⁷ M⁻¹, preferably at least 10⁸ M⁻¹, more preferably at least 10⁹ M⁻¹, such as at least 10¹⁰ M⁻¹. Any KD value greater than 10⁻⁴ M is generally considered to indicate non-specific binding. Specifically binding of an antibody to an antigen or antigenic determinant can be determined in any suitable manner known, including, for example, Scatchard analysis and/or competitive binding assays, such as radioimmunoassays (RIA), enzyme immunoassays (EIA) and sandwich competition assays, and the different variants thereof known in the art.

In this application, the term "KD" generally refers to the equilibrium dissociation constant, which is the ratio of dissociation rate constant (kdis, also known as "off-rate (koff)" or "kd") and on-rate constants (kon, also called "on-rate" or "ka"). The binding affinity of an antigen-binding protein (*e.g.,* an antibody) for an antigen can be expressed using the association rate constant (kon), the dissociation rate constant (kdis), and the equilibrium dissociation constant (KD). Methods for determining association and dissociation rate constants are well known in the art, including but not limited to biofilm interference technique (BLI), radioimmunoassay (RIA), equilibrium dialysis, surface plasmon resonance (SPR), and fluorescence resonance energy transfer (FRET), co-immunoprecipitation (Co-IP) and protein chip technology. The measured affinity for a particular protein-protein interaction can differ if measured under different conditions (*e.g*., salt concentration, pH).

As used herein, the term "sequence identity" refers to the extent to which two sequences (amino acid) have the same residue at the same positions in an alignment. For example, "an amino acid sequence is X% identical to SEQ ID NO: Y" refers to % identity of the amino acid sequence to SEQ ID NO: Y and is elaborated as X% of residues in the amino acid sequence are identical to the residues of sequence disclosed in SEQ ID NO: Y

Generally, computer programs are employed for such calculations. Exemplary programs that compare and align pairs of sequences, include ALIGN (Myers and Miller, 1988), FASTA (Pearson and Lipman, 1988; Pearson, 1990) and gapped BLAST (Altschul et al., 1997), BLASTP, BLASTN, or GCG (Devereux et al., 1984).

As used herein, the term "monoclonal antibody" refers to an antibody obtained from a substantially homogeneous antibody population. That is, each antibody constituting the population are the same, except for possible naturally occurring mutations in small amount. Monoclonal antibodies are highly specific and are directed against a single antigen. Furthermore, unlike conventional polyclonal antibody preparations, which typically have different antibodies directed against different determinants, each monoclonal antibody is directed against a single determinant on the antigen. In addition to their specificity, the advantage of monoclonal antibodies is that they can be synthesized by hybridoma culture without contamination by other immunoglobulins. The modifier "monoclonal" indicates the characteristic of the antibody being obtained from a substantially homogeneous population of antibodies and is not to be construed as requiring the antibody to be produced by any particular method. For example, the monoclonal antibodies used in accordance with the present invention may be prepared in hybridoma cells, or may be prepared by recombinant DNA methods.

The term "bispecific antibody" is in the context of the present invention to be understood as an antibody having two different antigen-binding regions defined by different antibody sequences. This can be understood as different target binding but includes as well binding to different epitopes in one target. The term "bispecific antibody" as used herein should be understood in its broadest sense, including full-length bispecific antibodies and antigen-binding fragments thereof. Bispecific antibodies may contain additional modifications, such as non-naturally occurring amino acids, mutations in Fc regions, and mutations in glycosylation sites. Bispecific antibodies also include post-translation modified antibodies, fusion proteins containing the antigenic determinants of the antibody, and immunoglobulin molecules containing any other modifications to antigen recognition sites, as long as these antibodies exhibit desired biological activity.

In this application, the term "chimeric antibody" generally refers to an antibody in which the variable region is derived from one species and the constant region is derived from another species. Typically, the variable region is derived from an antibody of an experimental animal such as a rodent ("parent antibody"), and the constant region is derived from a human antibody, so that the resulting chimeric antibody is less likely to induce an adverse immune response in a human individual than the parent (*e.g*., mouse-derived) antibody.

In this application, the term "humanized antibody" generally refers to an antibody in which some or all of the amino acids outside the CDR region of a non-human antibody (*e.g*., a mouse antibody) are replaced by the corresponding amino acids derived from a human immunoglobulin. In the CDR region, small additions, deletions, insertions, substitutions or modifications of amino acids may also be permitted, as long as they still retain the ability of the antibody to bind to a specific antigen. Humanized antibodies may optionally contain at least a portion of the constant region of a human immunoglobulin. "Humanized antibodies" retain antigen specificity similar to that of the original antibody. "Humanized" forms of non-human (*e.g*., murine) antibodies may minimally contain chimeric antibodies derived from sequences of non-human immunoglobulins. In some cases, CDR region residues in a human immunoglobulin (recipient antibody) may be replaced with CDR region residues of a non-human species (donor antibody) (such as mouse, rat, rabbit or non-human primate) having the desired properties, affinity and/or ability. In some cases, FR region residues of a human immunoglobulin may be replaced with corresponding non-human residues. In addition, humanized antibodies may contain amino acid modifications that are not present in the recipient antibody or in the donor antibody. These modifications may be made to further improve the performance of the antibody, such as binding affinity.

In this application, the term "fully human antibody" generally refers to antibodies expressed by an animal by transferring a human antibody-encoding gene into a genetically engineered antibody gene-deficient animal. All parts of the antibody (including the variable and constant regions of the antibody) are encoded by genes of human origin. Fully human antibodies can greatly reduce the immune side effects caused by heterologous antibodies to the human body. Methods for obtaining fully human antibodies in this field may include phage display technology, transgenic mouse technology, ribosome display technology, and RNA-peptide technology.

As used herein, the term "single-armed antibody" refers to a monovalent genetically engineered antibody with only one Fab fragment but an Fc structure.

In this application, the term "subject" generally refers to a mammal. Mammals include, but are not limited to, domesticated animals (*e.g*., cows, sheep, cats, dogs, and horses), primates (*e.g.,* humans and non-human primates, such as monkeys), rabbits, and rodents (*e.g.,* mice and rats). The term "primate" generally refers to monkey and ape species, and includes monkey species such as monkeys from the genera *Macaca* (*e.g., Macaca fascicularis* and *Macaca mulatto*) and *baboon* (*Papio ursinus*), as well as marmosets (species from the genus *Callithrix),* squirrel monkeys (species from the genus *Saimiri)* and tamarins (species from the genus *Saguinus*), and ape species such as *Pan troglodytes,* and also includes *Homo sapiens.*

In this application, the term "nucleic acid" generally refers to nucleotides, deoxyribonucleotides or ribonucleotides or their analogs in an isolated form of any length, isolated from their natural environment or artificially synthesized.

In this application, the term "vector" generally refers to a nucleic acid molecule capable of self-replication in a suitable host, which transfers the inserted nucleic acid molecule into and/or between host cells. The vector may include a vector mainly used to insert DNA or RNA into a cell, a vector mainly used to replicate DNA or RNA, and a vector mainly used for expression of transcription and/or translation of DNA or RNA. The vector also includes vectors with multiple of the above functions. The vector may be a polynucleotide that can be transcribed and translated into a polypeptide when introduced into a suitable host cell. Generally, by culturing a suitable host cell containing the vector, the vector can produce the desired expression product.

In this application, the term "host cell" generally refers to an individual cell, cell line or cell culture that can or already contain a plasmid or vector including a nucleic acid molecule described in the present application, or can express an antibody or antigen-binding fragment thereof described in the present application. The cell may include the offspring of a single host cell. Due to natural, accidental or intentional mutations, the daughter cells may not necessarily be completely identical to the original parent cells in morphology or genome, but they can express the antibodies or antigen-binding fragments thereof described in the present application. The cells can be obtained by transfecting cells *in vitro* using the vectors described in the present application. The cells can be prokaryotic cells (such as Escherichia coli) or eukaryotic cells (such as yeast cells, such as COS cells, Chinese hamster ovary (CHO) cells, HeLa cells, HEK293 cells, COS-1 cells, NS0 cells or myeloma cells). In some cases, the cells can be mammalian cells. For example, the mammalian cells can be CHO-K1 cells. In the present application, the term "recombinant cell" generally refers to a cell into which a recombinant expression vector is introduced. The recombinant host cells include not only certain specific cells, but also the progeny of these cells.

In the present application, the term "pharmaceutical composition" generally refers to a preparation that is in a form that allows the biological activity of the active ingredient to be effective and does not contain additional components that are unacceptably toxic to the subject to whom the composition is administered.

In this application, the term "pharmaceutically acceptable carrier" generally includes pharmaceutically acceptable carriers, excipients or stabilizers that are non-toxic to cells or mammals exposed thereto at the doses and concentrations employed. Typically, a physiologically acceptable carrier is a pH buffered aqueous solution. Examples of physiologically acceptable carriers may include buffers, antioxidants, low molecular weight (less than about 10 residues) polypeptides, proteins, hydrophilic polymers, amino acids, monosaccharides, disaccharides and other carbohydrates, chelating agents, sugar alcohols, salt-forming counterions such as sodium; and/or nonionic surfactants.

As used herein, the terms "treatment", "treating", "treat", and the like, refer to administering an agent, or carrying out a procedure, for the purposes of obtaining an effect. The effect may be prophylactic in terms of completely or partially preventing a disease or symptom thereof and/or may be therapeutic in terms of effecting a partial or complete cure for a disease and/or symptom thereof. "Treatment", as used herein, may include treatment of a disease or disorder (*e.g*. cancer) in a mammal, particularly in a human, and includes: (a) preventing the disease or a symptom of a disease from occurring in a subject which may be predisposed to the disease but has not yet been diagnosed as having it (*e.g.,* including diseases that may be associated with or caused by a primary disease); (b) inhibiting the disease, *i.e.,* arresting its development; and (c) relieving the disease, *i.e.,* causing regression of the disease. Treating may refer to any indicia of success in the treatment or amelioration or prevention of a cancer, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms is based on one or more objective or subjective parameters; including the results of an examination by a physician. Accordingly, the term "treating" includes the administration of the antibodies or compositions or conjugates disclosed herein to prevent or delay, to alleviate, or to arrest or inhibit development of the symptoms or conditions associated with diseases (*e.g*. TL1A-mediated diseases).

As used herein, the term "TL1A" includes variants, isoforms, and species homologs of TL1A. Therefore, the antibodies of the invention can bind human TL1A and can cross-react with TL1A of species other than humans, for example, cynomolgus monkeys, rhesus monkeys. TL1A is also known as TNFSF15; TNF-like protein 1A; VEGI; TNFγβ. Human TL1A is called GeneID:9966 in EntrezGene and HGNC:11931 in HGNC. TL1A can be encoded by the gene called TNFSF15/TL1A. The complete amino acid sequence of an exemplary human TL1A has Swiss-Prot accession number O95150 (TNFSF15HUMAN) and its sequence is as follows:

MAEDLGLSFGETASVEMLPEHGSCRPKARSSSARWALTCCLVLLPFLAGLTTYLLVSQLRAQGEACVQ
FQALKGQEFAPSHQQVYAPLRADGDKPRAHLTVVRQTPTQHFKNQFPALHWEHELGLAFTKNRMNYTN
KFLLIPESGDYFIYSQVTFRGMTSECSEIRQAGRPNKPDSITVVITKVTDSYPEPTQLLMGTKSVCEV
GSNWFQPIYLGAMFSLQEGDKLMVNVSDISLVDYTKEDKTFFGAFLL (SEQ ID NO: 68)

As used herein, an "autoimmune disease" is a disease or condition that originates from and targets an individual's own tissues. Examples of autoimmune diseases or disorders include, but are not limited to, inflammatory responses such as inflammatory skin diseases including psoriasis and dermatitis (*e.g*., atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (*e.g*., Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, inflammatory myopathies, interstitial lung disease, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia); myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia, *etc.*

As used herein, the term "inflammatory disease" is to be understood in its broadest sense and includes inflammatory diseases and chronic inflammation-promoting diseases. For example, inflammatory disease refers to those diseases or conditions that are characterized by one or more of the signs of pain (dolor, from the generation of noxious substances and the stimulation of nerves), heat (calor, from vasodilatation), redness (rubor, from vasodilatation and increased blood flow), swelling (tumor, from excessive inflow or restricted outflow of fluid), and loss of function (functio laesa, which may be partial or complete, temporary or permanent). Inflammation takes many forms and includes, but is not limited to, inflammation that is one or more of the following: acute, adhesive, atrophic, catarrhal, chronic, cirrhotic, diffuse, disseminated, exudative, fibrinous, fibrosing, focal, granulomatous, hyperplastic, hypertrophic, interstitial, metastatic, necrotic, obliterative, parenchymatous, plastic, productive, proliferous, pseudomembranous, purulent, sclerosing, seroplastic, serous, simple, specific, subacute, suppurative, toxic, traumatic, and/or ulcerative. Inflammatory disorders further include, without being limited to those affecting the blood vessels (polyarteritis); joints (arthritis: temporal, crystalline, osteo-, psoriatic, reactive, rheumatoid, Reiter's); gastrointestinal tract (inflammatory bowel disease, Crohn's disease, ulcerative colitis, *etc*.); skin (dermatitis, psoriasis, *etc*.); allergic airway diseases (asthma, *etc*.); or those affecting multiple organs and tissues (systemic lupus erythematosus). In addition, chronic inflammation contributes to various diseases, such as cardiovascular disease, cancer, diabetes, chronic kidney disease, fibrosis (pulmonary fibrosis, *etc*.), chronic obstructive pulmonary disease, acne scarring, atherosclerosis, and non-alcoholic fatty liver disease as well as autoimmune diseases and neurodegenerative diseases (Alzheimer's disease, Parkinson's disease, *etc*.), these diseases promoted by chronic inflammation are also included in the definition of inflammatory diseases.

As used herein, "fibrosis" refers to the formation of excessive fibrous connective tissue due to excessive deposition of extracellular matrix components, such as collagen. The cellular and molecular mechanisms of fibrosis are described in Wynn, J. Pathol. (2008) 214(2):199210, and Wynn and Ramalingam, Nature Medicine (2012) 18:10281040, which are incorporated herein by reference in their entirety. As used herein, "fibrotic disease" refers to a disease/condition characterized by fibrosis, including but not limited to subretinal fibrosis (*e*.*g*., associated with macular degeneration (*e.g*., wet age-related macular degeneration (AMD)), nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

The term "effective amount" as used herein means the amount that, when administered to a subject for treating a disease, is sufficient to effect treatment for that disease.

As used herein, singular forms "a", "and," and "the" include plural referents unless the context clearly indicates otherwise. Thus, for example, reference to "an antibody" includes a plurality of antibodies and reference to "an antibody" in some embodiments includes multiple antibodies, and so forth.

Unless indicated or defined otherwise, the term "comprise", "include", "contain" and "have", should be understood to imply the inclusion of a stated elements or step or group of elements or steps but not the exclusion of any other element or step or group of elements or steps.

In this application, the term "about" generally refers to a variation within the range of 0.5%-10% above or below the specified value, such as a variation within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10%.

### Antigen-binding protein or antigen-binding fragment thereof that binds to TL1A

In a first aspect, the invention provides an antigen-binding protein or antigen-binding fragment thereof that binds to TL1A, wherein the antigen-binding protein or antigen-binding fragment thereof binds to an epitope in TL1A, the epitope comprising one or more amino acid residues selected from the group consisting of: R103, G124, M196, Y238, T239, K240, E241, H118, E120, H121, E122, L123, V102 and T105, wherein the amino acid positions are numbered according to the amino acid sequence of TL1A as set forth in SEQ ID NO: 68.

In some embodiments, the epitope comprises R103, G124, Y238, T239, E120, V102. In a preferred embodiment, the epitope further comprises one or more amino acid residues selected from the group consisting of M196, K240, E241, H118, H121, E122, L123 and T105.

In some embodiments, the epitope comprises amino acid residues R103, G124, M196, Y238, T239, K240, E241, H118, E120, H121, E122, L123, V102, and T105.

In some embodiments, the epitope consists of amino acid residues R103, G124, M196, Y238, T239, K240, E241, H118, E120, H121, E122, L123, V102, and T105.

In some embodiments of the TL1A-binding antigen-binding protein or antigen-binding fragment thereof disclosed herein, the antigen-binding protein comprises a heavy chain variable region (VH) and a light chain variable region (VL), wherein
(1) The VH comprises heavy chain CDR1, heavy chain CDR2 and heavy chain CDR3 of the heavy chain variable region having the amino acid sequence as set forth in SEQ ID NO: 2, and the VL comprises light chain CDR1, light chain CDR2 and light chain CDR3 of the light chain variable region having the amino acid sequence as set forth in SEQ ID NO: 1;
(2) The VH comprises heavy chain CDR1, heavy chain CDR2 and heavy chain CDR3 of the heavy chain variable region having the amino acid sequence as set forth in SEQ ID NO: 4, and the VL comprises light chain CDR1, light chain CDR2 and light chain CDR3 of the light chain variable region having the amino acid sequence as set forth in SEQ ID NO: 3;
(3) The VH comprises heavy chain CDR1, heavy chain CDR2 and heavy chain CDR3 of the heavy chain variable region having the amino acid sequence as set forth in SEQ ID NO: 6, and the VL comprises light chain CDR1, light chain CDR2 and light chain CDR3 of the light chain variable region having the amino acid sequence as set forth in SEQ ID NO: 5;
(4) The VH comprises heavy chain CDR1, heavy chain CDR2 and heavy chain CDR3 of the heavy chain variable region having the amino acid sequence as set forth in SEQ ID NO: 8, and the VL comprises light chain CDR1, light chain CDR2 and light chain CDR3 of the light chain variable region having the amino acid sequence as set forth in SEQ ID NO: 7;
(5) The VH comprises heavy chain CDR1, heavy chain CDR2 and heavy chain CDR3 of the heavy chain variable region having the amino acid sequence as set forth in SEQ ID NO: 10, and the VL comprises light chain CDR1, light chain CDR2 and light chain CDR3 of the light chain variable region having the amino acid sequence as set forth in SEQ ID NO: 9; or
(6) The VH comprises heavy chain CDR1, heavy chain CDR2 and heavy chain CDR3 of the heavy chain variable region having the amino acid sequence as set forth in SEQ ID NO: 12, and the VL comprises light chain CDR1, light chain CDR2 and light chain CDR3 of the light chain variable region having the amino acid sequence as set forth in SEQ ID NO: 11.

In certain embodiments, the heavy chain variable region has the amino acid sequence as set forth in SEQ ID NO: 10, and the light chain variable region has the amino acid sequence as set forth in SEQ ID NO: 9.In some embodiments, the VH comprises heavy chain CDRs 1-3 having the amino acid sequence as set forth in SEQ ID NO: 16-18 respectively, and the VL comprises light chain CDRs 1-3 having the amino acid sequence as set forth in SEQ ID NO: 13-15 respectively. In some embodiments, the VH comprises heavy chain CDRs 1-3 having the amino acid sequence as set forth in SEQ ID NO: 22-24 respectively, and the VL comprises light chain CDRs 1-3 having the amino acid sequence as set forth in SEQ ID NO: 19-21 respectively. In some embodiments, the VH comprises heavy chain CDRs 1-3 having the amino acid sequence as set forth in SEQ ID NO: 28-30 respectively, and the VL comprises light chain CDRs 1-3 having the amino acid sequence as set forth in SEQ ID NO: 25-27 respectively. In some embodiments, the VH comprises heavy chain CDRs 1-3 having the amino acid sequence as set forth in SEQ ID NO: 34-36 respectively, and the VL comprises light chain CDRs 1-3 having the amino acid sequence as set forth in SEQ ID NO: 31-33 respectively. In some embodiments, the VH comprises heavy chain CDRs 1-3 having the amino acid sequence as set forth in SEQ ID NO: 40-42 respectively, and the VL comprises light chain CDRs 1-3 having the amino acid sequence as set forth in SEQ ID NO: 37-39 respectively. In some embodiments, the VH comprises heavy chain CDRs 1-3 having the amino acid sequence as set forth in SEQ ID NO: 46-48 respectively, and the VL comprises light chain CDRs 1-3 having the amino acid sequence as set forth in SEQ ID NO: 43-45 respectively.

In certain embodiments, the VH CDRs 1-3 have the amino acid sequences as set forth in SEQ ID NO: 40-42, respectively, and the VL CDRs have the amino acid sequences as set forth in SEQ ID NO: 37-39, respectively.

In some embodiments, CDR sequences are defined according to Kabat definition. In some embodiments, CDR sequences are defined according to Chothia definition. In some embodiments, CDR sequences are defined according to Combined definition. In some embodiments, CDR sequences are defined according to AbM definition. In some embodiments, CDR sequences are defined according to CONTACT definition.

In a preferred embodiment, the CDR sequences are defined according to Kabat definition rules, and the VH comprises heavy chain CDRs 1-3 having the amino acid sequence as set forth in SEQ ID NO: 16-18 respectively, and the VL comprises light chain CDRs 1-3 having the amino acid sequence as set forth in SEQ ID NO: 13-15 respectively; the VH comprises heavy chain CDRs 1-3 having the amino acid sequence as set forth in SEQ ID NO: 22-24 respectively, and the VL comprises light chain CDRs 1-3 having the amino acid sequence as set forth in SEQ ID NO: 19-21 respectively; the VH comprises heavy chain CDRs 1-3 having the amino acid sequence as set forth in SEQ ID NO: 28-30 respectively, and the VL comprises light chain CDRs 1-3 having the amino acid sequence as set forth in SEQ ID NO: 25-27 respectively; the VH comprises heavy chain CDRs 1-3 having the amino acid sequence as set forth in SEQ ID NO: 34-36 respectively, and the VL comprises light chain CDRs 1-3 having the amino acid sequence as set forth in SEQ ID NO: 31-33 respectively; the VH comprises heavy chain CDRs 1-3 having the amino acid sequence as set forth in SEQ ID NO: 40-42 respectively, and the VL comprises light chain CDRs 1-3 having the amino acid sequence as set forth in SEQ ID NO: 37-39 respectively; the VH comprises heavy chain CDRs 1-3 having the amino acid sequence as set forth in SEQ ID NO: 46-48 respectively, and the VL comprises light chain CDRs 1-3 having the amino acid sequence as set forth in SEQ ID NO: 43-45 respectively.

In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 2 and the VL comprises an amino acid sequence having at least 80%, at least 85%, at least 90%, at least 95%, at least 96%, at least 97%, at least 98%, at least 99%, or 100% sequence identity to SEQ ID NO: 1. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 4 and the VL comprises an amino acid sequence having at least 80%, at least 85%, at least 90%, at least 95%, at least 96%, at least 97%, at least 98%, at least 99%, or 100% sequence identity to SEQ ID NO: 3. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 6 and the VL comprises an amino acid sequence having at least 80%, at least 85%, at least 90%, at least 95%, at least 96%, at least 97%, at least 98%, at least 99%, or 100% sequence identity to SEQ ID NO: 5. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 8 and the VL comprises an amino acid sequence having at least 80%, at least 85%, at least 90%, at least 95%, at least 96%, at least 97%, at least 98%, at least 99%, or 100% sequence identity to SEQ ID NO: 7. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 10 and the VL comprises an amino acid sequence having at least 80%, at least 85%, at least 90%, at least 95%, at least 96%, at least 97%, at least 98%, at least 99%, or 100% sequence identity to SEQ ID NO: 9. In some embodiments, the VH comprises an amino acid sequence having at least 80%, at least 85%, at least 90%, at least 95%, at least 96%, at least 97%, at least 98%, at least 99%, or 100% sequence identity to SEQ ID NO: 12 and the VL comprises an amino acid sequence having at least 80%, at least 85%, at least 90%, at least 95%, at least 96%, at least 97%, at least 98%, at least 99%, or 100% sequence identity to SEQ ID NO: 11.

In certain embodiments, the VH comprises an amino acid sequence having at least 80%, at least 85%, at least 90%, at least 95%, at least 96%, at least 97%, at least 98%, at least 99%, or 100% sequence identity to SEQ ID NO: 10, and the VL comprises an amino acid sequence having at least 80%, at least 85%, at least 90%, at least 95%, at least 96%, at least 97%, at least 98%, at least 99%, or 100% sequence identity to SEQ ID NO: 9.

In some embodiments, the VH comprises a functional variant formed by insertion, deletion and/or substitution of one or more amino acids in the amino acid sequence set forth in any one of SEQ ID NO: 2, 4, 6, 8, 10 and 12, provided that the functional variant retains the ability to bind TL1A. In some embodiments, the VL comprises a functional variant formed by insertion, deletion and/or substitution of one or more amino acids in the amino acid sequence set forth in any one of SEQ ID NO: 1, 3, 5, 7, 9 and 11, provided that the functional variant retains the ability to bind TL1A.

The functional variant comprises or consists of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the parent polypeptide.

In the context of the functional variant, the number of the inserted, deleted and/or substituted amino acid is preferably no more than 40% of the total number of amino acids in the parent amino acid sequence, more preferably no more than 35%, more preferably 1-33%, and more preferably 5- 30%, more preferably 10-25%, more preferably 15-20%. For example, the number of the inserted, deleted and/or substituted amino acid can be 1-20, preferably 1-10, more preferably 1-7, still more preferably 1-5, and most preferably 1-2. In a preferred embodiment, the number of the inserted, deleted and/or substituted amino acid is 1, 2, 3, 4, 5, 6, or 7.

In some embodiments, the insertion, deletion and/or substitution can be performed at framework (FR) regions, *e.g.,* at FR1, FR2, FR3, and/or FR4.

In some embodiments, the substitution of one or more amino acid(s) can be conservative substitution of one or more amino acid(s). Such conservative substitutions preferably are substitutions in which one amino acid within the following groups (a)-(e) is substituted by another amino acid residue within the same group: (a) small aliphatic, nonpolar or slightly polar residues: Ala, Ser, Thr, Pro and Gly; (b) polar, negatively charged residues and their (uncharged) amides: Asp, Asn, Glu and Gln; (c) polar, positively charged residues: His, Arg and Lys; (d) large aliphatic, nonpolar residues: Met, Leu, Ile, Val and Cys; and (e) aromatic residues: Phe, Tyr and Trp.

Particularly preferred conservative substitutions are as follows: Ala into Gly or into Ser; Arg into Lys; Asn into Gln or into His; Asp into Glu; Cys into Ser; Gln into Asn; Glu into Asp; Gly into Ala or into Pro; His into Asn or into Gln; Ile into Leu or into Val; Leu into Ile or into Val; Lys into Arg, into Gln or into Glu; Met into Leu, into Tyr or into Ile; Phe into Met, into Leu or into Tyr; Ser into Thr; Thr into Ser; Trp into Tyr; Tyr into Trp; and/or Phe into Val, into Ile or into Leu.

In some embodiments, the VH comprises an amino acid sequence as set forth in SEQ ID NO: 2 and the VL comprises an amino acid sequence as set forth in SEQ ID NO: 1. In some embodiments, the VH comprises an amino acid sequence as set forth in SEQ ID NO: 4 and the VL comprises an amino acid sequence as set forth in SEQ ID NO: 3. In some embodiments, the VH comprises an amino acid sequence as set forth in SEQ ID NO: 6 and the VL comprises an amino acid sequence as set forth in SEQ ID NO: 5. In some embodiments, the VH comprises an amino acid sequence as set forth in SEQ ID NO: 8 and the VL comprises an amino acid sequence as set forth in SEQ ID NO: 7. In some embodiments, the VH comprises an amino acid sequence as set forth in SEQ ID NO: 10 and the VL comprises an amino acid sequence as set forth in SEQ ID NO: 9. In some embodiments, the VH comprises an amino acid sequence as set forth in SEQ ID NO: 12 and the VL comprises an amino acid sequence as set forth in SEQ ID NO: 11.

In certain embodiments, the VH comprises an amino acid sequence as set forth in SEQ ID NO: 10, and the VL comprises an amino acid sequence as set forth in SEQ ID NO: 9.

In some embodiments of the presently disclosed antigen-binding protein or antigen-binding fragment thereof that binds to TL1A, the antigen-binding protein is an antibody.

In some embodiments, the antibody is a monoclonal antibody, a bispecific antibody, or a multispecific antibody.

In some embodiments, the antibody is a bispecific antibody. A bispecific antibody comprises an antibody described herein that binds to TL1A, or an antigen-binding fragment thereof, and a second antigen-binding domain that binds to a second antigen. The second antigen can be selected from the group consisting of tumor-associated antigens, immune cell antigens, and immune checkpoint molecules.

In some embodiments, the second antigen is an immune cell antigen, such as a T cell antigen. In some embodiments, the T-cell antigen is selected from the group consisting of T cell receptor (TCR), CD3, CD4, CD8, CD16, CD25, CD28, CD38, CD44, CD62L, CD69, ICOS, 41-BB (CD137), and NKG2D or any combination thereof. In some embodiments, the T-cell antigen is CD3, and the second antigen binding region binds to any of γ chain, δ chain, ε chain, ζ chain and η chain of CD3.

In some embodiments, the second antigen is an immune checkpoint molecule. In some embodiments, the immune checkpoint molecule can be selected from PD-1, PD-L1, CTLA-4, CD4, CD40, CD80, CD86, B7-H3, LAG3, TIM-3, IDO1, *etc.*

In some embodiments, the antibody is a murine antibody, a chimeric antibody, a humanized antibody, or a fully human antibody.

In some embodiments, the antibodies of the invention may comprise an Fc region. The Fc region can be of any isotype, including but not limited to IgG1, IgG2, IgG3, and IgG4, and can contain one or more mutations or modifications. In one embodiment, the Fc region is or derived from the IgG1 isotype, optionally with one or more mutations or modifications. In one embodiment, the Fc region is a human IgG Fc. In one embodiment, the Fc region is human IgG1 Fc. In one embodiment, the Fc region is a murine IgG Fc. In one embodiment, the Fc region is a murine IgG1 Fc.

Based on the amino acid sequence of the constant region of the antibody heavy chain, immunoglobulin molecules can be divided into five categories (isotypes): IgA, IgD, IgE, IgG, and IgM, and can be further divided into different subtypes, such as IgG1, IgG2, IgG3, IgG4, IgA1, IgA2, *etc.* Based on the amino acid sequence of the light chain, the light chain of an antibody can be divided into lambda (λ) chain and kappa (κ) chain. The antibodies disclosed herein may be of any of the classes or subtypes described above.

In some embodiments, the antibody is of an isotype selected from IgG, IgA, IgM, IgE, and IgD. In some embodiments, the antibody is of a subtype selected from IgG1, IgG2, IgG3, and IgG4. In a preferred embodiment, the antibody is an IgG1 antibody.

In some embodiments, the antibody comprises a heavy chain (HC) and a light chain (LC), the heavy chain comprises an amino acid sequence having at least 80%, at least 85%, at least 90%, at least 95%, at least 96%, at least 97%, at least 98%, at least 99%, or 100% sequence identity to SEQ ID NO: 50 and the light chain comprises an amino acid sequence having at least 80%, at least 85%, at least 90%, at least 95%, at least 96%, at least 97%, at least 98%, at least 99%, or 100% sequence identity to SEQ ID NO: 49. In some embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 52 and the light chain comprises an amino acid sequence having at least 80%, at least 85%, at least 90%, at least 95%, at least 96%, at least 97%, at least 98%, at least 99%, or 100% sequence identity to SEQ ID NO: 51. In some embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 54 and the light chain comprises an amino acid sequence having at least 80%, at least 85%, at least 90%, at least 95%, at least 96%, at least 97%, at least 98%, at least 99%, or 100% sequence identity to SEQ ID NO: 53. In some embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 56 and the light chain comprises an amino acid sequence having at least 80%, at least 85%, at least 90%, at least 95%, at least 96%, at least 97%, at least 98%, at least 99%, or 100% sequence identity to SEQ ID NO: 55. In some embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 58 and the light chain comprises an amino acid sequence having at least 80%, at least 85%, at least 90%, at least 95%, at least 96%, at least 97%, at least 98%, at least 99%, or 100% sequence identity to SEQ ID NO: 57. In some embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 60 and the light chain comprises an amino acid sequence having at least 80%, at least 85%, at least 90%, at least 95%, at least 96%, at least 97%, at least 98%, at least 99%, or 100% sequence identity to SEQ ID NO: 59.

In certain embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 58, and the light chain comprises an amino acid sequence having at least 80%, at least 85%, at least 90%, at least 95%, at least 96%, at least 97%, at least 98%, at least 99%, or 100% sequence identity to SEQ ID NO: 57.

In some embodiments, the heavy chain comprises an amino acid sequence as set forth in SEQ ID NO: 50 and the light chain comprises an amino acid sequence as set forth in SEQ ID NO: 49. In some embodiments, the heavy chain comprises an amino acid sequence as set forth in SEQ ID NO: 52 and the light chain comprises an amino acid sequence as set forth in SEQ ID NO: 51. In some embodiments, the heavy chain comprises an amino acid sequence as set forth in SEQ ID NO: 54 and the light chain comprises an amino acid sequence as set forth in SEQ ID NO: 53. In some embodiments, the heavy chain comprises an amino acid sequence as set forth in SEQ ID NO: 56 and the light chain comprises an amino acid sequence as set forth in SEQ ID NO: 55. In some embodiments, the heavy chain comprises an amino acid sequence as set forth in SEQ ID NO: 58 and the light chain comprises an amino acid sequence as set forth in SEQ ID NO: 57. In some embodiments, the heavy chain comprises an amino acid sequence as set forth in SEQ ID NO: 60 and the light chain comprises an amino acid sequence as set forth in SEQ ID NO: 59.

In certain embodiments, the heavy chain comprises an amino acid sequence as set forth in SEQ ID NO: 58, and the light chain comprises an amino acid sequence as set forth in SEQ ID NO: 57.

In a related additional first aspect of the invention, the invention provides an isolated antibody or an antigen-binding fragment thereof that specifically binds to TL1A, wherein the isolated antibody comprises an immunoglobulin heavy chain (HC) comprising a heavy chain variable region (VH) comprising a heavy chain CDR1, a heavy chain CDR2, and a heavy chain CDR3 (VH CDRs 1-3), and an immunoglobulin light chain (LC) comprising a variable region (VL) comprising a light chain CDR1, a light chain CDR2, and a light chain CDR3 (VL CDRs 1-3), wherein the VH CDRs 1-3 have the amino acid sequences as set forth in SEQ ID NO: 40-42, respectively, and the VL CDRs 1-3 have the amino acid sequences as set forth in SEQ ID NO: 37-39, respectively.

In certain embodiments, the VH comprises an amino acid sequence having at least 80%, at least 85%, at least 90%, at least 95%, at least 96%, at least 97%, at least 98%, at least 99%, or 100% amino acid sequence identity to SEQ ID NO: 10, and the VL comprises an amino acid sequence having at least 80%, at least 85%, at least 90%, at least 95%, at least 96%, at least 97%, at least 98%, at least 99%, or 100% amino acid sequence identity to SEQ ID NO: 9.

In certain embodiments, the VH comprises an amino acid sequence as set forth in SEQ ID NO: 10, and the VL comprises an amino acid sequence as set forth in SEQ ID NO: 9.

In certain embodiments, the heavy chain comprises an amino acid sequence having at least 80%, at least 85%, at least 90%, at least 95%, at least 96%, at least 97%, at least 98%, at least 99%, or 100% amino acid sequence identity to SEQ ID NO: 58, and the light chain comprises an amino acid sequence having at least 80%, at least 85%, at least 90%, at least 95%, at least 96%, at least 97%, at least 98%, at least 99%, or 100% amino acid sequence identity to SEQ ID NO: 57.

In certain embodiments, the heavy chain comprises an amino acid sequence set forth in SEQ ID NO: 58, and the light chain comprises an amino acid sequence set forth in SEQ ID NO: 57.

In certain embodiments, the isolated antibody is a monoclonal antibody, a bispecific antibody or a multispecific antibody.

In certain embodiments, the isolated antibody is a murine antibody, a chimeric antibody, a humanized antibody, or a fully human antibody.

In certain embodiments, the isolated antibody or antigen-binding fragment thereof binds to an epitope in TL1A, wherein the epitope comprises one or more amino acid residues selected from the group consisting of: R103 , G124, M196, Q193, Y238, T239, K240, E241, H118, E120, H121, E122, L123, V102 and T105, and wherein the amino acid positions are numbered according to the amino acid sequence of TL1A as set forth in SEQ ID NO: 68.

In certain embodiments, the epitope consists of amino acid residues R103, G124, M196, Y238, T239, K240, E241, H118, E120, H121, E122, L123, V102 and T105.

The antibodies disclosed herein can be intact antibodies or antigen-binding fragments thereof. In some embodiments, the antibodies disclosed herein are single-arm antibodies.

In some embodiments, the single-arm antibody comprises a heavy chain (HC) and a light chain (LC), and wherein:
(1) The heavy chain comprises an amino acid sequence having at least 80%, at least 85%, at least 90%, at least 95%, at least 96%, at least 97%, at least 98%, at least 99%, or 100% sequence identity to SEQ ID NO: 62 and the light chain comprises an amino acid sequence having at least 80%, at least 85%, at least 90%, at least 95%, at least 96%, at least 97%, at least 98%, at least 99%, or 100% sequence identity to SEQ ID NO: 49;
(2) The heavy chain comprises an amino acid sequence having at least 80%, at least 85%, at least 90%, at least 95%, at least 96%, at least 97%, at least 98%, at least 99%, or 100% sequence identity to SEQ ID NO: 63 and the light chain comprises an amino acid sequence having at least 80%, at least 85%, at least 90%, at least 95%, at least 96%, at least 97%, at least 98%, at least 99%, or 100% sequence identity to SEQ ID NO: 51;
(3) The heavy chain comprises an amino acid sequence having at least 80%, at least 85%, at least 90%, at least 95%, at least 96%, at least 97%, at least 98%, at least 99%, or 100% sequence identity to SEQ ID NO: 64 and the light chain comprises an amino acid sequence having at least 80%, at least 85%, at least 90%, at least 95%, at least 96%, at least 97%, at least 98%, at least 99%, or 100% sequence identity to SEQ ID NO: 53;
(4) The heavy chain comprises an amino acid sequence having at least 80%, at least 85%, at least 90%, at least 95%, at least 96%, at least 97%, at least 98%, at least 99%, or 100% sequence identity to SEQ ID NO: 65 and the light chain comprises an amino acid sequence having at least 80%, at least 85%, at least 90%, at least 95%, at least 96%, at least 97%, at least 98%, at least 99%, or 100% sequence identity to SEQ ID NO: 55;
(5) The heavy chain comprises an amino acid sequence having at least 80%, at least 85%, at least 90%, at least 95%, at least 96%, at least 97%, at least 98%, at least 99%, or 100% sequence identity to SEQ ID NO: 66 and the light chain comprises an amino acid sequence having at least 80%, at least 85%, at least 90%, at least 95%, at least 96%, at least 97%, at least 98%, at least 99%, or 100% sequence identity to SEQ ID NO: 57; or
(6) The heavy chain comprises an amino acid sequence having at least 80%, at least 85%, at least 90%, at least 95%, at least 96%, at least 97%, at least 98%, at least 99%, or 100% sequence identity to SEQ ID NO: 67 and the light chain comprises an amino acid sequence having at least 80%, at least 85%, at least 90%, at least 95%, at least 96%, at least 97%, at least 98%, at least 99%, or 100% sequence identity to SEQ ID NO: 59, and the single-arm antibody further comprises the Fc chain of SEQ ID NO: 61.

In certain embodiments, the heavy chain comprises an amino acid sequence having at least 80%, at least 85%, at least 90%, at least 95%, at least 96%, at least 97%, at least 98%, at least 99%, or 100% sequence identity to SEQ ID NO: 66 and the light chain comprises an amino acid sequence having at least 80%, at least 85%, at least 90%, at least 95%, at least 96%, at least 97%, at least 98%, at least 99%, or 100% sequence identity to SEQ ID NO: 57.

In some embodiments, the heavy chain comprises an amino acid sequence as set forth in SEQ ID NO: 62 and the light chain comprises an amino acid sequence as set forth in SEQ ID NO: 49. In some embodiments, the heavy chain comprises an amino acid sequence as set forth in SEQ ID NO: 63 and the light chain comprises an amino acid sequence as set forth in SEQ ID NO: 51. In some embodiments, the heavy chain comprises an amino acid sequence as set forth in SEQ ID NO: 64 and the light chain comprises an amino acid sequence as set forth in SEQ ID NO: 53. In some embodiments, the heavy chain comprises an amino acid sequence as set forth in SEQ ID NO: 65 and the light chain comprises an amino acid sequence as set forth in SEQ ID NO: 55. In some embodiments, the heavy chain comprises an amino acid sequence as set forth in SEQ ID NO: 66 and the light chain comprises an amino acid sequence as set forth in SEQ ID NO: 57. In some embodiments, the heavy chain comprises an amino acid sequence as set forth in SEQ ID NO: 67 and the light chain comprises an amino acid sequence as set forth in SEQ ID NO: 59.

In certain embodiments, the heavy chain comprises an amino acid sequence as set forth in SEQ ID NO: 58, and the light chain comprises an amino acid sequence as set forth in SEQ ID NO: 57.

The Fc region described herein may comprise one or more modifications, such as one or more amino acid mutations, insertions, or deletions that enhance antibody affinity or biological activity, increase Fc dimerization, enhance antibody stability, extend antibody half-life, reduce effector function, reduce the immunogenicity of the antibody, or reduce post-translational modifications of the antibody; for example, such modifications include mutations that enhance binding to FcRn and/or effector function-silencing mutations.

In some embodiments, the Fc region can contain modifications that increase dimerization. In some embodiments, the Fc region of the antibodies disclosed herein contains a knob-in-hole mutation.

Detailed descriptions of the knob and hole concept can be found, for example, in U.S. Patent Nos. 5,731,168 and 7,186,076; and Ridgway et al., Protein Engineering, Design and Selection, 1996, 9(7):617-621, Atwell et al., JMol Biol, 1997, 270(1): 26-35; Merchant et al., Nat Biotechnol, 1998, 16: 677-681; and Carter, J. Immunological Methods, 2001, 24(1-2): 7-15. Briefly, a knob can be created at the CH3 domain interface of the first IgG Fc chain by replacing a smaller one with a larger amino acid side chain (e.g., T366W); and a hole can be created in a juxtaposed position at the CH3 interface of the second IgG Fc chain by replacing the larger one with a smaller amino acid side chain (e.g., Y407V).

The amino acid residues forming the knob are typically naturally occurring amino acid residues and are selected from the group consisting of arginine (R), phenylalanine (F), tyrosine (Y) and tryptophan (W). In some preferred embodiments, the amino acid residues are tryptophan and tyrosine. In one embodiment, the original residues forming the konb have a small side chain volume, such as alanine, asparagine, aspartic acid, glycine, serine, threonine, or valine. Exemplary amino acid substitutions in the CH3 domain that form a knob include, but are not limited to, T366W, T366Y, or F405W substitutions.

The amino acid residues forming the hole are generally naturally occurring amino acid residues and are selected from the group consisting of alanine (A), serine (S), threonine (T) and valine (V). In some preferred embodiments, the original residues forming the hole have a large side chain volume, such as tyrosine, arginine, phenylalanine, or tryptophan. Exemplary amino acid substitutions in the CH3 domain that create a hole include, but are not limited to, T366S, L368A, F405A, Y407A, Y407T, and Y407V substitutions. In some embodiments, the konb contains the T366W substitution and the hole contains the T366S/L368A/Y407V substitution.

In general, the preferred Fc domain for use herein is a human IgG domain, and typically IgG1 or IgG4. In some cases, such as when effector function is undesirable, IgG4 is used, and in some cases, the S228P variant is contained in the hinge domain as this prevents arm swapping.

The Fc region interacts with a number of receptors or ligands, including but not limited to Fc receptors (eg, FcyRI, FcyRIIA, FcyRIIIA), complement protein Clq, and other molecules such as protein A and protein G. These interactions are required for multiple effector functions and downstream signaling events, including but not limited to antibody-dependent cell-mediated cytotoxicity (ADCC), antibody-dependent cellular phagocytosis (ADCP), and complement-dependent cytotoxicity (CDC). The Fc region can also interact with FcRn. The circulating half-life of an antibody or antibody Fc region conjugate can be affected by modulating the Fc region-FcRn interaction.

An increase in the circulating half-life of an antibody or antibody Fc region conjugate may be achieved through increased binding to FcRn and result in improved efficacy, reduced dosage or frequency of administration, or improved delivery to the target.

In one embodiment, the Fc region has increased binding to FcRn. Fc region variants with increased affinity for FcRn have longer serum half-lives, and such molecules would have useful applications in methods of treating mammals where long-lasting systemic half-lives of the administered antibody Fc region conjugates are required, for example, to treat a chronic disease or condition.

For example, it has been reported that modifications to the Fc region that can increase binding to FcRn include one or more amino acid changes at amino acid positions 238, 256, 265, 272, 286, 303, 305, 307, 311, 312, 317, 340, 356, 360, 362, 376, 378, 380, 382, 413, 424 and/or 434 (see US7371826). Studies have also shown that modifications to the Fc region that can increase binding to FcRn include one or more mutations at amino acid residues Thr 250, Met 252, Ser 254, Thr 256, Thr 307, Glu 380, Met 428, His 433 and Asn 434 (see CN 105899534). For example, Fc region modifications that increase binding to FcRn include the combination of mutations M428L and N434S. As another example, Fc region modifications capable of increasing binding to FcRn include a combination of mutations M252Y, S254T, and T256E (see Dall' Acqua, W F. et al. J. Biol. Chem. 281 (2006) 23514-23524).

In one embodiment, the Fc region has reduced effector function, such as reduced ADCC, ADCP, CDC and/or Clq, FcyRI, FcyRII or FcyRIIIA binding. For example, the Fc region may be of an IgG1 isotype, or a non-IgG1 type, such as IgG2, IgG3, or IgG4, which has been mutated such that the ability to mediate effector functions is reduced or even eliminated. Such mutations have been described, for example, in Dall'Acqua WF et al., J Immunol. 177(2):1129-1138 (2006) and Hezareh M, J Virol.; 75(24):12161-12168 (2001). For example, the Fc region may comprise an amino acid sequence with one or more of the following amino acid substitutions compared to a wild-type sequence: E233P, L234A, L234F, L235A, L235E, G237A, N297A, N297D, P331S, and P329G.

In one embodiment, the Fc region contains mutations that remove receptor sites for Asn-linked glycosylation or mutations that are otherwise manipulated to alter glycosylation properties. For example, in the IgG1 Fc region, the N297Q mutation can be used to remove Asn-linked glycosylation sites. Thus, in a specific embodiment, the Fc region comprises an IgG1 sequence with the N297Q mutation.

In a further embodiment, the Fc region is glycoengineered to reduce fucose and thus enhance ADCC, for example by adding compounds to the culture medium during antibody production, as described in US2009317869 or van Berkel et al. (2010) Biotechnol. Bioeng. 105:350, or by using FUT8 knockout cells, for example as described in Yamane-Ohnuki et al. (2004) Biotechnol. Bioeng 87:614. Alternatively, ADCC can be optimized using the method described by Umaña et al. (1999) Nature Biotech 17:176. In another embodiment, the Fc region is engineered to enhance complement activation, for example as described in Natsume et al. (2009) Cancer Sci. 100:2411.

The antigen-binding fragment may be any fragment of an antibody that retains the ability to specifically bind to TL1A. Examples of antigen-binding fragments include, but are not limited to: Fab fragments; F(ab')2 fragments; Fab' fragments; Fd fragments; Fd' fragments; Fv fragments; scFv fragments; dAb fragments; individual complementarity determining regions (CDRs); Nanobodies; linear antibodies consisting of a pair of tandem Fd fragments (VH-CH1-VH-CH1) and modified forms of any of the foregoing fragments that retain antigen-binding activity.

In some embodiments, the antigen-binding fragment is selected from Fab, Fab', F(ab')2, Fv, scFv, and ds-scFv. In a preferred embodiment, the antigen-binding fragment is a Fab. In another preferred embodiment, the antigen-binding fragment is an Fv. In another preferred embodiment, the antigen-binding fragment is a scFv.

In some embodiments, the antibody of the invention forms a complex of smaller size when bound to TL1A compared to an anti-TL1A antibody that does not bind to the epitope described herein. In some embodiments, the complex formed by the antibody of the invention with TL1A has a particle size of less than 33 nm, less than 30 nm, less than 25 nm, less than 20 nm, as detected by DLS. In some embodiments, the particle size of the complex formed by the antibody of the invention and TL1A is between 17-20 nm, between 18-20 nm, between 18-19.5 nm, as detected by DLS. In some embodiments, the particle size of the complex formed by the antibody of the invention and TL1A is about 18.0 nm, about 18.1 nm, about 18.2 nm, about 18.3 nm, about 18.4 nm, about 18.5 nm, about 18.6 nm, about 18.7 nm, about 18.8 nm, about 18.9 nm, about 19.0 nm, about 19.1 nm, about 19.2 nm, about 19.3 nm, about 19.4 nm or about 19.5 nm. In some embodiments, the antibody of the invention forms a uniformly dispersed small particle with TL1A, as observed by negative stain electron microscopy experiments.

In some embodiments, the antibody of the invention binds to TL1A at a 1:1 ratio to form an antigen-antibody complex. In some embodiments, the antibody of the invention comprises two heavy chains and two light chains. In some embodiments, the Fc valency of the complex formed by the antibody of the invention with TL1A is <5. In some embodiments, the Fc valency of the complex formed by the antibody of the invention with TL1A is <4. In some embodiments, the Fc valency of the complex formed by the antibody of the invention with TL1A is <3. In some embodiments, the Fc valency of the complex formed by the antibody of the invention with TL1A is 2.

In some embodiments, the antibody of the invention has pH-dependent binding to TL1A. In some embodiments, the antibody of the invention binds strongly to TL1A under neutral pH conditions and weakly to TL1A under acidic conditions. In some embodiments, the binding of the antibody of the invention to TL1A in endosomes is significantly reduced compared to binding to TL1A in serum. In some embodiments, the binding of the antibody to TL1A under neutral pH conditions is stronger than the binding to TL1A under acidic conditions. In some embodiments, the binding of the antibody to TL1A at pH7.4 is stronger than the binding to TL1A at pH6.0. In some embodiments, the binding of the antibody to TL1A at pH7.4 is stronger than the binding to TL1A at pH5.8. In some embodiments, the binding of the antibody to TL1A at pH7.4 is stronger than the binding to TL1A at pH5.6. In some embodiments, the binding of the antibody to TL1A at pH6.0 is stronger than the binding to TL1A at pH5.8. In some embodiments, the binding of the antibody to TL1A at pH5.8 is stronger than the binding to TL1A at pH5.6. In some embodiments, the antibody of the invention has a binding affinity constant (KD) of 10⁻⁹M for TL1A at pH 7.4 and does not bind to TL1A at pH 5.6.

In some embodiments, the antibody of the invention has low immunogenicity. In some embodiments, the antibody of the invention has a high safety profile. In some embodiments, the antibody of the invention can accelerate the clearance of TL1A in lysosomes, thereby reducing the level of TL1A in the body. In some embodiments, the antibody of the invention has increased blood circulation level. In some embodiments, the antibody of the invention has an extended half-life.

In some embodiments, the antibody of the invention is capable of binding soluble TL1A. In some embodiments, the antibody of the invention is capable of binding membrane-bound TL1A. In some embodiments, the antibody of the invention is capable of inhibiting T cell activation by exogenous TL1A. In some embodiments, the antibody of the invention is capable of inhibiting the activation of immune cells by endogenous TL1A.

### Nucleic acid

In yet another aspect, the invention provides a nucleic acid molecule comprising a nucleotide sequence encoding the antigen-binding protein disclosed in the invention or the antigen-binding fragment thereof.

The term "nucleic acid" includes single-stranded and double-stranded nucleotide polymers. The nucleic acid may be a ribonucleotide or a deoxyribonucleotide or a modified form of either type of nucleotide. The modifications include base modifications such as bromouridine and inosine derivatives, ribose modifications such as 2',3'-dideoxyribose, and internucleotide bond modifications such as phosphorothioate, phosphorodithioates, selenophosphates, diselenophosphates, phenylthiophosphates, aniline phosphates and phosphoramidates.

For example, the invention provides nucleic acid molecules encoding any of the heavy chain variable region sequences disclosed herein. The invention further provides nucleic acid molecules that are at least 90%, at least 95%, at least 96%, at least 97%, at least 98%, or at least 99% identical to the nucleic acid encoding any of the heavy chain variable region sequences disclosed herein.

For example, the invention provides nucleic acid molecules encoding any of the light chain variable region sequences disclosed herein. The invention further provides nucleic acid molecules that are at least 90%, at least 95%, at least 96%, at least 97%, at least 98%, or at least 99% identical to the nucleic acid encoding any of the light chain variable region sequences disclosed herein.

For example, the invention provides nucleic acid molecules encoding heavy chain variable region sequences that comprise the CDR sequences of any of the heavy chain variable region sequences disclosed herein. The invention further provides nucleic acid molecules encoding heavy chain variable region sequences, which comprise CDR sequences that are at least 90%, at least 95%, and at least 96%, at least 97%, at least 98% or at least 99% identical to the CDR sequences of any heavy chain variable region sequence disclosed herein.

For example, the invention provides nucleic acid molecules encoding light chain variable region sequences that comprise the CDR sequences of any of the light chain variable region sequences disclosed herein. The invention further provides nucleic acid molecules encoding light chain variable region sequences, which comprise CDR sequences that are at least 90%, at least 95%, and at least 96%, at least 97%, at least 98% or at least 99% identical to the CDR sequences of any light chain variable region sequence disclosed herein.

In some embodiments, the nucleic acid is ribonucleic acid (RNA) or deoxyribonucleic acid (DNA). In some embodiments, ribonucleic acid (RNA) comprising a nucleotide sequence encoding the antibody disclosed herein is provided. In some embodiments, the invention provides deoxyribonucleic acid (DNA) comprising a deoxynucleotide sequence encoding the antibody disclosed herein.

In some embodiments, deoxyribonucleic acid (DNA) can be introduced into human cells *in vivo.* In some embodiments, the deoxyribonucleic acid (DNA) of the invention is contained in a vector or delivery agent. In some embodiments, the deoxyribonucleic acid (DNA) of the invention is integrated into the genome of the cell.

In some embodiments, ribonucleic acid (RNA) can be introduced into human cells *in vivo.* In some embodiments, the ribonucleic acid (RNA) of the invention is contained in a vector or delivery agent.

### Vector

In another aspect, the invention provides a vector comprising the nucleic acid disclosed herein encoding the TL1A antigen-binding protein or the antigen-binding fragment thereof disclosed herein.

In some embodiments, the vector is an expression vector capable of expressing a polypeptide comprising the heavy or light chain variable region of an antigen-binding protein. For example, the present invention provides expression vectors comprising any of the nucleic acid molecules described above.

Any vector may be suitable for the present disclosure. In some embodiments, the vector is a viral vector. In some embodiments, the vector is a retroviral vector, a DNA vector, a murine leukemia virus vector, an SFG vector, a plasmid, a RNA vector, an adenoviral vector, a baculoviral vector, an Epstein Barr viral vector, a papovaviral vector, a vaccinia viral vector, a herpes simplex viral vector, an adenovirus associated vector (AAV), a lentiviral vector, or any combination thereof. Suitable exemplary vectors include *e*.*g*., pBY, pGAR, pBABE-puro, pBABE-neo largeTcDNA, pBABE-hygro-hTERT, pMKO.1 GFP, MSCV-IRES-GFP, pMSCV PIG (Puro IRES GFP empty plasmid), pMSCV-loxp-dsRed-loxp-eGFP-Puro-WPRE, MSCV IRES Luciferase, pMIG, MDH1-PGK-GFP_2.0, TtRMPVIR, pMSCV-IRES-mCherry FP, pRetroX GFP T2ACre, pRXTN, pLncEXP, and pLXIN-Luc.

A recombinant expression vector may be any suitable recombinant expression vector. Suitable vectors comprise those designed for propagation and expansion or for expression or both, such as plasmids and viruses. For example, a vector may be selected from the pUC series (Fermentas Life Sciences, Glen Burnie, Md.), the pBluescript series (Stratagene, LaJolla, Calif), the pET series (Novagen, Madison, Wis.), the pGEX series (Pharmacia Biotech, Uppsala, Sweden), and the pEX series (Clontech, Palo Alto, Calif). Bacteriophage vectors, such as λGT10, λGT11, λZapII (Stratagene), λEMBL4, and λNM1149, also may be used. Examples of plant expression vectors useful in the context of the disclosure comprise pBY, pBI01, pBI101.2, pBI101.3, pBI121 and pBIN19 (Clontech). Examples of animal expression vectors useful in the context of the disclosure comprise pcDNA, pEUK-Cl, pMAM, and pMAMneo (Clontech).

Recombinant expression vectors may be prepared using standard recombinant DNA techniques described in, for example, Sambrook et al., Molecular Cloning: A Laboratory Manual, 3rd ed., Cold Spring Harbor Press, Cold Spring Harbor, N.Y. 2001; and Ausubel et al., Current Protocols in Molecular Biology, Greene Publishing Associates and John Wiley & Sons, NY, 1994. Constructs of expression vectors, which are circular or linear, may be prepared to contain a replication system functional in a prokaryotic or eukaryotic host cell. Replication systems may be derived, *e.g.,* from ColEl, 2µ plasmid, λ, SV40, bovine papilloma virus, and the like.

For example, the vector can be an adenoviral vector containing nucleotide sequences encoding the antibodies disclosed herein. The vector can be administered into a subject and then enter the subject's cells *in vivo,* thereby integrating the nucleotide sequences encoding the antibodies disclosed herein into the genome of the cell, which then expresses the antibodies disclosed herein.

### Host cell

In another aspect, the invention provides a host cell comprising the nucleic acid disclosed herein or the vector disclosed herein.

Any cell may be used as a host cell for the nucleic acids or the vectors of the present disclosure. In some embodiments, the cell can be a prokaryotic cell, fungal cell, yeast cell, or higher eukaryotic cells such as a mammalian cell. Suitable prokaryotic cells include, without limitation, eubacteria, such as Gram-negative or Gram-positive organisms, for example, *Enterobactehaceae* such as *Escherichia, e.g., E. coli; Enterobacter; Erwinia; Klebsiella; Proteus; Salmonella, e.g., Salmonella typhimurium; Serratia, e.g., Serratia marcescans,* and *Shigella; Bacilli* such as *B. subtilis* and *B. licheniformis*; *Pseudomonas* such as *P. aeruginosa*; and *Streptomyces.* In some embodiments, the cell is a human cell. In some embodiments, the cell is an immune cell. In some embodiments, host cells include, for example, CHO cells, such as CHOS cells and CHO-K1 cells, or HEK293 cells, such as HEK293A, HEK293T and HEK293FS.

The host cells of the invention are prepared by introducing the vector disclosed herein or the nucleic acid disclosed herein *in vitro* or *ex vivo.* The host cells of the invention can be administered to a subject and express the antibodies disclosed herein *in vivo.*

### Preparation method

In another aspect, the invention provides a method for preparing the antigen-binding protein disclosed herein or the antigen-binding fragment thereof, which includes culturing the host cells provided by the present disclosure under conditions for expression of the antigen-binding protein or antigen-binding fragment thereof; and isolating the antigen-binding protein or antigen-binding fragment thereof from the culture and/or culture supernatant of the host cells.

Any method suitable for producing antigen-binding proteins can be used to produce the antigen-binding proteins of the present application. For example, antibodies can be prepared using hybridoma methods, such as those described in Kohler and Milstein, Nature, 256:495 (1975). In the hybridoma approach, a mouse, hamster, or other suitable host animal is typically immunized with an immune agent to elicit lymphocytes that produce or are capable of producing antibodies that will specifically bind the immune agent. Alternatively, lymphocytes can be immunized *in vitro.*

Immunizing agents typically include protein antigens, fragments thereof, or fusion proteins thereof. In this application, any suitable form of TL1A can be used as an immunizing agent (antigen) for generating non-human antibodies specific for TL1A and screening the biological activity of the antibodies. Immunizing agent can be full-length mature human TL1A, including native homodimers, or single/multiple epitope-containing peptides. Immunizing agent may be used alone or in combination with one or more immunogenicity enhancing agents known in the art. Typically, if cells of human origin are desired, peripheral blood lymphocytes are used; if non-human mammalian sources are desired, splenocytes or lymph node cells are used. The lymphocytes are then fused to the immortalized cell line using a suitable fusion agent (*e.g*., polyethylene glycol) to form immortalized hybridoma cells (Goding, Monoclonal Antibodies: Principles and Practice, Academic Press, (1986) pp. 59-103). Immortalized cell lines are typically transformed mammalian cells, particularly myeloma cells of rodent, bovine, and human origin. Typically, rat or mouse myeloma cell lines are used. Hybridoma cells can be cultured in a suitable medium, which preferably contains one or more substances that inhibit the growth or survival of unfused immortalized cells. For example, if the parental cells lack the hypoxanthine guanine phosphoribosyltransferase (HGPRT or HPRT), the hybridoma's culture medium will usually contain hypoxanthine, aminopterin, and thymidine ("HAT medium"), which can prevent the growth of HGPRT-deficient cells.

Preferred immortalized cell lines are those that fuse efficiently, support stable high-level expression of the antibody by the selected antibody-producing cells, and are sensitive to medium such as HAT medium. A more preferred immortalized cell line is a murine myeloma cell line, which is available, for example, from the Salk Institute Cell Distribution Center in San Diego, California, and the American Type Culture Collection in Manassas, Virginia. Human myeloma and mouse-human heterologous myeloma cell lines for the production of human monoclonal antibodies have also been described (see Kozbor, J. Immunol., 133:3001 (1984); Brodeur et al., Monoclonal Antibody Production Techniques and Applications, Marcel Dekker, Inc., New York, (1987) pp. 51-63)).

The culture medium in which the hybridoma cells are cultured can then be determined for the presence of monoclonal antibodies directed against the antigen. Preferably, the binding specificity of the monoclonal antibodies produced by the hybridoma cells is determined by immunoprecipitation or by an *in vitro* binding assay such as a radioimmunoassay (RIA) or an enzyme-linked immunosorbent assay (ELISA). Such techniques and assays are known in the art. The binding affinity of a monoclonal antibody can be determined, for example, by the Scatchard analysis of Munson and Pollard, Anal. Biochem., 107:220 (1980). Furthermore, in the therapeutic application of monoclonal antibodies, it is important to identify antibodies with high specificity and high binding affinity for the target antigen.

After the desired hybridoma cells have been identified, the clones can be subcloned by limiting dilution procedures and cultured by standard methods (see Goding, Monoclonal Antibodies: Principles and Practice, Academic Press, (1986) pp. 59-103). Medium suitable for this purpose include, for example, Dulbecco's Modified Eagle's Medium and RPMI-1640 medium. Alternatively, hybridoma cells can be grown *in vivo* as mammalian ascites fluid.

Monoclonal antibodies secreted by the subclones can be isolated or purified from the culture medium or ascitic fluid by conventional immunoglobulin purification procedures, such as protein A-Sepharose, hydroxyapatite chromatography, gel electrophoresis, dialysis, or affinity chromatography.

Monoclonal antibodies can also be prepared by recombinant DNA methods, such as U.S. Patent No. 4,816,567. DNA encoding the monoclonal antibodies of the invention can be readily isolated and sequenced using conventional procedures (eg, by using oligonucleotide probes capable of specifically binding to genes encoding murine antibody heavy and light chains). The hybridoma cells of the present invention serve as a preferred source of such DNA. The isolated DNA can be placed in an expression vector and then transfected into host cells, such as simian COS cells, Chinese hamster ovary (CHO) cells or myeloma cells that do not produce immunoglobulins, to obtain antibodies in the synthetic recombinant host cells of monoclonal antibodies. The DNA can also be modified, for example, by substituting coding sequences for human heavy and light chain constant domains for homologous murine sequences (see U.S. Patent No. 4,816,567; Morrison, Nature 368, 812-13 (1994)) or by covalently linking all or part of the coding sequence for a non-immunoglobulin polypeptide to an immunoglobulin coding sequence. Such non-immunoglobulin polypeptides can replace the constant domains of the antibodies of the invention, or can replace the variable domains of one antigen-binding site of the antibodies of the invention to produce chimeric bivalent antibodies.

Fully human antibodies are antibody molecules in which the entire sequence of the light and heavy chains, including the CDRs, is derived from human genes. Such antibodies are referred to herein as "human antibodies" or "fully human antibodies." Fully human monoclonal antibodies can be prepared using trioma technology; human B cell hybridoma technology (see Kozbor et al., 1983 Immunol Today 4:72); and EBV hybridoma technology for the production of human monoclonal antibodies (see Cole et al., 1985 In: MONOCLONAL ANTIBODIES AND CANCER THERAPY, Alan R. Liss, Inc., pp. 77-96). Human monoclonal antibodies can be used, and can be obtained by using human hybridomas (see Cote et al., 1983. Proc Natl Acad Sci USA 80:2026-2030) or by transforming human B cells with Epstein Barr virus *in vitro* (see Cole et al., 1985 In: MONOCLONAL ANTIBODIES AND CANCER THERAPY, Alan R. Liss, Inc., pp. 77-96).

The sequence of the antigen-binding protein of the present application can be obtained using conventional techniques, such as PCR amplification or genome library screening. In addition, the coding sequences of the light and heavy chains can also be fused together to form single-chain antibodies.

Once the relevant sequence is obtained, recombination can be used to obtain the relevant sequence in large quantities. This is usually done by cloning it into a vector, transforming it into cells, and then isolating the relevant sequence from the propagated host cells by conventional methods. In addition, artificial synthesis methods can also be used to synthesize relevant sequences, especially when the fragment length is short. Often, fragments with long sequences are obtained by first synthesizing multiple small fragments and then ligating them. The nucleic acid molecule can then be introduced into a variety of existing DNA molecules (or vectors) and cells known in the art.

### Pharmaceutical composition

In another aspect, the invention provides a pharmaceutical composition comprising the antigen-binding protein disclosed herein or the antigen-binding fragment thereof, the nucleic acid molecule disclosed herein, the vector disclosed herein, and/or the cell disclosed herein, and optionally a pharmaceutically acceptable carrier.

The antigen-binding proteins of the invention, or antigen-binding fragments or agents thereof (also referred to herein as "active compounds"), may be incorporated into pharmaceutical compositions suitable for administration. Such compositions typically comprise an antigen-binding protein or an antigen-binding fragment thereof or an agent and a pharmaceutically acceptable carrier. As used herein, the term "pharmaceutically acceptable carrier" is intended to include any and all solvents, dispersion media, coatings, antibacterial and antifungal agents, isotonic and absorption delaying agents, and the like that are compatible with drug administration. Preferred examples of such carriers or excipients include, but are not limited to, water, saline, Ringer's solution, dextrose solution, and 5% human serum albumin. Liposomes and non-aqueous vehicles such as fixed oils may also be used. The use of such media and agents for pharmaceutically active substances is well known in the art. Unless any conventional media or agent is incompatible with the active compound, its use in the compositions is contemplated. The pharmaceutical compositions of the present application may also contain more than one active compound, typically those with complementary activities that do not adversely affect each other. The type and effective amount of such drugs depend, for example, on the amount and type of antagonist present in the formulation, and on the clinical parameters of the subject.

In some embodiments of the pharmaceutical compositions disclosed herein, the pharmaceutical composition further comprises a second therapeutic agent. In some embodiments, the second therapeutic agent is selected from the group consisting of antibodies, chemotherapeutic agents, radiotherapeutic agents, siRNA, antisense oligonucleotides, polypeptides, and small molecule drugs.

In some embodiments, the second therapeutic agent is an immune checkpoint inhibitor. Examples of immune checkpoint inhibitors include, but are not limited to, PD-1 inhibitors, PD-L1 inhibitors, CTLA-4 inhibitors, adenosine A2A receptor inhibitors, B7-H3 inhibitors, B7-H4 inhibitors, BTLA Inhibitors, KIR inhibitors, LAG3 inhibitors, TIM-3 inhibitors, VISTA inhibitors, galectin-9 inhibitors or TIGIT inhibitors. In some embodiments, the checkpoint inhibitor is a PD-1 or PD-L1 inhibitor.

In some embodiments, the second therapeutic agent is a cytokine. Examples of cytokines include, but are not limited to, interleukins (*e.g*., IL-2, IL-7, IL-10, IL-12, IL-15) and interferons (*e.g*., IFNa, IFNy).

In some embodiments, the second therapeutic agent is a chemotherapeutic agent. The chemotherapeutic agents can include, for example, cytotoxic agents, anti-metabolite agents (*e.g.,* folate antagonists, purine analogs, pyrimidine analogs, *etc.),* topoisomerase inhibitors (*e.g*., camptothecin derivatives, anthracenedione, anthracyclines, epipodophyllotoxins, quinoline alkaloids, *etc.*)*,* anti-microtubule agents (*e.g.,* taxanes, vinca alkaloids), protein synthesis inhibitors (*e.g*., cephalotaxine, camptothecin derivatives, quinoline alkaloids), alkylating agents (*e.g*., alkyl sulfonates, ethylenimines, nitrogen mustards, nitrosoureas, platinum derivatives, triazenes, *etc.*), alkaloids, terpenoids, and kinase inhibitors.

In some embodiments, the second therapeutic agent is a radiotherapeutic agent. Radiotherapeutic agents refer to those agents routinely employed in the therapeutic field and include photons of sufficient energy to ionize chemical bonds, such as alpha (alpha), beta (beta) and gamma (gamma) rays from radioactive nuclei and X-rays.

The pharmaceutical compositions of the present invention may be formulated to be compatible with their intended route of administration. The administration route of the pharmaceutical composition of the present invention is preferably parenteral administration, injection administration or oral administration. The injection administration preferably includes intravenous injection, intramuscular injection, intraperitoneal injection, intradermal injection or subcutaneous injection. The pharmaceutical composition is in various conventional dosage forms in this field, preferably in the form of solid, semi-solid or liquid, that is, in the form of aqueous solution, non-aqueous solution or suspension, and more preferably in the form of tablets, capsules, granules agents, injections or infusions, *etc.* More preferred is administration via intravascular, subcutaneous, intraperitoneal or intramuscular route. Preferably, the pharmaceutical composition can also be administered as an aerosol or coarse spray, that is, nasal administration; or, intrathecal, intramedullary or intraventricular administration. More preferably, the pharmaceutical composition can also be administered transdermally, percutaneously, topically, enterally, intravaginally, sublingually or rectally. The pharmaceutical composition of the present invention can be made into various dosage forms according to needs, and can be administered by a physician based on the patient's type, age, weight, general disease status, administration method and other factors to determine a dose that is beneficial to the patient. The administration method can be, for example, injection or other treatment methods.

The dosage level of the pharmaceutical composition administered according to the present invention can be adjusted according to the amount of the composition required to achieve the desired therapeutic result. The administration regimen may also be a single injection or multiple injections, or may be modified. The dosage level and regimen selected are appropriately adjusted depending on various factors including the activity and stability (ie, half-life), formulation, route of administration, combination with other drugs or treatments, the disease or condition to be detected and/or treated, and the health condition and prior medical history of the subject to be treated with.

### Treatment methods and uses

The present disclosure provides a method of treating a disease in a subject, comprising administering to the subject an effective amount of the antigen-binding protein disclosed herein or the antigen-binding fragment thereof, the nucleic acid disclosed herein, the vector disclosed herein, the host cell disclosed herein or the pharmaceutical compositions disclosed herein.

The present disclosure provides the use of the antigen-binding protein or the antigen-binding fragment thereof, the nucleic acid disclosed herein, the vector disclosed herein, the host cell disclosed herein, or the pharmaceutical composition disclosed herein in the manufacture of a medicament for treating a disease in a subject.

The present disclosure also provides the antigen-binding protein or antigen-binding fragment thereof, the nucleic acid disclosed herein, the vector disclosed herein, the host cell disclosed herein, or the pharmaceutical composition disclosed herein for use in treating a disease in a subject.

In some embodiments, the disease is a TL1A-mediated disease. In some embodiments, the disease is a disease associated with TL1A expression. In some embodiments, the disease is a disease associated with DR3 expression. In some embodiments, the disease is a disease associated with DcR3 expression. In some embodiments, the disease is a disease associated with the TL1A/DR3 signaling pathway. In some embodiments, the disease is selected from autoimmune diseases, inflammatory diseases, and fibrotic diseases.

In some embodiments, the disease is an inflammatory disease or condition. Non-limiting examples of inflammatory diseases include allergies, ankylosing spondylitis, asthma, atopic dermatitis, autoimmune diseases or conditions, cancer, celiac disease, chronic obstructive pulmonary disease (COPD), chronic peptic ulcer, cystic fibrosis, diabetes mellitus (e.g., type 1 diabetes and type 2 diabetes), glomerulonephritis, gout, hepatitis (e.g., active hepatitis), immune-mediated disease or disorder, inflammatory bowel disease (IBD) such as Rohn's disease and ulcerative colitis, myositis, osteoarthritis, pelvic inflammatory disease (PID), multiple sclerosis, neurodegenerative diseases of aging, periodontal disease (e.g., periodontitis), preperfusion injury, transplantation Rejection, psoriasis, pulmonary fibrosis, rheumatic diseases, scleroderma, sinusitis, tuberculosis.

In some embodiments, the disease is an autoimmune disease or disorder. Non-limiting examples of autoimmune diseases or conditions include Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AM AN), Balo disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS) or Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne In versa), Hypogammaglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myositis, Narcolepsy, Neonatal Lupus, Neuromyelitis optica, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonnage-Turner syndrome,, Pemphigus, Peripheral neuropathy, Perivenous encephalomyelitis, Pernicious anemia (PA), POEMS syndrome, Polyarteritis nodosa, Polyglandular syndrome type I, Polyglandular syndrome type II, Polyglandular syndrome type III, Polymyalgia rheumatica, Polymyositis, Postmyocardial infarction syndrome, Postpericardiotomy syndrome, Primary biliary cirrhosis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjogren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, and Vogt-Koyanagi-Harada Disease.

In some embodiments, the disease is a fibrotic disease. "Fibrotic disease" means a disease/condition characterized by fibrosis, including but not limited to: respiratory conditions such as pulmonary fibrosis, cystic fibrosis, idiopathic pulmonary fibrosis, progressive massive fibrosis, scleroderma, obliterative bronchiolitis, Hermansky-Pudlak syndrome, asbestosis, silicosis, chronic pulmonary hypertension, AIDS associated pulmonary hypertension, sarcoidosis, tumor stroma in lung disease, and asthma; chronic liver disease, primary biliary cirrhosis (PBC), schistosomal liver disease, liver cirrhosis; cardiovascular conditions such as hypertrophic cardiomyopathy, dilated cardiomyopathy (DCM), fibrosis of the atrium, atrial fibrillation, fibrosis of the ventricle, ventricular fibrillation, myocardial fibrosis, Brugada syndrome, myocarditis, endomyocardial fibrosis, myocardial infarction, fibrotic vascular disease, hypertensive heart disease, arrhythmogenic right ventricular cardiomyopathy (ARVC), tubulointerstitial and glomerular fibrosis, atherosclerosis, varicose veins, cerebral infarcts; neurological conditions such as gliosis and Alzheimer's disease; muscular dystrophy such as Duchenne muscular dystrophy (DMD) or Beckers muscular dystrophy (BMD); gastrointestinal conditions such as Chron's disease, microscopic colitis and primary sclerosing cholangitis (PSC); skin conditions such as scleroderma, nephrogenic systemic fibrosis and cutis keloid; arthrofibrosis; Dupuytren's contracture; mediastinal fibrosis; retroperitoneal fibrosis; myelofibrosis; Peyronie's disease; adhesive capsulitis; kidney disease (e.g., renal fibrosis, nephritic syndrome, Alport's syndrome, HIV associated nephropathy, polycystic kidney disease, Fabry's disease, diabetic nephropathy, chronic glomerulonephritis, nephritis associated with systemic lupus); progressive systemic sclerosis (PSS); chronic graft versus host disease; diseases of the eye such as Grave's opthalmopathy, epiretinal fibrosis, retinal fibrosis, subretinal fibrosis (*e.g.* associated with macular degeneration (*e.g.* wet age-related macular degeneration (AMD)), diabetic retinopathy, glaucoma, corneal fibrosis, post-surgical fibrosis (*e.g.* of the posterior capsule following cataract surgery, or of the bleb following trabeculectomy for glaucoma), conjunctival fibrosis, subconjunctival fibrosis; arthritis; fibrotic pre-neoplastic and fibrotic neoplastic disease; and fibrosis induced by chemical or environmental insult (*e*.*g*., cancer chemotherapy, pesticides, radiation/cancer radiotherapy).

In a preferred embodiment, the disease is selected from the group consisting of inflammatory bowel disease (*e*.*g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

In some embodiments, dosage administered to a subject may vary with the embodiment, the medicament employed, the method of administration, and the site and subject being treated. However, a dose should be sufficient to provide a therapeutic response. A clinician may determine the effective amount to be administered to a human or other subject in order to treat a medical condition. The precise amount required to be therapeutically effective may depend upon numerous factors, *e*.*g*., such as the activity of the antibody, and the route of administration.

A dose of the antibodies, compositions or conjugates described herein may be administered to a mammal at one time or in a series of sub-doses administered over a suitable period of time, *e.g.,* on a daily, semi-weekly, weekly, bi-weekly, semi-monthly, bi-monthly, semi-annual, or annual basis, as needed. A dosage unit comprising an effective amount of antibodies, compositions or conjugates may be administered in a single daily dose, or the total daily dosage may be administered in two, three, four, or more divided doses administered daily, as needed.

A suitable means of administration may be selected by a medical practitioner. Route of administration may be parenteral, for example, administration by injection, transnasal administration, transpulmonary administration, or transcutaneous administration. Administration may be systemic or local by intravenous injection, intramuscular injection, intraperitoneal injection, subcutaneous injection. In some embodiments, the antibodies, compositions or conjugates are selected for parenteral delivery, for inhalation, or for delivery through the digestive tract, such as orally. Dose and method of administration may vary depending on the weight, age, condition, and the like of the subject, and may be suitably selected.

The methods and compositions described herein can be used alone or in conjunction or combination with other therapeutic agents and/or modalities. As used herein, the term "joint" or "combination" administration shall be understood to mean the delivery of two (or more) different treatments to a subject over the course of the subject's condition such that the effects of the treatments on the patient to overlap at certain time points. In some embodiments, delivery of one treatment is still ongoing when delivery of the second treatment begins, thus there is an overlap in administration. This is sometimes referred to herein as "simultaneous" or "simultaneous delivery." In other embodiments, delivery of one treatment ends before delivery of another treatment begins. In some embodiments of either case, the treatment is more effective due to combined administration. For example, the same effect may be observed with less of the second treatment when administered in combination with the first treatment than would be observed if the second treatment was administered alone without the first treatment, or the second treatment relieves the symptoms to a greater extent; or a similar situation exists with the first treatment. In some embodiments, coadministration results in a reduction in symptoms or other parameters associated with a condition that is greater than that observed in the absence of the other treatment. The effects of two treatments can be partially additive, fully additive, or greater than additive. Coadministration can be such that the effect of a first treatment administered is still detectable when a second treatment is administered.

The present disclosure provides methods of treating a subject by administering a second therapeutic agent in combination with the anti-TL1A antigen-binding protein or antigen-binding fragment thereof disclosed herein. Therefore, in some embodiments, the method further comprises administering to the subject a second therapeutic agent.

The present disclosure provides the use of the antigen-binding protein or the antigen-binding fragment thereof, the nucleic acid disclosed herein, the vector disclosed herein, the host cell disclosed herein, or the pharmaceutical composition disclosed herein and a second therapeutic agent in the manufacture of a medicament for treating a disease in a subject.

The present disclosure provides the use of the antigen-binding protein or the antigen-binding fragment thereof, the nucleic acid disclosed herein, the vector disclosed herein, the host cell disclosed herein, or the pharmaceutical composition disclosed herein in the manufacture of a medicament for administration in combination with a second therapeutic agent to treat a disease in a subject.

The present disclosure provides the use of a second therapeutic agent in the manufacture of a medicament for administration in combination with the antigen-binding protein or the antigen-binding fragment thereof, the nucleic acid disclosed herein, the vector disclosed herein, the host cell disclosed herein, or the pharmaceutical composition disclosed herein to treat a disease in a subject.

The present disclosure also provides the antigen-binding protein or antigen-binding fragment thereof, the nucleic acid disclosed herein, the vector disclosed herein, the host cell disclosed herein, or the pharmaceutical composition disclosed herein for use in administration in combination with a second therapeutic agent to treat a disease in a subject.

The amounts of the antigen-binding protein or antigen-binding fragment thereof and the second therapeutic agent as well as the relative times of administration can be selected to achieve the desired combined therapeutic effect. For example, when a combination therapy is administered to a patient in need of such administration, the therapeutic agents in the combination or one or more pharmaceutical compositions comprising the therapeutic agents can be administered in any order such as sequentially, jointly, together, simultaneously. Furthermore, for example, the antigen-binding protein or antigen-binding fragment thereof can be administered while the second therapeutic agent is exerting its prophylactic or therapeutic effect, or vice versa.

In certain embodiments, the antigen-binding protein or antigen-binding fragment thereof, nucleic acid, vector, host cell, or pharmaceutical composition disclosed herein is administered before, substantially simultaneously with, or after the administration of the second therapeutic agent.

In some embodiments, the second therapeutic agent is an immune checkpoint inhibitor. Examples of immune checkpoint inhibitors include, but are not limited to, PD-1 inhibitors, PD-L1 inhibitors, CTLA-4 inhibitors, adenosine A2A receptor inhibitors, B7-H3 inhibitors, B7-H4 inhibitors, BTLA Inhibitors, KIR inhibitors, LAG3 inhibitors, TIM-3 inhibitors, VISTA inhibitors, galectin-9 inhibitors or TIGIT inhibitors. In some embodiments, the checkpoint inhibitor is a PD-1 or PD-L1 inhibitor.

In some embodiments, the second therapeutic agent is a cytokine. Examples of cytokines include, but are not limited to, interleukins (*e*.*g*., IL-2, IL-7, IL-10, IL-12, IL-15) and interferons (*e*.*g*., IFNa, IFNy).

In some embodiments, the second therapeutic agent is a chemotherapeutic agent. The chemotherapeutic agents can include, for example, cytotoxic agents, anti-metabolite agents (*e.g.,* folate antagonists, purine analogs, pyrimidine analogs, *etc*.), topoisomerase inhibitors (*e*.*g*., camptothecin derivatives, anthracenedione, anthracyclines, epipodophyllotoxins, quinoline alkaloids, *etc.*)*,* anti-microtubule agents (*e.g.,* taxanes, vinca alkaloids), protein synthesis inhibitors (*e*.*g*., cephalotaxine, camptothecin derivatives, quinoline alkaloids), alkylating agents (*e*.*g*., alkyl sulfonates, ethylenimines, nitrogen mustards, nitrosoureas, platinum derivatives, triazenes, *etc.*), alkaloids, terpenoids, and kinase inhibitors.

In some embodiments, the second therapeutic agent is a radiotherapeutic agent. Radiotherapeutic agents refer to those agents routinely employed in the therapeutic field and include photons of sufficient energy to ionize chemical bonds, such as alpha (alpha), beta (beta) and gamma (gamma) rays from radioactive nuclei and X-rays.

### Detection methods and uses

In another aspect, the present disclosure provides a method of detecting a TL1A protein *in vitro* or *in vivo,* comprising contacting a sample with the TL1A antigen-binding protein disclosed herein to detect the presence of the TL1A protein.

Here provides methods for detecting the presence or levels of TL1A in samples, comprising:
(1) contacting the sample with the antigen-binding protein disclosed herein or the antigen-binding fragment thereof; and
(2) determining the presence or level of TL1A in the sample by detecting the binding of the antigen-binding protein to the sample.

In some embodiments, the sample can be any sample including, but not limited to, blood samples, tissue from biopsies, autopsy and pathology specimens. Samples also include body fluids such as blood, serum, plasma, sputum, spinal fluid, or urine. In some embodiments, the control sample is a sample from a subject without disease. In particular embodiments, the sample is a blood or tissue sample.

In preferred embodiments, the disease is selected from the group consisting of inflammatory bowel disease (*e*.*g*., ulcerative colitis and Crohn's disease), rheumatoid arthritis, psoriatic arthritis, ankylosing spondylosis inflammation, psoriasis, hidradenitis suppurativa, uveitis, asthma, atopic dermatitis, systemic lupus erythematosus, multiple sclerosis, transplant rejection, central nervous system damage, optic neuritis, age-related macular degeneration, sjogren syndrome, scleroderma, systemic sclerosis, vasculitis, atherosclerosis, chronic kidney disease, nephrogenic systemic fibrosis, liver fibrosis, chronic bronchitis, interstitial pneumonia, pulmonary fibrosis, chronic obstruction pulmonary disease, idiopathic pulmonary fibrosis, pulmonary fibrosis caused by tuberculosis, retroperitoneal fibrosis, cystic fibrosis, endocardial fibrosis, atrial fibrosis, mediastinal fibrosis, myeloid fibrosis, progressive fibrosis block fibrosis, and arthrofibrosis.

In some embodiments of the detection method, the anti-TL1A antibody is directly labeled with a detectable label. In another embodiment, the anti-TL1A antibody (the first antibody) is unlabeled, and the second antibody or other molecule that can bind to the first antibody is labeled. As is well known to those skilled in the art, secondary antibodies that are capable of specifically binding to the first antibody for a particular species and class are selected. For example, if the first antibody is a human IgG, the second antibody can be an anti-human IgG. Other molecules that can bind to antibodies include, but are not limited to, Protein A and Protein G, both of which are commercially available.

Suitable labels for antibodies or secondary antibodies include various enzymes, prosthetic groups, fluorescent materials, luminescent materials, magnetic agents and radioactive materials. Non-limiting examples of suitable enzymes include horseradish peroxidase, alkaline phosphatase, beta-galactosidase or acetylcholinesterase. Non-limiting examples of suitable prosthetic group complexes include streptavidin/biotin and avidin/biotin. Non-limiting examples of suitable fluorescent materials include umbelliferone, fluorescein, fluorescein isothiocyanate, rhodamine, dichlorotriazinamide fluorescein, dansyl chloride or phycoerythrin. A non-limiting exemplary luminescent material is luminol; a non-limiting exemplary magnetic agent is gadolinium, and non-limiting exemplary radioactive labels include ¹²⁵I, ¹³¹I, ³⁵S or ³H.

In an alternative embodiment, TL1A can be determined in a biological sample by a competitive immunoassay utilizing a TL1A protein standard labeled with a detectable substance and an unlabeled anti-TL1A antibody. In this assay, a biological sample, a labeled TL1A protein standard, and an anti-TL1A antibody are combined, and the amount of the labeled TL1A protein standard that binds to the unlabeled antibody is determined. The amount of TL1A in a biological sample is inversely proportional to the amount of labeled TL1A protein standard bound to the anti-TL1A antibody.

The immunoassays and detection methods disclosed herein can be used for a variety of purposes. In one embodiment, anti-TL1A antibodies can be used to detect the production of TL1A in cells in cell culture. In another embodiment, the antibody can be used to detect the amount of TL1A in a biological sample, such as a tissue sample or a blood or serum sample. In some examples, TL1A is cell surface TL1A.

### Kit/delivery device

In another aspect, the invention provides a kit or delivery device comprising the antigen-binding protein disclosed herein or the antigen-binding fragment thereof, the nucleic acid molecule disclosed herein, the vector disclosed herein, the host cell disclosed herein, and/or the pharmaceutical composition disclosed herein.

In some embodiments, the kit further includes (i) a device for administering the aforementioned antigen-binding protein or antigen-binding fragment thereof, the aforementioned nucleic acid molecule, the aforementioned vector, the aforementioned host cell, and/or the aforementioned pharmaceutical composition; and/or (ii) instructions for use.

In some embodiments, the application provides a kit, which may comprise the antigen-binding protein of the present disclosure or the antigen-binding fragment thereof, the nucleic acid molecule, the vector, the host cell, and/or the pharmaceutical composition. It can include the antibody, nucleic acid molecule, vector, host cell, pharmaceutical composition described herein in a single commonly used container. In some cases, the kit may include instructions for use that include information about the antibody, pharmaceutical composition, and dosage form in the kit. In general, such information assists patients and physicians in using encapsulated antibody, pharmaceutical composition, and dosage form effectively and safely. Containers used in such kits may typically comprise at least one vial, test tube, flask, bottle, syringe or other suitable container into which one or more of the detection and/or therapeutic compositions may be placed, and preferably aliquoted appropriately. Where a second therapeutic agent is also provided, the kit may also contain a second, different container in which the second detection and/or therapeutic composition may be placed. Alternatively, multiple compounds can be prepared as a single pharmaceutical composition and packaged in a single container device such as a vial, flask, syringe, bottle, or other suitable single container.

In one embodiment, the kit is used to detect TL1A in a biological sample. Kits for detecting polypeptides typically include an anti-TL1A antibody, such as any of the monoclonal antibodies disclosed herein. In further embodiments, the antibody is labeled (*e*.*g*., fluorescently, radioactively, or enzymatically).

In some embodiments, the kit may also include additional components to facilitate the application for which the kit is designed. Thus, for example, the kit may additionally contain means for detecting the label *(e.g.,* an enzyme substrate for enzymatic labeling, a filter set for detecting a fluorescent label, a suitable secondary label such as a secondary antibody, *etc.).* Kits may also include buffers and other reagents conventionally used in performing a particular method. Such kits and suitable contents are well known to those skilled in the art.

In some embodiments, the application provides a drug delivery device (e.g., plastic or vial, such as a hollow pin or syringe barrel) that can be used to administer the antigen-binding protein or antigen-binding fragment thereof, nucleic acid molecule, vector, host cell and/or pharmaceutical composition of the disclosure. The device can introduce a substance into the patient via parenteral routes (such as intramuscularly, subcutaneously, or intravenously). For example, the injection device may be a syringe (*e.g.,* a prefilled syringe with an antibody or pharmaceutical composition described herein, such as an autoinjector), which may include a syringe and a needle (can be used to pierce skin and/or blood vessels) containing the fluid to be injected (*e.g.,* the antibody or pharmaceutical composition described herein). The method of administration may vary. Routes of administration may include oral administration, intramuscular injection, subcutaneous injection, rectal administration, *etc.*

### EXAMPLES

The present invention is further described below by way of examples, but the present invention is not limited to the scope of the described examples.

### Example 1. Expression and purification of TL1A

The cDNA sequence encoding human TL1A (HuTL1A) was cloned into the pcDNA3.4 vector (Invitrogen) and expressed in EXPI-293 cells (Invitrogen). The expressed protein was purified by Ni-NTA affinity chromatography and size exclusion chromatography to obtain TL1A antigen with a purity >95%.

### Example 2. Screening of TL1A-specific antibodies from an antibody phage library

The library used for antibody screening is a scFv phage display library composed of natural and synthetic libraries. Each scFv in the library was fused to the G3 protein of M13 phage, allowing it to be displayed on the M13 phage surface. The phage library was used to screen scFv antibody fragments that specifically bind to HuTL1A.

Protein-based phage antibody panning technology was used to select antibodies that specifically bind to HuTLIA from the phage library through three rounds of screening. The specific method was as follows: in the first round of panning, HuTLIA was coated in a microplate; the corresponding phage library was premixed with an equal volume of 2% skim milk, and was then transfered to the microplate wells coated by HuTL1A for binding. After 1 hour incubation, the supernatant in the wells was removed and the wells were washed with sterile PBST (0.1% Tween 80). The bound pahges were then eluted by pH 3.0 buffer. The eluted phage solution was then neutralized, and M13K07 helper phages were added to amplify the eluted phages by ~100 fold. Then the second and third rounds of screening were conducted in a manner similar to the first round of screening. After three rounds of screening, an enriched phage library was obtained. SS320 *E. coli* (SS320 (MC1061 F') electrotransfected competent cells, Lucigen) bacteria were then transformed with the enriched phage library, and were spread on agarose plates for culturing. Single colonies were picked and inoculated, in wells of a 96-well deep well plate with 2YT medium containing ampicillin and kanamycin. After overnight shaking at 37°C, the cells were pelleted, and the supernatant containing monoclonal phages were obtained. Each monoclonal supernatant was divided into two parts, and transferred into two 96-well plates coated with HuTL1A, and incubated for 1 hour. One plate was washed with sterile PBST at pH 7.4 after incubation, and the other plate was washed with pH 6.0 sterile PBST. The bound phages were detected by addition of anti-M13-HRP (Sino Biological), followed by color development reaction.. Obsorbance at 455 nm were measured by microplate reader, and anti-TL1A monoclonal phages that had strong binding to the TL1A antigen at pH 7.4 and weak binding to the TL1A antigen at pH 6.0 was selected.

The phagemids of all the selected monoclonal phage clones from previous step were sequenced. The sequencing results were analyzed. Based on the sequence pattern, new pH-dependent affinity maturation phage library was then constructed for next round of screening. The above screening process was repeated to obtain antibodies with stronger affinity and more significant pH-dependent binding property binding to TL1A.

### Example 3. Preparation of recombinant TL1A monoclonal antibody

The cDNA sequences of the light chain and heavy chain variable regions of the selected high-affinity pH-dependent monoclonal phages were cloned into the pcDNA3.4 vector (Invitrogen) that already contains the antibody constant region, and multiple high-affinity pH-dependent anti-TL1A monoclonal antibodies were obtained, named herein as the "M701 antibodies." These M701 antibodies were numbered as 3756, 3876, 3912, 3966, 4007 and 4019 respectively. 3756 was from the original library, and the other antibodies were from the library optimized based on 3756 (3756 was subjected to affinity maturation and modification of pH-dependent antigen binding properties). Single-arm antibodies were prepared for these antibodies (methods were as described in Ridgway, J.B.; Presta, L.G.; Carter, P. Protein Eng. 1996, 9, 617-621) for affinity testing. The corresponding single-arm antibody numbers are: 3672, 3900, 4035, 3952, 3973 and 3990. At the same time, TL1A control antibodies were prepared, including Pfizer/Roivant's TL1A monoclonal antibody RVT-3101 (sequence from patent US9683998B2), named BM1 antibody, and the corresponding single-arm antibody named SA-BM1 antibody, and Merck/Prometheus' TL1A monoclonal antibody PRA023 (INN sequence 12368), named BM2 antibody, and the corresponding single-arm antibody was named as SA-BM2 antibody. The EXPI-293 cells (Invitrogen) were transintly transfected by the prepared plasmid using the PEI method, and inoculated for 7-10 days to express protein of interest. The cells were then pelleted down and the supernatant was collected. The supernatant was purified by protein A affinity chromatography to obtain purified anti-TL1A monoclonal antibody.

The sequences of the light chain variable regions and heavy chain variable regions of the monoclonal antibodies and single-arm antibodies of the present invention are shown in Table 1 below, and the corresponding CDR sequences (defined according to the Kabat numbering system) are shown in Table 2 below. The full-length heavy chain and light chain sequences of the monoclonal antibodies are shown in Table 3 below, and the full-length heavy chain, light chain and Fc sequences of the single-arm antibodies are shown in Table 4 below.

**Table 1. Variable region sequences of M701 antibodies**

| | |
|---|---|
| 3756 (3672) VL | DIQLTQSPGTLSLSPGERATLSCRASQSVSNHLAWYQQKPGQAPR LLIYGASKRATGIPDRFSGSGSGTDFTLTISRLEPEDFAVYYCQQ YADAPLTFGQGTKVEIK (SEQ ID NO: 1) |
| 3756 (3672) VH | ELQLQESGPGLVKPSETLSLTCAVSGLSFTDFYWAWIRQPPGKGL EWIGEINHKGNTSYNPSLKSRVTVSIDTSKNQFSLKLSSVTAADT AVYFCARDFPNYTFDFWGQGTLVTVSS (SEQ ID NO: 2) |
| 3876 (3900) VL | AIQMTQSPGTLSLSPGERATLSCRASQSVSSHLAWYQQKPGQAPR LLIYGASRRATGIPARFSGSGSGTDFTLTISRLEPEDFAVYYCQQ YADSPLTFGQGTKVEIK (SEQ ID NO: 3) |
| 3876 (3900) VH | EVQLQESGPGLVKPSETLSLTCAVSGVSYSDYHWAWIRQPPGKGL EWIGDINHKGKTNYNPSLKSRVTISVDTSKNQFSLKLSSVTAADT AVYYCARDYPNFLFDYWGQGTLVTVSS (SEQ ID NO: 4) |
| 3912 (4035) VL | DIQMTQSPATLSLSPGERATLSCRASQSVSTYLAWYQQKPGQAPR LLIYGASRRATGIPARFSGSGSGTDFTLTISSLQSEDFAVYYCQQ YGDTPLTFGQGTKVEIK (SEQ ID NO: 5) |
| 3912 (4035) VH | ELQLQESGPGLVKPSETLSLTCAVSGISFNDFHWAWIRQPPGKGL EWIGDINHRGHTSYNPSLKSRVTVSVDTSKNQFSLKLSSVTAADT AVYYCARDYPNFTFDLWGQGTLVTVSS (SEQ ID NO: 6) |
| 3966 (3952) VL | DIQLTQSPGTLSLSPGERATLSCRASQSVSSYLAWYQQKPGQAPR LLIYGASKRATGIPDRFSGSGSGTDFTLTISRLEPEDFAVYYCQQ YGDSPLTFGQGTKVEIK (SEQ ID NO: 7) |
| 3966 (3952) VH | ELQLQESGPGLVKPSETLSLTCAVSGVSFSDYHWAWIRQPPGKGL EWIGDINHRGHTNYNPSLKSRVTVSIDTSKNQFSLKLSSVTAADT AVYFCARDFPNFIFDYWGQGTLVTVSS (SEQ ID NO: 8) |
| 4007 (3973) VL | DIQLTQSPGTLSLSPGERATLSCRASQSVSTYLAWYQQKPGQAPR LLIYGASKRATGIPDRFSGSGSGTDFTLTISRLEPEDFAVYYCQQ YGDSPLTFGQGTKVEIK (SEQ ID NO: 9) |
| 4007 (3973) VH | ELQLQESGPGLVKPSETLSLTCAVSGVSFSDYHWAWIRQPPGKGL EWIGDINHRGHTNYNPSLKSRVTVSIDTSKNQFSLKLSSVTAADT AVYFCARDFPNFIFDFWGQGTLVTVSS (SEQ ID NO: 10) |
| 4019 (3990) VL | DIQLTQSPGTLSLSPGERATLSCRASQSVSNYLAWYQQKPGQAPR LLIYGASRRATGIPARFSGSGSGTDFTLTISSLEPEDFAVYYCQQ YAASPLTFGQGTKVEIK (SEQ ID NO: 11) |
| 4019 (3990) VH | ELQLQESGPGLVKPSETLSLTCAVSGVSFTDYHWAWIRQPPGKGL EWIGDINHRGHTNYNPSLKSRVTVSIDTSKNQFSLKLSSVTAADT AVYYCARDYPNFIYDYWGQGTLVTVSS (SEQ ID NO: 12) |

**Table 2. CDR region sequences of M701 antibodies**

| | |
|---|---|
| 3756 (3672) CDR L1 | RASQSVSNHLA (SEQ ID NO: 13) |
| 3756 (3672) CDR L2 | GASKRAT (SEQ ID NO: 14) |
| 3756 (3672) CDR L3 | QQYADAPLT (SEQ ID NO: 15) |
| 3756 (3672) CDR H1 | DFYWA (SEQ ID NO: 16) |
| 3756 (3672) CDR H2 | EINHKGNTSYNPSLKS (SEQ ID NO: 17) |
| 3756 (3672) CDR H3 | DFPNYTFDF (SEQ ID NO: 18) |
| 3876 (3900) CDR L1 | RASQSVSSHLA (SEQ ID NO: 19) |
| 3876 (3900) CDR L2 | GASRRAT (SEQ ID NO: 20) |
| 3876 (3900) CDR L3 | QQYADSPLT (SEQ ID NO: 21) |
| 3876 (3900) CDR H1 | DYHWA (SEQ ID NO: 22) |
| 3876 (3900) CDR H2 | DINHKGKTNYNPSLKS (SEQ ID NO: 23) |
| 3876 (3900) CDR H3 | DYPNFLFDY (SEQ ID NO: 24) |
| 3912 (4035) CDR L1 | RASQSVSTYLA (SEQ ID NO: 25) |
| 3912 (4035) CDR L2 | GASRRAT (SEQ ID NO: 26) |
| 3912 (4035) CDR L3 | QQYGDTPLT (SEQ ID NO: 27) |
| 3912 (4035) CDR H1 | DFHWA (SEQ ID NO: 28) |
| 3912(4035) CDR H2 | DINHRGHTSYNPSLKS (SEQ ID NO: 29) |
| 3912(4035) CDR H3 | DYPNFTFDL (SEQ ID NO: 30) |
| 3966 (3952) CDR L1 | RASQSVSSYLA (SEQ ID NO: 31) |
| 3966 (3952) CDR L2 | GASKRAT (SEQ ID NO: 32) |
| 3966 (3952) CDR L3 | QQYGDSPLT (SEQ ID NO: 33) |
| 3966 (3952) CDR H1 | DYHWA (SEQ ID NO: 34) |
| 3966 (3952) CDR H2 | DINHRGHTNYNPSLKS (SEQ ID NO: 35) |
| 3966 (3952) CDR H3 | DFPNFI FDY (SEQ ID NO: 36) |
| 4007 (3973) CDR L1 | RASQSVSTYLA (SEQ ID NO: 37) |
| 4007 (3973) CDR L2 | GASKRAT (SEQ ID NO: 38) |
| 4007 (3973) CDR L3 | QQYGDSPLT (SEQ ID NO: 39) |
| 4007 (3973) CDR H1 | DYHWA (SEQ ID NO: 40) |
| 4007 (3973) CDR H2 | DINHRGHTNYNPSLKS (SEQ ID NO: 41) |
| 4007 (3973) CDR H3 | DFPNFI FDF (SEQ ID NO: 42) |
| 4019 (3990) CDR L1 | RASQSVSNYLA (SEQ ID NO: 43) |
| 4019 (3990) CDR L2 | GASRRAT (SEQ ID NO: 44) |
| 4019 (3990) CDR L3 | QQYAASPLT (SEQ ID NO: 45) |
| 4019 (3990) CDR H1 | DYHWA (SEQ ID NO: 46) |
| 4019 (3990) CDR H2 | DINHRGHTNYNPSLKS (SEQ ID NO: 47) |
| 4019 (3990) CDR H3 | DYPNFIYDY (SEQ ID NO: 48) |

**Table 3. Full-length sequences of M701 antibodies**

| | |
|---|---|
| 3756 LC | DIQLTQSPGTLSLSPGERATLSCRASQSVSNHLAWYQQKPGQAPRLLIYGASKR ATGIPDRFSGSGSGTDFTLTISRLEPEDFAVYYCQQYADAPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 49) |
| 3756 HC | ELQLQESGPGLVKPSETLSLTCAVSGLSFTDFYWAWIRQPPGKGLEWIGEINHK GNTSYNPSLKSRVTVSIDTSKNQFSLKLSSVTAADTAVYFCARDFPNYTFDFWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED |
| | PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 50) |
| 3876 LC | AIQMTQSPGTLSLSPGERATLSCRASQSVSSHLAWYQQKPGQAPRLLIYGASRR ATGIPARFSGSGSGTDFTLTISRLEPEDFAVYYCQQYADSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 51) |
| 3876 HC | EVQLQESGPGLVKPSETLSLTCAVSGVSYSDYHWAWIRQPPGKGLEWIGDINHK GKTNYNPSLKSRVTISVDTSKNQFSLKLSSVTAADTAVYYCARDYPNFLFDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 52) |
| 3912 LC | DIQMTQSPATLSLSPGERATLSCRASQSVSTYLAWYQQKPGQAPRLLIYGASRR ATGIPARFSGSGSGTDFTLTISSLQSEDFAVYYCQQYGDTPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 53) |
| 3912 HC | ELQLQESGPGLVKPSETLSLTCAVSGISFNDFHWAWIRQPPGKGLEWIGDINHR GHTSYNPSLKSRVTVSVDTSKNQFSLKLSSVTAADTAVYYCARDYPNFTFDLWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 54) |
| 3966 LC | DIQLTQSPGTLSLSPGERATLSCRASQSVSSYLAWYQQKPGQAPRLLIYGASKR ATGIPDRFSGSGSGTDFTLTISRLEPEDFAVYYCQQYGDSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 55) |
| 3966 HC | ELQLQESGPGLVKPSETLSLTCAVSGVSFSDYHWAWIRQPPGKGLEWIGDINHR GHTNYNPSLKSRVTVSIDTSKNQFSLKLSSVTAADTAVYFCARDFPNFIFDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 56) |
| 4007 LC | DIQLTQSPGTLSLSPGERATLSCRASQSVSTYLAWYQQKPGQAPRLLIYGASKR ATGIPDRFSGSGSGTDFTLTISRLEPEDFAVYYCQQYGDSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 57) |
| 4007 HC | ELQLQESGPGLVKPSETLSLTCAVSGVSFSDYHWAWIRQPPGKGLEWIGDINHR GHTNYNPSLKSRVTVSIDTSKNQFSLKLSSVTAADTAVYFCARDFPNFIFDFWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 58) |
| 4019 LC | DIQLTQSPGTLSLSPGERATLSCRASQSVSNYLAWYQQKPGQAPRLLIYGASRR ATGIPARFSGSGSGTDFTLTISSLEPEDFAVYYCQQYAASPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 59) |
| 4019 HC | ELQLQESGPGLVKPSETLSLTCAVSGVSFTDYHWAWIRQPPGKGLEWIGDINHR GHTNYNPSLKSRVTVSIDTSKNQFSLKLSSVTAADTAVYYCARDYPNFIYDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALAAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVLHEAL HSHYTQKSLSLSPG (SEQ ID NO: 60) |

**Table 4. Full-length sequence of single-arm antibodies**

| | |
|---|---|
| Fc Chain | DKTHTCPPCPAPEAAGGPSVFLFPPKPKDTLMISRTPEVTCVVVDVSHEDPEVK FNWYVDGVEVHNAKTKPREEQYNSTYRVVSVLTVLHQDWLNGKEYKCKVSNKAL GAPIEKTISKAKGQPREPQVCTLPPSREEMTKNQVSLSCAVKGFYPSDIAVEWE SNGQPENNYKTTPPVLDSDGSFFLVSKLTVDKSRWQQGNVFSCSVMHEALHNHY TQKSLSLSPG (SEQ ID NO: 61) |
| 3672 LC | DIQLTQSPGTLSLSPGERATLSCRASQSVSNHLAWYQQKPGQAPRLLIYGASKR ATGIPDRFSGSGSGTDFTLTISRLEPEDFAVYYCQQYADAPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 49) |
| 3672 HC | ELQLQESGPGLVKPSETLSLTCAVSGLSFTDFYWAWIRQPPGKGLEWIGEINHK GNTSYNPSLKSRVTVSIDTSKNQFSLKLSSVTAADTAVYFCARDFPNYTFDFWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 62) |
| 3900 LC | AIQMTQSPGTLSLSPGERATLSCRASQSVSSHLAWYQQKPGQAPRLLIYGASRR ATGIPARFSGSGSGTDFTLTISRLEPEDFAVYYCQQYADSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 51) |
| 3900 HC | EVQLQESGPGLVKPSETLSLTCAVSGVSYSDYHWAWIRQPPGKGLEWIGDINHK GKTNYNPSLKSRVTISVDTSKNQFSLKLSSVTAADTAVYYCARDYPNFLFDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 63) |
| 4035 LC | DIQMTQSPATLSLSPGERATLSCRASQSVSTYLAWYQQKPGQAPRLLIYGASRR ATGIPARFSGSGSGTDFTLTISSLQSEDFAVYYCQQYGDTPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 53) |
| 4035 HC | ELQLQESGPGLVKPSETLSLTCAVSGISFNDFHWAWIRQPPGKGLEWIGDINHR GHTSYNPSLKSRVTVSVDTSKNQFSLKLSSVTAADTAVYYCARDYPNFTFDLWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 64) |
| 3952 LC | DIQLTQSPGTLSLSPGERATLSCRASQSVSSYLAWYQQKPGQAPRLLIYGASKR ATGIPDRFSGSGSGTDFTLTISRLEPEDFAVYYCQQYGDSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 55) |
| 3952 HC | ELQLQESGPGLVKPSETLSLTCAVSGVSFSDYHWAWIRQPPGKGLEWIGDINHR GHTNYNPSLKSRVTVSIDTSKNQFSLKLSSVTAADTAVYFCARDFPNFIFDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 65) |
| 3973 LC | DIQLTQSPGTLSLSPGERATLSCRASQSVSTYLAWYQQKPGQAPRLLIYGASKR ATGIPDRFSGSGSGTDFTLTISRLEPEDFAVYYCQQYGDSPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 57) |
| 3973 HC | ELQLQESGPGLVKPSETLSLTCAVSGVSFSDYHWAWIRQPPGKGLEWIGDINHR GHTNYNPSLKSRVTVSIDTSKNQFSLKLSSVTAADTAVYFCARDFPNFIFDFWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 66) |
| 3990L C | DIQLTQSPGTLSLSPGERATLSCRASQSVSNYLAWYQQKPGQAPRLLIYGASRR ATGIPARFSGSGSGTDFTLTISSLEPEDFAVYYCQQYAASPLTFGQGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 59) |
| 3990 HC | ELQLQESGPGLVKPSETLSLTCAVSGVSFTDYHWAWIRQPPGKGLEWIGDINHR GHTNYNPSLKSRVTVSIDTSKNQFSLKLSSVTAADTAVYYCARDYPNFIYDYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCKVS |
| | NKALGAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPG (SEQ ID NO: 67) |

### Example 4. Characterization of binding affinity and pH-dependent binding of TL1A antibodies

pH-dependent antigen-binding enables an antibody to bind to the HuTLIA antigen with high affinity in serum. However, once the antigen-antibody complex is endocytosed into the cell and enters endosomes with a pH lower than 6.0, the binding affinity decreases significantly, allowing the antigen to be quickly released and subsequently degraded in lysosomes. Meanwhile, the released antibody will bind the FcRn receptor and re-enter into blood circulation to play its role. pH-dependent antigen-binding also accelerates the clearance of TL1A antigen in lysosomes, thereby reducing the level of TL1A in circulation and increasing the effective exposure of the TL1A-binding antibodies.

Enzyme-linked immunosorbent assay (ELISA) was used to assess the HuTLIA binding ability of monoclonal antibodies under different pH conditions. A 96-well microplate was coated with 10 µg/ml streptavidin, blocked with 1% BSA, folowed by addition of 1 µg/ml biotin-labeled HuTLIA antigen. Corresponding antibodies were added to specific wells, and the antibodies were diluted with sodium dihydrogen phosphate-citrate buffer at pH 7.4, pH 6.0, pH 5.8, or pH 5.6. Binding assay was carried out at room temperature for 30 minutes. After the incubation, the excess antibodies were removed and the wells were washed by PBST. HRP-anti-Human Fc (Sino Biological) was then added into each well and incubated for 30 minutes to detect the bound antibody. The substrate TMB was finally added to develop color for 5-10 minutes. The color development reaction was terminated with 1M sulfuric acid. OD450 nm was recorded on a microplate reader, and the data were processed and fitted by 4 parameter logistic regression.

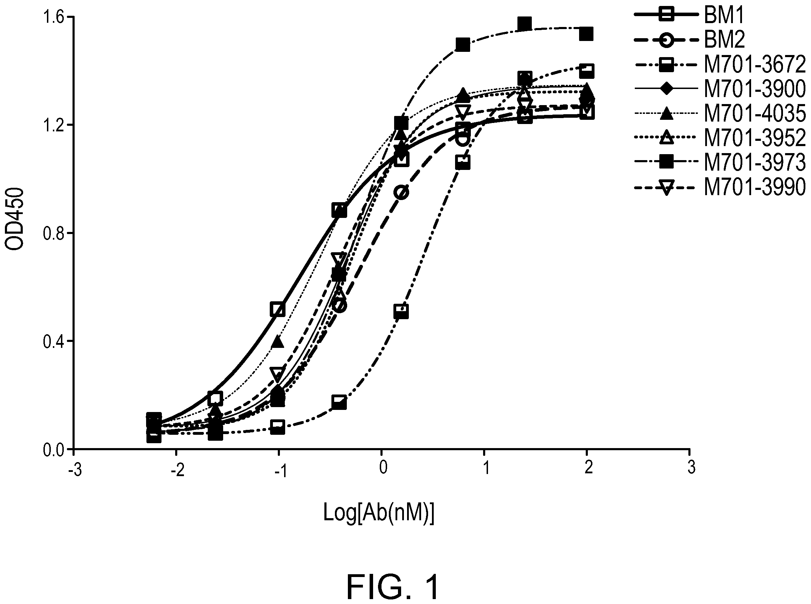

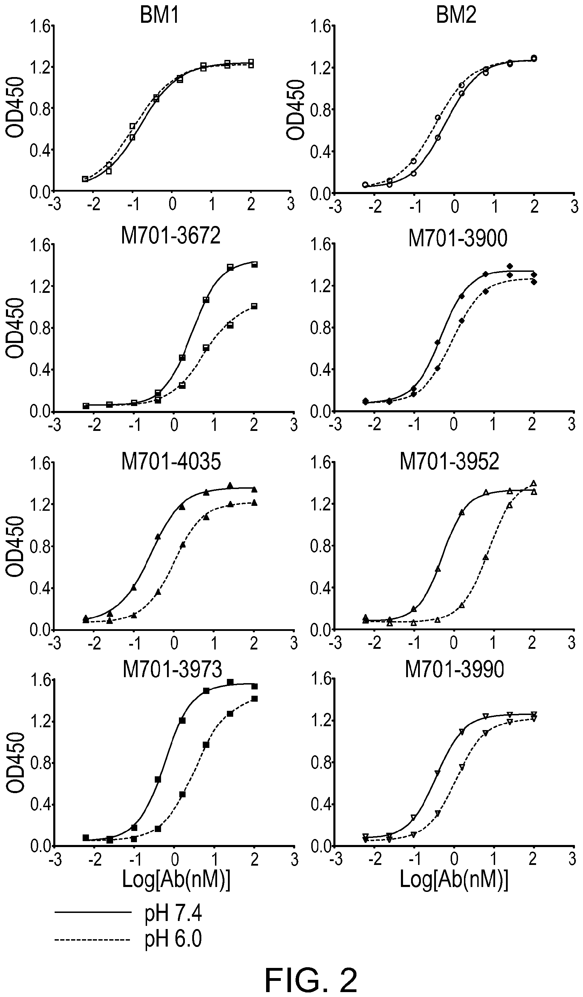

The ELISA results showed that, at pH 7.4, compared to the single-arm antibody 3672 obtained from the initial library, the binding affinity of single-arm antibodies 3900, 4035, 3952, 3973 and 3990 (all based on the sequence optimization of 3672) to the HuTLIA had significantly improved, which was stronger than SA-BM2, but slightly weaker or equivalent to SA-BM1 (Figure 1). For both control antibodies SA-BM1 and SA-BM2, the binding affinity to HuTLIA was not significantly different at pH 7.4 and 6.5. In contrast, the binding affinity of M701 antibodies 3672, 3900, 4035, 3952, 3973 and 3990 to HuTLIA decreased significantly at pH 6.0 compared to pH 7.4, indicating a pH-dependent antigen-binding property (Figure 2).

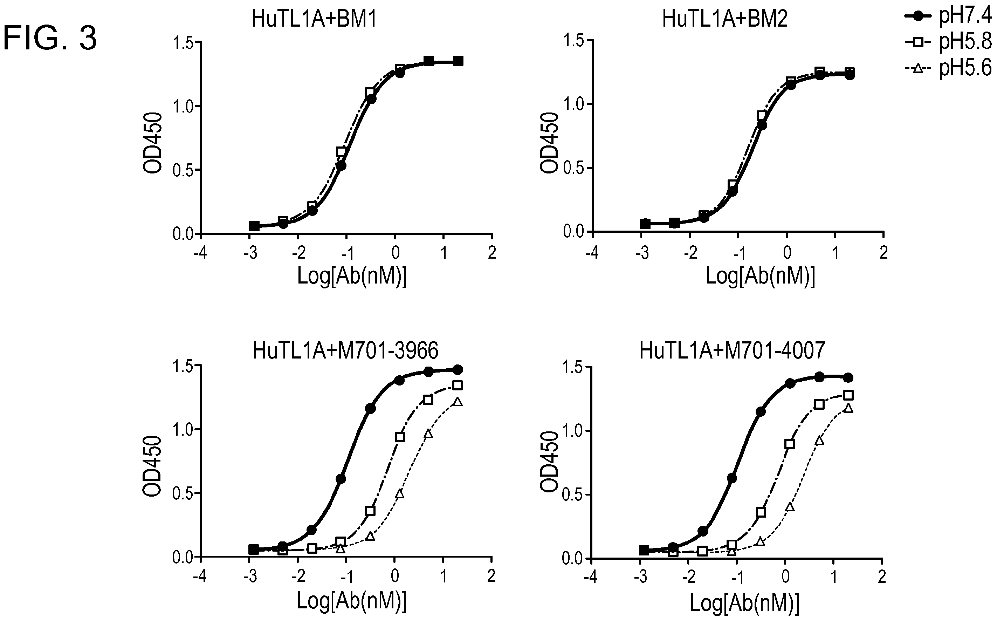

Since the pH of endosome is often lower than 6.0, the TL1A binding abilities of the M701 antibodies and the control antibodies at pH 7.4 and pH lower than 6.0 were compared. The results showed that there was no significant difference in the binidng affinity between the control antibodies BM1 and BM2 to HuTLIA at pH 7.4, pH 5.8, and pH 5.6, while binding of the M701 antibodies 3966 and 4007 to HuTLIA was obviously pH-dependent, in that the binding at pH 5.6 was weaker than that at pH 5.8, and the binding at these low pH values was significantly weaker than that at pH 7.4 (Figure 3).

The binding kinetics between monoclonal antibodies and antigens was measured using Gator, a label-free biomolecule interaction analyzer based on the principle of biolayer interference (BLI). The specific method was as follows: using Flex SA probe, the HuTLIA antigen labelled with Biotin was diluted to 50 nM and captured by the SA probe. Then a serial dilutions of single-arm M701 antibodies and control antibodies were added. The antibodies interacted with the antigen captured by the SA probe, and the interaction was analyzed by detecting the signal change of the reflection interference spectrum of the probe surface, and finally the binding kinetics constants of the antibodies were calculated. The kinetic constants of antigen and antibodies were measured at pH 7.4 and 5.6 in sodium dihydrogen phosphate-citrate buffer.

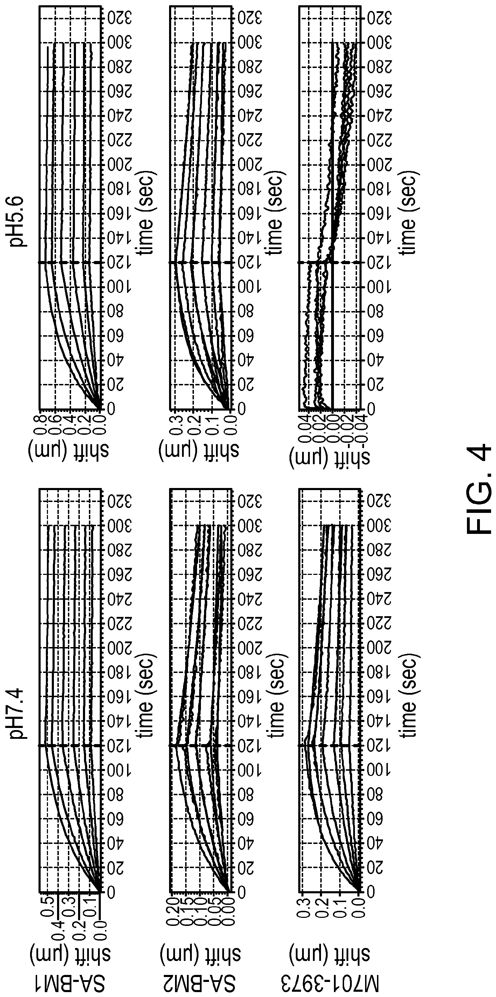

The results showed that the M701 antibodies 3952, 3973, and 3990 had high binding affinity to HuTLIA at pH 7.4, but they barely bind to the HuTLIA antigen at pH 5.6, showing significant pH-dependent antigen-binding property. The control antibodies either had no significant difference in binding affinity to TL1A at pH 7.4 and 5.6 (see SA-BM1), or had stronger affinity to the antigen at pH 5.6 (see SA-BM2) (Table 5, Figure 4).

**Table 5. Kinetic constants for antibodies binding to HuTLIA**

| Antibody | pH7.4 | | | pH5.6 | | |
|---|---|---|---|---|---|---|
| | k_{off}(1/s) | kₒₙ(1/Ms) | K_{D}(M) | k_{off}(1/s) | kₒₙ(1/Ms) | K_{D}(M) |
| SA-BM1 | 3.39E-04 | 1.90E+05 | 1.78E-09 | 3.18E-04 | 2. 52E+05 | 1.26E-09 |
| SA-BM2 | 3.38E-03 | 1.71E+05 | 1.98E-08 | 2.19E-03 | 2.90E+05 | 7.55E-09 |
| M701-3952 | 3.06E-03 | 5.82E+05 | 5.25E-09 | NA | NA | NA |
| M701-3973 | 2.14E-03 | 3.81E+05 | 5.62E-09 | NA | NA | NA |
| M701-3990 | 1.68E-03 | 3.94E+05 | 4.26E-09 | NA | NA | NA |

| | | | | | | |
|---|---|---|---|---|---|---|
| NA: No fitted value, no binding signal | | | | | | |

### Example 5. Species specificity of TL1A antibodies

The binding kinetics between monoclonal antibodies and human TL1A and cynomolgus monkey/rhesus monkey TL1A were measured using Gator, a label-free biomolecule interaction analyzer based on the principle of biolayer interference (BLI). Using anti-His probe, the HuTLIA antigen with His-tag was diluted to 50 nM and captured by the anti-His probe. Then a serial dilutions of single-arm M701 antibodies and control antibodies were added. The antibodies interacted with the His-tagged TL1A antigen captured by the anti-His probe, and the interaction was analyzed by detecting the signal change of the reflection interference spectrum of the probe surface, and finally, the binding kinetics constants of the antibodies were calculated. The results showed that the M701 antibodies 3952, 3973 and 3990 had high binding affinity to human TL1A and cynomolgus monkey/rhesus monkey TL1A, and the binding affinities were similar among the M701 antibodies (Table 6).

ELISA was also used to assess the binding of the antibodies to human TL1A, cynomolgus monkey/rhesus monkey TL1A, rat TL1A, and mouse TL1A. The results showed that the M701 antibodies were not able to bind to rat and mouse TL1A, but bind to both human and cynomolgus monkey/rhesus monkey TL1A, TL1A with similar binding affinity (Table 6) and pH-dependent property (results not shown).

**Table 6. Comparison of kinetic constants of antibodies binding to TL1A of different species**

| Antigen | Antibody | pH7.4 | | |
|---|---|---|---|---|
| | | k_{off}(1/s) | kₒₙ(1/Ms) | K_{D}(M) |
| Human TL1A | SA-BM1 | 3.60E-04 | 3.23E+05 | 1.11E-09 |
| | SA-BM2 | 2.12E-03 | 3.01E+05 | 7.03E-09 |
| | M701-3952 | 3.46E-03 | 6.10E+05 | 5.68E-09 |
| | M701-3973 | 3.53E-03 | 5.96E+05 | 5.92E-09 |
| | M701-3990 | 2.14E-03 | 4.38E+05 | 4.89E-09 |
| Cynomolgus/rhesus monkey TL1A | SA-BM1 | 3.69E-04 | 3.07E+05 | 1.20E-09 |
| | SA-BM2 | 1.52E-03 | 3.06E+05 | 4.97E-09 |
| | M701-3952 | 3.60E-03 | 6.26E+05 | 5.75E-09 |
| | M701-3973 | 3.75E-03 | 6.16E+05 | 6.09E-09 |
| | M701-3990 | 2.08E-03 | 4.62E+05 | 4.51E-09 |

### Example 6. Binding specificity of TL1A antibodies

In this experiment, ELISA was used to assess the binding ability of monoclonal antibodies to TL1A homologous molecules. Representative members of the TNF ligand superfamily, TNFα, LT-α, LIGHT and FASL, were selected for binding testing. 1 µg/mL of TNFα, LT-α, LIGHT or FASL was coated in a microplate with a carbonate buffer solution at pH=9.6, 100 µL per well, and the microplate was incubated at 4°C overnight. The microplate was then washed 5 times with PBST, blocked with 300 µL/well of PBS containing 1% BSA, and was incubated at room temperature for 1 hour. After washing 5 times with PBST, about 100 µL each of the M701 monoclonal antibodies and the control antibodies were added to each well, and were incubated at room temperature for 1 hour. After washing 5 times with PBST, 100 µL of HRP-anti-human Fc (Sino Biological) was added to each well, and was incubated at room temperature for 1 hour. After washing 5 times with PBST, 100 µL of colorimetric substrate TMB was added to each well, and color was developed at room temperature for 10 minutes, followed by addition of 1M sulfuric acid to terminate the reaction. OD450nm was recorded on a microplate reader. The results showed that neither the M701 antibodies nor the control TL1A antibodies bind to TL1A homologs from TNF superfamily (Table 7), suggesting high specificity of all the tested antibodies.

**Table 7. Antibody binding to members of the TNF ligand superfamily**

| | Ab(nM) | BM1 | BM2 | M701-3966 | M701-4007 | M701-4019 | Adalimumab |
|---|---|---|---|---|---|---|---|
| TNF-α | 100 | 0.0815 | 0.0757 | 0.0787 | 0.0790 | 0.1051 | 1.3970 |
| | 25 | 0.0601 | 0.0577 | 0.0593 | 0.0665 | 0.0660 | 1.3676 |
| | 6.25 | 0.0511 | 0.0514 | 0.0511 | 0.0553 | 0.0591 | 1.3135 |
| | 1.563 | 0.0523 | 0.0517 | 0.0528 | 0.0588 | 0.0570 | 1.3015 |
| | 0.391 | 0.0539 | 0.0522 | 0.0517 | 0.0557 | 0.0567 | 1.1121 |
| | 0.000 | 0.0666 | 0.0612 | 0.0706 | 0.0762 | 0.0748 | 0.2460 |
| | | | | | | | |

| | Ab(nM) | BM1 | BM2 | M701-3966 | M701-4007 | M701-4019 | Adalimumab |
|---|---|---|---|---|---|---|---|
| LIGHT | 100 | 0.0996 | 0.0864 | 0.0876 | 0.0853 | 0.0942 | 0.0804 |
| | 25 | 0.0916 | 0.0760 | 0.0674 | 0.0630 | 0.0610 | 0.0610 |
| | 6.25 | 0.0903 | 0.0719 | 0.0568 | 0.0542 | 0.0572 | 0.0533 |
| | 1.563 | 0.0887 | 0.0741 | 0.0573 | 0.0568 | 0.0539 | 0.0530 |
| | 0.391 | 0.0834 | 0.0643 | 0.0558 | 0.0534 | 0.0528 | 0.0514 |
| | 0.000 | 0.0644 | 0.0588 | 0.0600 | 0.0568 | 0.0563 | 0.0556 |
| | | | | | | | |

| | Ab(nM) | BM1 | BM2 | M701-3966 | M701-4007 | M701-4019 | Adalimumab |
|---|---|---|---|---|---|---|---|
| FASL | 100 | 0.0855 | 0.0828 | 0.0914 | 0.0890 | 0.0886 | 0.0865 |
| | 25 | 0.0609 | 0.0643 | 0.0662 | 0.0634 | 0.0612 | 0.0696 |
| | 6.25 | 0.0513 | 0.0533 | 0.0525 | 0.0523 | 0.0529 | 0.0638 |
| | 1.563 | 0.0518 | 0.0522 | 0.0522 | 0.0600 | 0.0514 | 0.0623 |
| | 0.391 | 0.0510 | 0.0511 | 0.0504 | 0.0504 | 0.0506 | 0.0605 |
| | 0.000 | 0.0544 | 0.0549 | 0.0692 | 0.0648 | 0.0602 | 0.0593 |
| | | | | | | | |

| | Ab(nM) | BM1 | BM2 | M701-3966 | M701-4007 | M701-4019 | Adalimumab |
|---|---|---|---|---|---|---|---|
| LT-α | 100 | 0.0823 | 0.0701 | 0.0709 | 0.0706 | 0.0670 | 0.0744 |
| | 25 | 0.0845 | 0.0659 | 0.0639 | 0.0628 | 0.0627 | 0.0599 |
| | 6.25 | 0.0909 | 0.0646 | 0.0600 | 0.0588 | 0.0566 | 0.0560 |
| | 1.563 | 0.0950 | 0.0701 | 0.0651 | 0.0625 | 0.0602 | 0.0579 |
| | 0.391 | 0.0978 | 0.0713 | 0.0604 | 0.0576 | 0.0572 | 0.0557 |
| | 0.000 | 0.0972 | 0.0803 | 0.0704 | 0.0662 | 0.0648 | 0.0628 |

### Example 7. Epitopes of TL1A antibodies

Alanine Scanning was used to determine the epitope of the M701 antibodies. 3-4 different amino acid sites were selected for point mutations in each representative region of the antigen, and all mutants were tested for binding by ELISA and Gator.

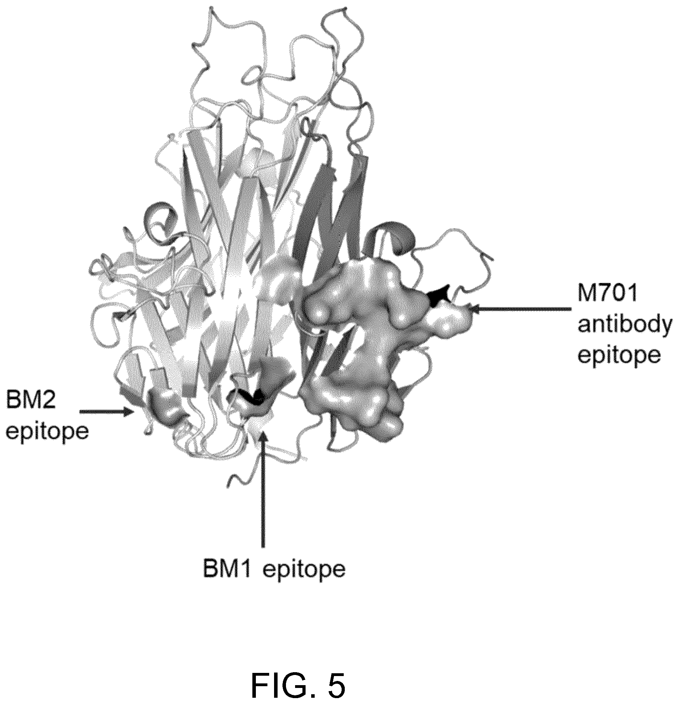

Experimental results showed that the epitope of the M701 antibodies is different from that of the control antibodies BM1 and BM2. The reported epitopes of BM1 are K183 and T192 of TL1A, and BM1 only binds to the TL1A trimer. The epitope of BM2 has not been disclosed, but the epitope binning study reported in patent US20210070871A1 shows that the epitopes of BM2 and BM1 are different, and BM2 binds to both TL1A trimers and TL1A monomer. To determine the epitope of the M701 antibodies, an alanine screen was performed on each representative region of the antigen. The results showed that the epitope of the M701 antibodies was in the region centered with R103, including amino acids R103, G124, M196, Y238, T239, E120, and V102 (Table 8, Figure 5). The epitope of BM2 is in the vicinity of K225 (Table 8, Figure 5). Combined with structural analysis, it can be seen that the epitope of the M701 antibodies is completely different from the epitopes of BM1 and BM2 (Figure 5).

**Table 8. Binding of antigen mutants by M701 and control antibodies**

| Antigen mutation site | Mutant number | ELISA EC₅₀ (ELISA) | | | |
|---|---|---|---|---|---|
| | | M701-3966 | M701-4007 | BM1 | BM2 |
| WT | 3907 | 0.94 | 1.39 | 0.24 | 1.34 |
| R103A | 4057 | No binding | No binding | 0.23 | 1.34 |
| G124R | 4059 | No binding | No binding | No binding | 6.16 |
| M196A | 4067 | 1.62 | 1.94 | 0.33 | 2.37 |

| Antigen mutation site | Mutant number | BLI K_{D} (M) | | | |
|---|---|---|---|---|---|
| | | M701-3973 | | SA-BM2 | |
| WT | 3907 | 8.39E-09 | | 3.72E-09 | |
| S234A | 4133 | 7.82E-09 | | 2.82E-09 | |
| Y238A | 4134 | 1.21E-05 | | 5.64E-09 | |
| T239A | 4135 | 1.16E-08 | | 2.80E-09 | |
| K240A | 4136 | 8.15E-09 | | 2.74E-09 | |
| H118A | 4137 | 7.73E-09 | | 1.14E-08 | |
| E120A | 4138 | 1.73E-08 | | 3.43E-09 | |
| V 102A | 4139 | 5.85E-06 | | 3.43E-09 | |
| T105A | 4140 | 6.65E-09 | | 2.35E-09 | |

BLI technology was used to perform epitope binning experiments, and Anti-His probes were used to capture the antigen HuTL1A. Then the first antibody (BM1 or BM2) was added to bind to the antigen, and the antigen was saturated by the first antibody. Then the second antibody (one of the M701 antibodies and BM1 and BM2) was added to check its binding to the antigen and the interaction was analyzed.

The results showed that the antigens could still bind to the M701 antibodies after binding to the BM antibodies, indicating that the M701 antibodies had different epitopes from those of BM1 and BM2, and did not compete with them (Table 9). At the same time, the antigen could continue to bind to BM2 after binding to BM1, indicating that BM1 and BM2 do not compete with each other, which is consistent with the results of the alanine screening experiment.

**Table 9. Epitope binning of M701 antibodies and control antibodies**

| Antibody 1 | Antibody 2 | | | | | |
|---|---|---|---|---|---|---|
| | M701-3728 | M701-3878 | M701-3973 | M701-3990 | BM1 | BM2 |
| BM1 | No Competition | No Competition | No Competition | No Competition | Competition | No Competition |
| BM2 | No Competition | No Competition | No Competition | No Competition | No Competition | Competition |

### Example 8. Size evaluation of antigen-antibody complexes

TL1A is a trimeric antigen that usually binds to bivalent antibodies and cross-links to form a very large immune complex, which is likely more immunogenic, and is thus more prone to cause undesirable immunogenicity issues (*e*.*g*., ADA issues), which in turn leads to decreased drug exposure, decreased efficiency, and may even lead to safety issues. Thus, controlling the size of the antigen-antibody complex is key to developing antibodies against trimeric antigens like TL1A.

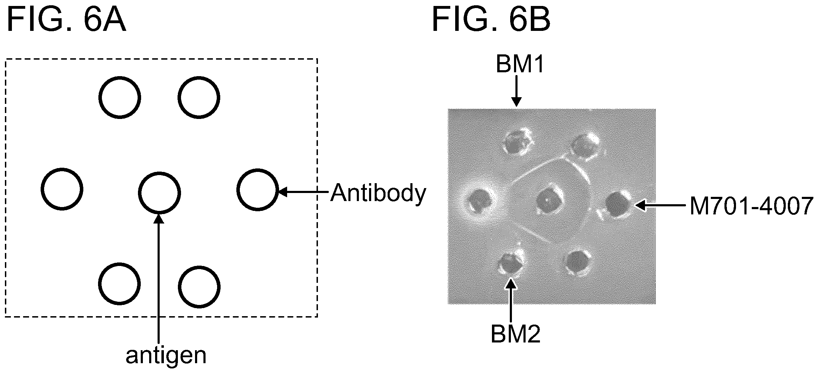

The Ouchterlony double immunodiffusion assay was used to detect the size of immune complexes formed by antibodies and antigens. 1.2% agarose was spreaded on the glass slide, and a mold was used to reserve 7 small holes of the same size. 10 µL of 120 µM antigen HuTLIA was placed in a middle well, and 10 µL of a M701 or a BM antibody was placed in two peripheral wells. They were placed in a 37°C incubator and incubated overnight, and the formation of a precipitation line was checked the next day. The formation of precipitation line indicates the formation of larger immune complexes. The results showed that the M701 antibodies did not form a white precipitation line with the antigen, and it was indicated that no large immune complex was formed; while the control antibodies BM1 and BM2 both formed obvious precipitation lines with the antigen (Figure 6), suggesting formation of larger immune complexes.

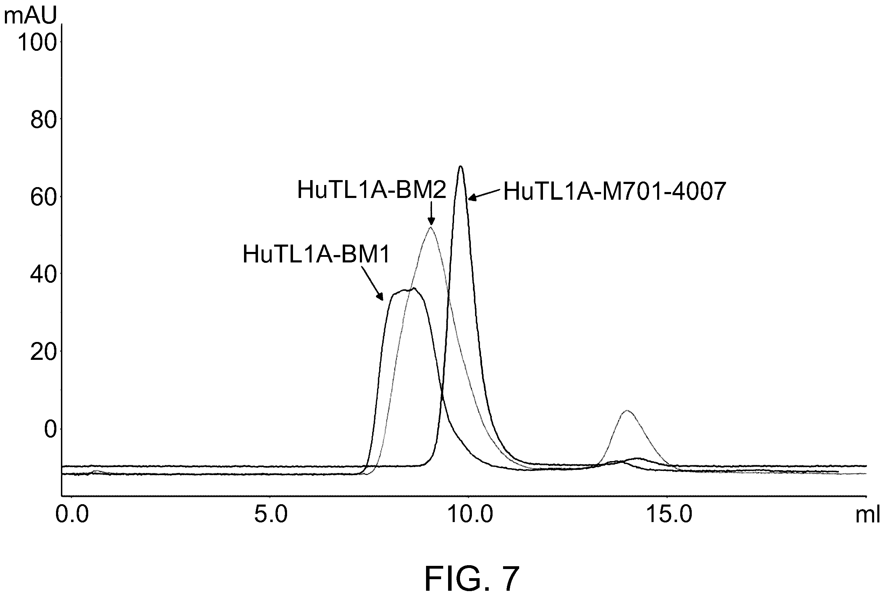

Dynamic light scattering (DLS) and size exclusion chromatography (SEC) were then used to confirm the size of the antigen-antibody complex. 60 µM antigen and antibody was mixed in equal moles, incubated at room temperature for 30 minutes, and then the molecular size was detected using UNCLE or SEC. The results showed that the DLS detected particle size of the complexes formed by the M701 antibodies and the TL1A antigen is approximately 18 nm, which is significantly smaller than the 86 nm complex of BM1 and the 33 nm complex of BM2 (Table 10); the elution volume of the M701 antibody-antigen complex on SEC was significantly larger than the elution volume of the control antibody-antigen complex (Figure 7), further confirming that the M701 antibodies and antigen formed smaller complex than the BM antibodies.

**Table 10. Particle size of the complex formed after M701 and BM antibodies bind to TL1A tested by DLS**

| Antigen-antibody complex | Ave Z-dia (nm) |
|---|---|
| HuTL1A+M701-3756 | 19.08 |
| HuTL1A+M701-3876 | 18.67 |
| HuTL1A+M701-3912 | 18.46 |
| HuTL1A+M701-3966 | 18.56 |
| HuTL1A+M701-4007 | 18.72 |
| HuTL1A+M701-4019 | 18.53 |
| HuTL1A+BM1 | 85.66 |
| HuTL1A+BM2 | 33.14 |

Finally, the particle size of the antigen-antibody complex was observed through negative staining electron microscopy experiments. The antigen and antibody were mixed according to a specific molar ratio (after mixing, a complex was formed, and there was no unbound antigen or antibody). The negative staining experiment is to adsorb protein particles onto a grid containing a hydrophilic membrane, and heavy metal salts with high electron density is used to stain biological samples spread on the grid. The macromolecular particles are only coated with a thin layer of heavy metal salts, while the background scattering of electrons is enhanced due to the deposition of dye solution. When observed through transmission electron microscopy, the sample structure is transparent and light-colored, and the background is gray or black, allowing preliminary evaluation of particle concentration, particle size, uniformity, aggregation state, etc. at lower resolution.

The protein samples were added to the negative staining grid which had been treated with glow discharge hydrophilization (Xinxing Bairui, Cat. No.: T10044), and were let stand for a period of time to allow the particles to adhere to the grid. Filter paper was used to absorb excess sample. The grid was then washed with deionized water to lessen the impact of salt ions in the sample buffer on subsequent experiments. The cleaned particles were then stained with a staining solution at room temperature. After staining, filter paper was used to touch the edge of the carrier grid to absorb excess liquid to allow the particles to dry naturally. Sample observation and data collection were completed on a Thermo Fisher Krios G4 300 kV transmission electron microscope. The electron detector used was a CETA direct electron counting detector. Data collection was completed using SerialEM software in super-resolution mode.

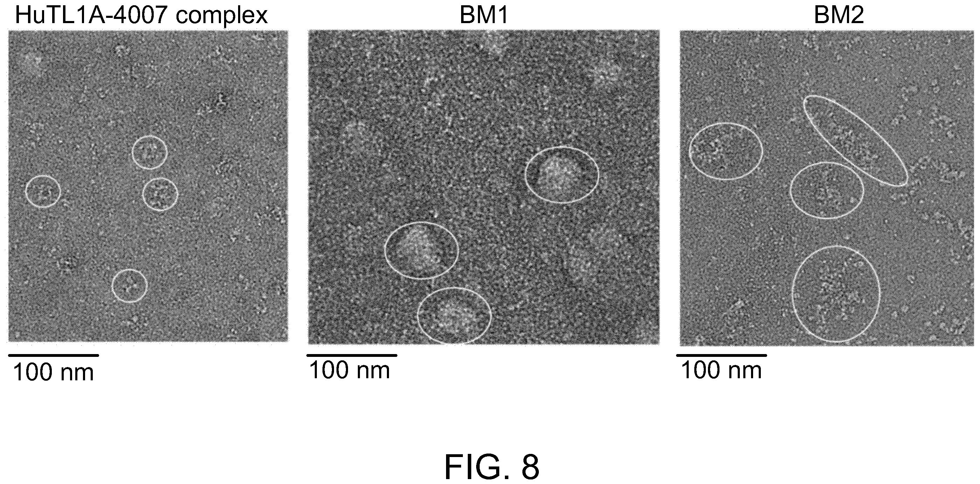

Based on negative staining electron microscopy experimental results, it was directly observed that the size of the antigen-antibody complex formed between the M701 antibodies and the TL1A antigen was much smaller than the size of the complexes formed between the control antibodies and the TL1A antigen. Dispersed small particles with regular shapes were formed. See the white circle in Figure 8 showsing a representative antigen-antibody complex. It can be seen that the complex formed between HuTLIA and M701-4007 was uniformly dispersed small particles, while the complexes formed between HuTLIA and control antibodies largely aggregated (Figure 8). Specifically, the HuTL1A-BM1 complex highly aggregated, and there was barely single particles that can be seen; and a large proportion of HuTL1A-BM2 complexes heavily aggregated, while some small aggregates also existed. The negative staining results were consistent with the SEC results (Figure 7). Specifically, Figure 7 shows that, although the main SEC peak of BM2 partially overlaped with the elution volume of the main peak of the M701 antibody, its peak distribution was significantly wider than that of the M701 antibody due to the size heterogeneity of its particle. The negative stain electron microscopy results were also consistent with that of DLS (Table 8).

### Example 9. Conformation of antigen-antibody complex

Through 2D Average analysis of negative staining electron microscopy images, the conformation of the complex formed by M701 antibodies and antigen was obtained. Different underfocus or radiation damage affect the results of 2D particle classification and averaging. Cistern software or cryosmart was used to correct the contrast transfer function (CTF) to reduce the above effects. A box that is 2-3 times larger than the longest particle diameter was used to automatically extract particles from the photo. Particles with poor quality, due to factors such as poor staining, too close proximity to other particles, and too close to photo edges were eliminated. The remaining particles were utilized in 2D classification using software such as Cistern or cryosmart. Through classification averaging, the background noise was filtered out, and the common features/conformations between particles were enhanced.

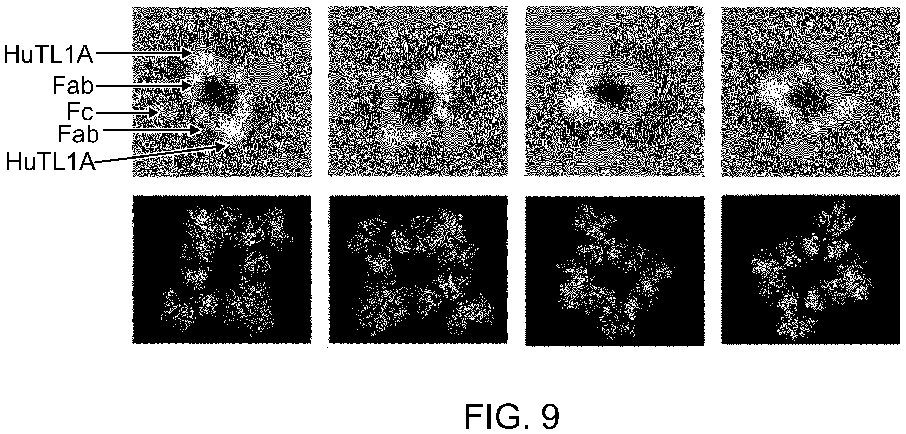

The 2D average analysis results showed that the M701 antibodies and the antigen form a fixed antigen-antibody complex with a stable structure in the form of 2 antibodies + 2 antigens (Figure 9). It is believed that the antigen-antibody complex in this conformation prevented the antigen-antibody complexes from forming larger cross-linked structures.

It is believed that the size of the antigen-antibody complex is related to immunogenicity, and that the valence of Fc in the antigen-antibody complex is also related to immunogenicity. An Fc value that is too high (such as ≥5) may lead to the activation of immune cells, especially antigen-presenting cells, thereby increasing ADA issues. The Fc valence of the M701 antibody-antigen complex was determined to be 2, while the Fc valence of the control antibody-antigen complex was believed to be much greater than 5 due to the cross-linked network structure formed. Thus, based on the size of the antibody-antigen complex or the valence of Fc, the M701 antibody potentially has lower immunogenicity risks than the control antibodies.

### Example 10. Formation mechanism of special conformation of antigen-antibody complex

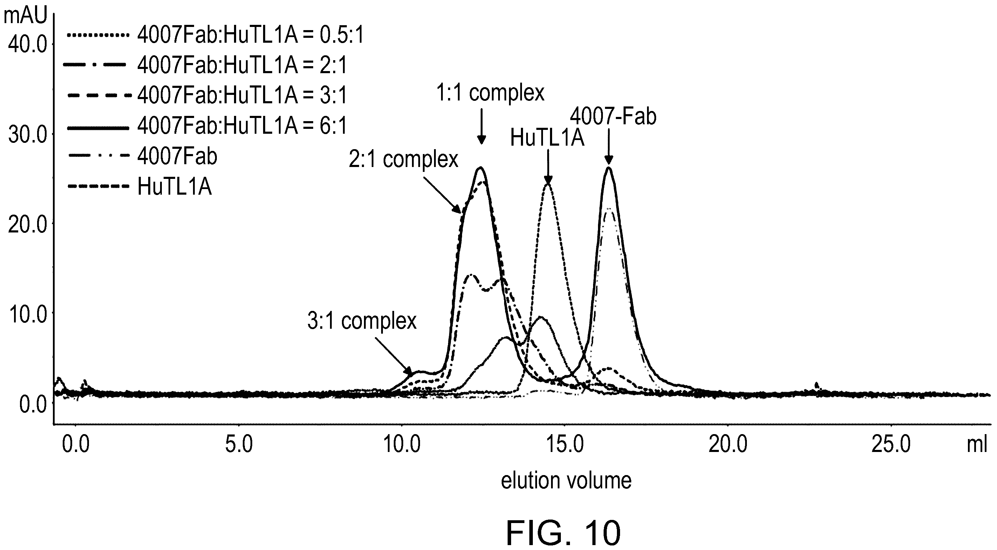

SEC analysis of M701 antibody Fab: antigen mixtures at different ratios was conducted. The results showed that in the presence of a significant excess of Fab, a trimeric TL1A antigen was still difficult to bind to three antibody Fabs (Figure 10). It is speculated that after the antibody binds to the first and second epitopes of the antigen, the conformation of the third epitope may change, so that the third epitope can no longer bind to the third Fab.

In the aforementioned epitope mapping experiment, the epitope region of M701 was determined. This region is located in the middle to lower section of the subunit interface and overlaps or partially overlaps with the binding region of DR3-TL1A. It is believed that when the M701 antibody binds to the first and second epitopes of TL1A, it directly blocks its binding to DR3, and the third epitope undergoes a conformational change and loses the ability to bind to DR3.

### Example 11. Reporter gene method to determine the neutralizing activity on soluble TL1A

A reporter gene method was used to determine the activity of TL1A antibodies in blocking the biological functions of soluble TL1A.

HEK293T/DR3-NFκB cells (which express DR3, and contain a SEAP reporter gene under the control of an NF-κB response element) were constructed through lentiviral transfection. When DR3 on HEK293T/DR3-NFκB cells is activated by binding to soluble TL1A, the activation of downstream NF-κB signaling is induced, and then the production of SEAP is in turn induced. Adding anti-TL1A antibodies to neutralize TL1A can reduce the production of SEAP. The amount of secreted SEAP can be detected by QUANTI-Blue reagent, which can reflect the extent of blocking TL1A/DR3 binding by anti-TL1A antibodies.

The specific method/assay was carried out as follows: HEK293T/DR3-NFκB cells were collected, resuspended in complete culture medium (RPMI 1640 containing 10% FBS) at 3E5/mL, and spreaded evenly into a 96-well plate at 100 µL/well, that is, 3E4 cells per well; then 50 µL/well of serially diluted anti-TL1A antibody and control antibody, as well as 50 µL/well of human TL1A were added; the 96-well plate was incubated in a 37°C 5% CO₂ incubator for 20-24 hours; the 96-well plate was then taken out and centrifuged at 300g for 5 minutes; 40 µL/well supernatant was then transferred to a new 96-well plate, before 160 µL/well QUANTI-BLUE reagent (InvivoGen) was added to the supernatant, and incubated for 20-30 minutes in a 37°C 5% CO₂ incubator; finally, OD655 was read on a microplate reader (MD, SpectraMax iD3).

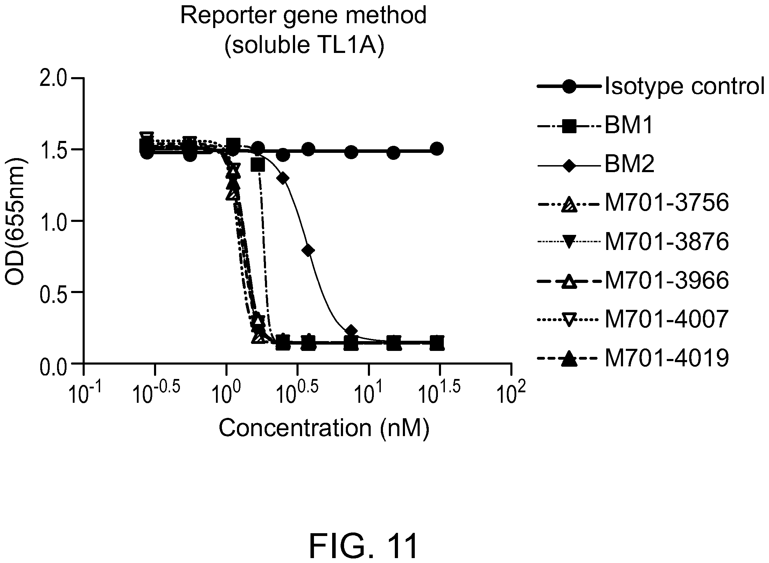

The results were shown in Figure 11. Although M701 antibodies 3756, 3876, 3966, 4007 and 4019 as well as the control antibodies BM1 and BM2 all significantly blocked the activation of downstream signals by soluble TL1A, the M701 antibodies have better TL1A neutralizing activity than BM1 antibody and BM2 antibody.

### Example 12. Reporter gene method to determine the neutralizing activity on membrane-bound TL1A

A reporter gene method was used to determine the activity of TL1A antibodies in blocking the biological functions of membrane-bound TL1A.

HEK293T/DR3-NFκB cells (which express DR3, and contain aSEAP reporter gene under the control of an NF-κB response element) and HEK293T/TL1A-M cells (which express membrane TL1A) were constructed through lentiviral transfection. Using these HEK293T/DR3-NFκB cells and HEK293T/TL1A-M cells, a reporter gene testing method for the neutralizing activity of membrane-bound TL1A was established.

The specific method/assay was carried out as follows: HEK293T/DR3-NFκB cells were collected, resuspended in complete culture medium (RPMI 1640 containing 10% FBS) at 3E5/mL, and spreaded evenly into a 96-well plate at 100 µL/well, that is, 3E4 cells per well; then 50 µL/well of serially diluted anti-TL1A antibodies and control antibodies and 50 µL/well of 293T/TL1A-M cells (cell density at 6E5/mL, that is, 3E4 cells per well) were added; the 96-well plate was incubated in a 37°C 5% CO₂ incubator for 20-24 hours; the 96-well plate was then taken out and centrifuged at 300g for 5 minutes; then 40 µL/well supernatant was transferred to a new 96-well plate, before 160 µL/well QUANTI-BLUE reagent (InvivoGen) was added to the supernatant, and incubated for 20-30 minutes in a 37°C 5% CO₂ incubator; finally, OD655 was read on a microplate reader (MD, SpectraMax iD3).

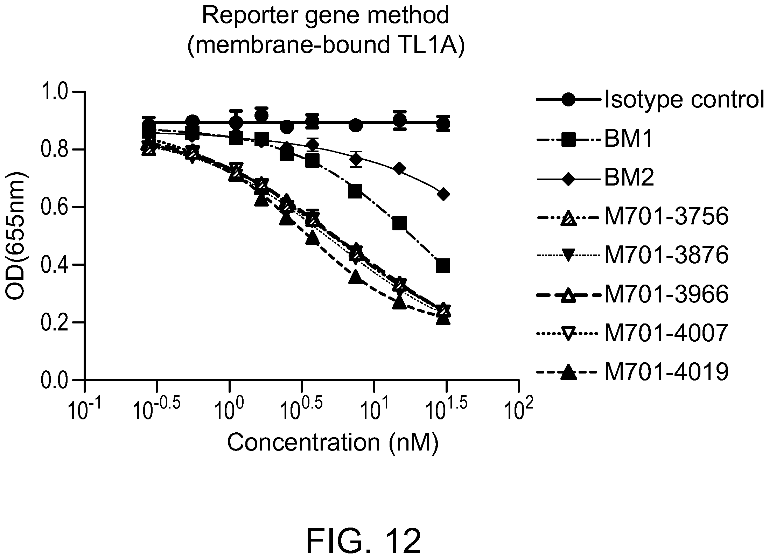

The results are shown in Figure 12. M701 antibodies 3756, 3876, 3966, 4007 and 4019 all significantly blocked the activation of downstream signals by membrane-bound TL1A, while the neutralizing activities of the control antibodies BM1 and BM2 were significantly weaker than those of the M701 antibodies.

### Example 13. Determination of the effect of TL1A antibodies on DcR3/TL1A interaction

TL1A has two receptors, namely DR3 and DcR3 (Decoy receptor 3). DcR3 lacks an intracellular signal transduction structure and functions as an endogenous TL1A inhibitor by competing with the DR3 receptor for binding to TL1A.

ELISA was used to detect the effects of M701 antibodies and control antibody on DcR3/TL1A interaction. The specific method was carried out as follows: DcR3 (AcroBiosystems, Cat. No.: TNB-H5255) was coated in a microplate with coating solution, 1 µg/mL, 100 µL per well, and incubated overnight at 4°C. The microplate was then washed 5 times with PBST, blocked with 200 µL/well of PBST containing 1% BSA, and incubated at room temperature for 1 hour. The microplate was then washed 5 times with PBST. M701 sample and control sample were gradient diluted in PBST containing 1% BSA, before Biotin-TL1A (FutureGen) were mixed in at 1:1. 100 µL of the mixture was then added to the enzyme microplate, and the microplate was incubated at room temperature for 1 hour. After washing 5 times with PBST, 100 µL of Streptavidin-HRP (Biolegend, Cat. No. 405210) diluted in PBST containing 1% BSA was added to each well, and the microplate was further incubated at room temperature for 30 minutes. Colorimetric substrate TMB (BD, Cat. No.: 555214) was then added at 100 µL per well, to allow color development at room temperature for 6 minutes. Finally, 1M sulfuric acid was added to each well to terminate the reaction. OD450nm was read on a microplate reader, and the results were analyzed, including fitting the binding curve with 4 parameters.

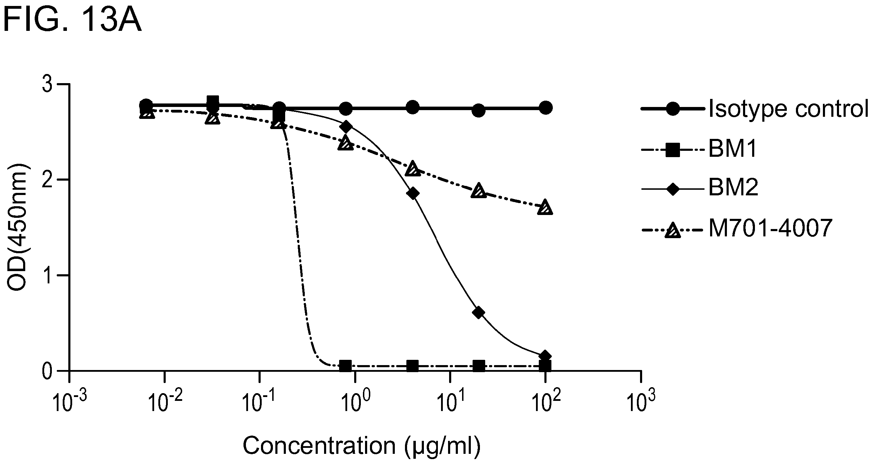

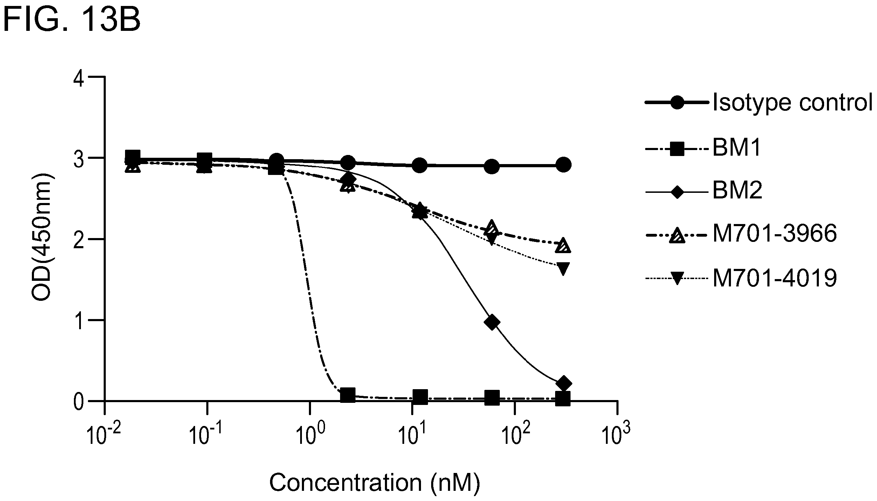

The results were shown in Figures 13A and 13B. The control antibody BM1 showed very strong DcR3/TL1A blocking activity. The control antibody BM2 also completely inhibited the DcR3/TL1A interaction but exhibited weaker activity than BM1. Meanwhile, the M701 antibodies 3966, 4007 and 4019 only exhibited slight interference with the DcR3/TL1A interaction. The data demonstrates that the M701 antibodies neutralized the biological function of TL1A while retaining the biological function of the endogenous TL1A inhibitor DcR3.

### Example 14. TL1A antibodies inhibit the activation of T cells by exogenous TL1A

Obtaining human T cells: human PBMC cells were recovered and collected, and human T cells were isolated according to the instructions of the Pan T cell isolation kit (Miltenyi Biotech, Cat. No. 130-096-535). Briefly, the PBMC cells were firstrwashed once with PBS, before resuspending the cells at 10⁷ cells per 40 µL separation buffer (PBS containing 2mM EDTA, 0.5% BSA, pH=7.2) (the following dosage is based on 10⁷ cells). 10 µL Pan T cell Biotin Antibody Cocktail was then added for incubation at 4°C for 5 minutes. Then 30 µL separation buffer and 20 µL Pan T cell MicroBead Cocktail were added for incubation at 4°C for 10 minutes. The T cells were obtained by passing through the MACS separation column.

T cells were resuspended at 2E6/mL in complete medium (RPMI 1640 with 10% FBS) containing 0.5 ng/mL IL-12 (Peprtech, 200-12) and 5 ng/mL IL-18 (MBL, B001-5), before addingto a 96-well plate at 100 µL/well, that is, 2e5/well; anti-TL1A antibody and control antibody serially diluted in complete medium were then added at 50 µL/well, followed by addition of human TL1A diluted in complete medium at 50 µL/well; the culture plate was then placed in a 37°C carbon dioxide incubator and incubated for 3 days. After the incubation, the supernatant was removed from the well, and the cytokine IFN-γ (Biolegend, Cat. No. 430101) was detected according to the kit instruction manual.

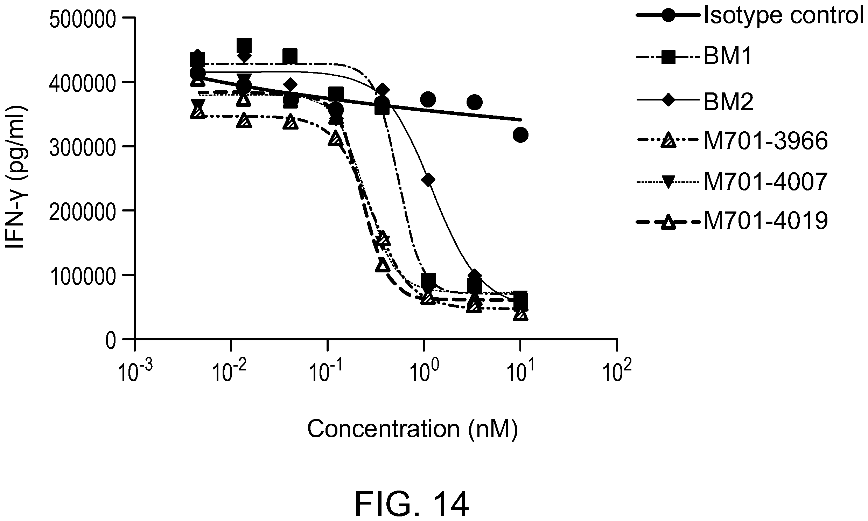

The results were shown in Figure 14. M701 antibodies 3966, 4007 and 4019 and control antibodies BM1 and BM2 all significantly inhibited activation of T cells by exogenous TL1A. However, M701 antibodies exhibited better biological activity than BM1 antibody and BM2 antibody.

### Example 15. TL1A antibodies inhibit the activation of immune cells by endogenous TL1A

Stimulating PBMC with immune complexes (IC) to induce endogenous TL1A and co-stimulates immune cells together with IL-12 and IL-18 to produce IFN-γ.

TL1A antibodies can inhibit the IFN-γ secretion of immune cells by inhibiting TL1A function. The secretion level of IFN-γ was detected in this example by ELISA in order to assess the ability of the M701 antibodies and control antibodies to neutralize endogenous TL1A. The specific method was carried out as follows: a 96-well flat-bottom plate (Corning, Cat. No. 3599) was coated with Flag-IgG fusion protein (FutureGen) in PBS at 25 µg/mL, 50 µL per well, and was incubated overnight at 4°C. After washing three times with PBS, anti-Flag antibody (FutureGen) was diluted with PBS, and added at a concentration of 25 µg/mL, 50 µL per well to the 96-well plate. After incubation at room temperature for 1 h, the cells were washed three times with PBS. PBMC cells were recovered, resuspended in complete culture medium (RPMI 1640 containing 10% FBS), adjusted to a density of 2×10⁶/mL, 100 µL per well, and were added with IL-12 at a final concentration of 0.5 ng/mL (Peprotech, Cat. No. 200-12) and IL-18 at 5 ng/mL (MBL, Cat. No. B001-5). The PBMC cells were then inoculated in the above-mentioned 96-well flat-bottom plate. Then the M701 antibodies and control antibody serially diluted in complete medium were added to the 96-well flat-bottom plate at 100 µL per well. The 96-well flat-bottom plate was then incubated in a 37°C, 5% CO₂ incubator for 3 days. After the incubation, the supernatant was removed from the wells, and the cytokine IFN-γ was detected according to the kit instruction manual (Biolegend, Cat. No. 430101).

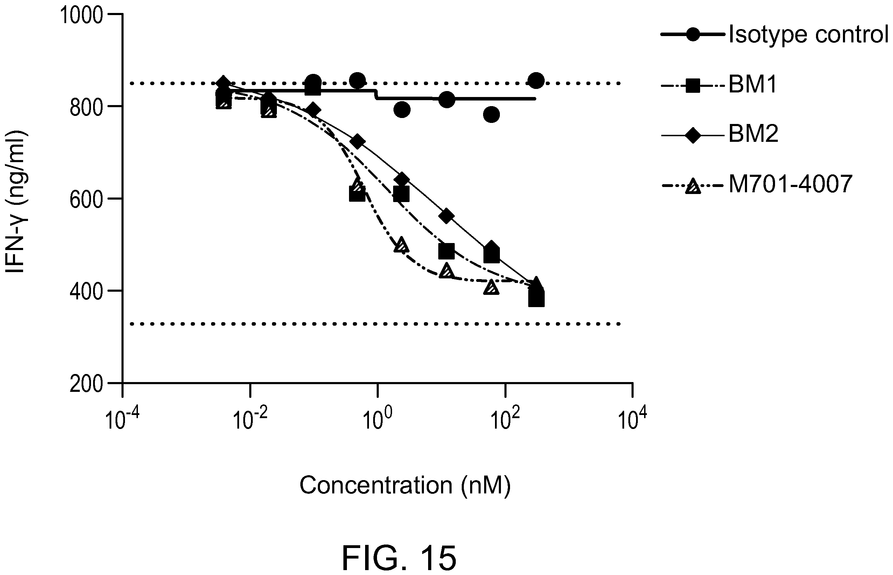

The results were shown in Figure 15. M701 antibody 4007 and control antibodies BM1 and BM2 all significantly inhibited the activation of immune cells by endogenous TL1A. However, the M701 antibodies have better biological activity than both the BM1 antibody and the BM2 antibody.

### Example 16. Pharmacokinetic study of TL1A antibodies in SCID mice

This example provides data to compare serum concentrationchange over time of the antibodies in SCID mice after a single administration of the M701 antibodies or control TL1A antibodies, with or without administration of exogenous human TL1A, as well as the serum concentration change over time of exogenous TL1A.

Female SCID mice (Beijing Vital River Laboratory Animal Technology Co., Ltd.) aged 6-8 weeks were randomly divided into 4 groups (G1, G2, G3, and G4), with 10 mice in each group. Mice in Groups G1, G2, G3, and G4 were each intravenously injected with 15 nmol/kg of the BM2 antibody, the M701 antibody, the BM2 antibody, and the M701 antibody. Groups G3 and G4 were also given 10 nmol/kg of human TL1A antigen by intravenous injection 1 hour after antibody infusion. Whole blood was collected and prepared as serum samples, before antibody administration, and at 30 minutes, 24 hours, 72 hours, 120 hours, 168 hours, 240 hours, 336 hours, and 480 hours after administration of human TL1A antigen (each time point in each group 5 mice, alternate blood collection). M701 antibodies or control TL1A antibodies and human TL1A antigen in serum were measured using ELISA. Pharmacokinetic parameters were calculated using the noncompartmental model of Phoenix WinNonlin 8.2 software.

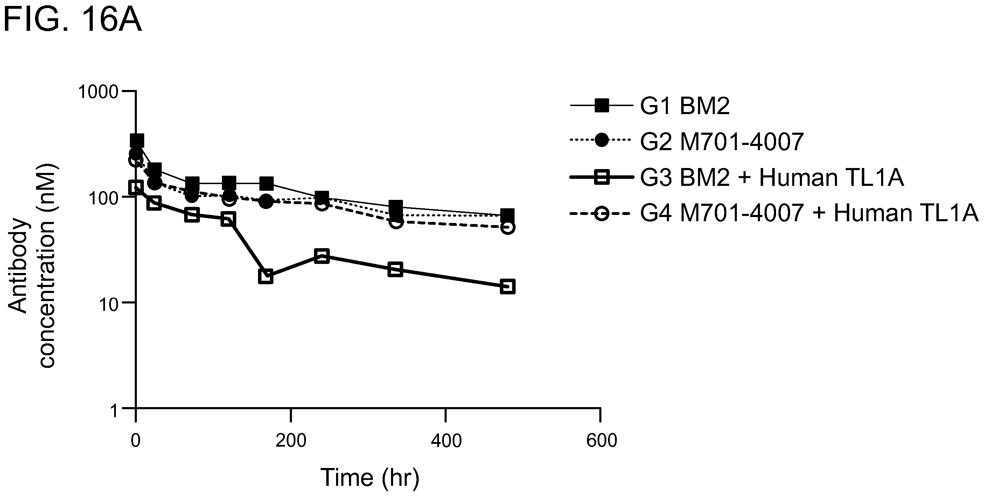

The serum concentration time course of the antibodies were shown in Figure 16A. Under the condition that no exogenous human TL1A antigen was administered, the BM2 antibody and the M701-4007 antibody exhibited similar pharmacokinetic characteristics (neither the BM2 antibody nor the M701-4007 antibody could bind to mouse TL1A); meanwhile, under the condition that exogenous human TL1A antigen was administered, drug exposure of the BM2 antibody significantly decreased, indicating that its clearance was accelerated after binding to the human TLIA antigen, while drug exposure of the M701-4007 antibody was basically unaffected ( Table 11). This may be because the BM2 antibody formed a large complex with human TL1A, while the M701-4007 antibody only formed a small complex with human TL1A in the form of 2+2 (see above). Large immune complexes are more easily recognized by the body, thereby speeding up complex clearance and possibly causing immunogenicity issues (this example using SCID mice without interference of ADA).

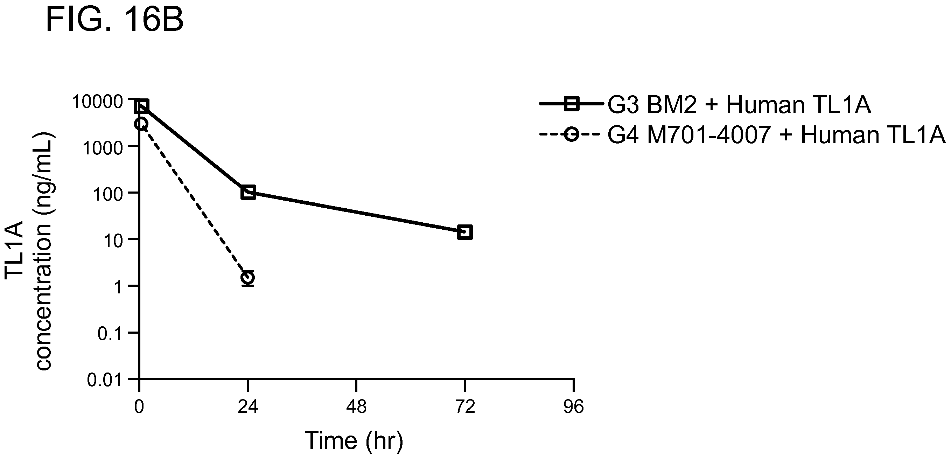

The serum concentration change over time of human TL1A were shown in Figure 16B. In the group given the BM2 antibody, human TL1A maintained a higher concentration for a long period of time, while in the group given the M701-4007 antibody, human TL1A was cleared significantly faster. This may be related to the pH-dependent binding of M701-4007 antibody to human TL1A. The pH-dependent antigen-binding properties enable the M701-4007 antibody to bind to the TL1A antigen with high affinity in serum. Once the antigen-antibody complex is endocytosed into the cell and enters endosomes with a pH lower than 6.0, antibody affinity for antigen decreases significantly. As a result, TL1A is quickly released and enters the lysosome for degradation, while the M701-4007 antibody is salvaged by FcRn and re-enters the blood circulation to play its role. In contrast, the BM2 antibody maintains its high binding affinity with TL1A even in endosomes. Thus, TL1A cannot be quickly released and degraded. Instead, it may be salvaged by FcRn along with the BM2 antibody, and may accumulate in the blood. The pH-dependent antigen-binding characteristics can accelerate the clearance of TL1A in lysosomes, thereby reducing the level of TL1A in the body and increasing the effective exposure of antibodies.

**Table 11. Comparison of drug exposure of M701 and BM antibodies in SCID mice**

| Administration group | AUC₀₋₄₈₀ₕᵣ (nM.hr) | AUC ratio |
|---|---|---|
| G1 BM2 Antibody | 54,856 | |
| G2 M701-4007 Antibody | 45,355 | |
| G3 BM2 Antibody + Human TL1A | 17,905 | G3/G1: 33% |
| G4 M701-4007 Antibody + Human TL1A | 41,615 | G4/G2: 92% |

### Example 17. Pharmacokinetic study of TLLA antibody in rhesus monkeys

The M701 antibody is an Fc-engineered IgG1 antibody that is expected to have slower elimination and longer half-life. This example provides data to compare serum drug concentration change over time in rhesus monkeys after a single administration of M701 antibodies or control TL1A antibodies.

Animal experiments were completed at Pharmaron Chemical (Beijing) Biotechnology Co., Ltd. Twelve male rhesus monkeys were randomly divided into 4 groups (Groups G1, G2, G3, and G4), with 3 animals in each group. Monkeys in Groups G1, G2, G3, and G4 were given intravenous infusion of 5 mg/kg of the BM1 antibody, the BM2 antibody, the M701-3966 antibody, and the M701- 4019 antibody, respectively, over an infusion time of 30 minutes, ata dosage volume of 10 mL/kg. Whole blood was collected and prepared as serum samples from each animal, before antibody administration, and at 30 minutes (*i.e.* immediately after the end of infusion), 2 hours, 8 hours, 24 hours, 72 hours, 120 hours, 168 hours, 240 hours, 336 hours, 504 hours, 672 hours, and 840 hours after the start of antibody infusion. M701 antibodies or control TL1A antibodies in serum were measured using ELISA. Pharmacokinetic parameters were calculated using the noncompartmental model of Phoenix WinNonlin 8.2 software.

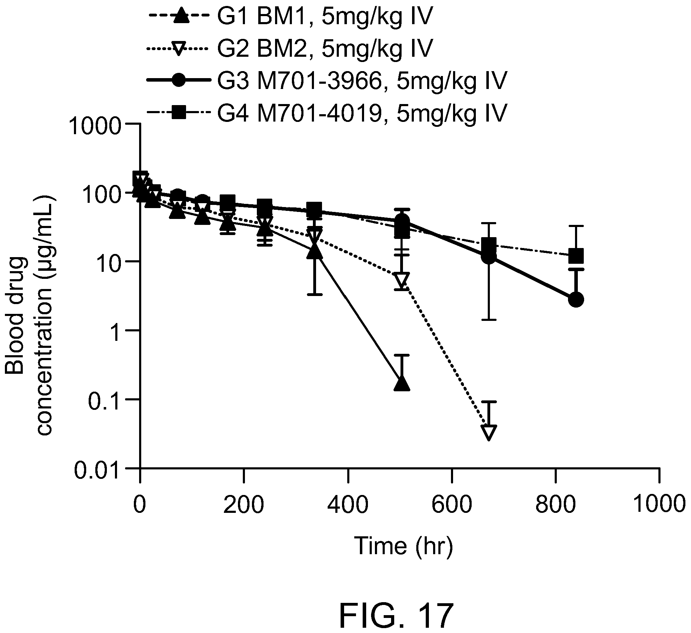

The experimental results, after the rhesus monkeys were given a single intravenous infusion of 5 mg/kg BM1 antibody, BM2 antibody, M701-3966 antibody, or M701-4019 antibody, were shown in Figure 17. Compared to BM1 and BM2 antibodies, M701-3966 and 4019 were eliminated more slowly and had a longer half-life in rhesus monkeys.

## Claims

1. An isolated antibody that specifically binds to TL1A, wherein the isolated antibody comprises an immunoglobulin heavy chain (HC) comprising a heavy chain variable region (VH) comprising a heavy chain CDR1, a heavy chain CDR2, and a heavy chain CDR3 (VH CDRs 1-3), and an immunoglobulin light chain (LC) comprising a variable region (VL) comprising a light chain CDR1, a light chain CDR2, and a light chain CDR3 (VL CDRs 1-3), wherein the VH CDRs 1-3 have the amino acid sequences as set forth in SEQ ID NO: 40-42, respectively, and the VL CDRs 1-3 have the amino acid sequences as set forth in SEQ ID NO: 37-39, respectively.

2. The isolated antibody of claim 1, wherein the VH comprises an amino acid sequence as set forth in SEQ ID NO: 10, and the VL comprises an amino acid sequence as set forth in SEQ ID NO: 9.

3. The isolated antibody of claim 1 or 2, wherein the heavy chain comprises an amino acid sequence set forth in SEQ ID NO: 58, and the light chain comprises an amino acid sequence set forth in SEQ ID NO: 57.

4. The isolated antibody of any one of claims 1-3, wherein the isolated antibody comprises an Fc region.

5. A nucleic acid comprising a nucleotide sequence encoding the isolated antibody according to any one of claims 1-4.

6. The nucleic acid of claim 5, comprising the nucleotide sequence encoding the isolated antibody according to claim 3.

7. A vector comprising the nucleic acid according to claim 5 or 6.

8. The vector of claim 7, comprising the nucleic acid according to claim 6.

9. A host cell comprising the nucleic acid according to claim 5 or 6, or the vector according to claim 7 or 8.

10. The host cell of claim 9, comprising the nucleic acid according to claim 6, or the vector according to claim 8.

11. A method for preparing the isolated antibody according to any one of claims 1-4, wherein the method comprises:
a) culturing the host cell according to claim 9 or 10 under conditions suitable for expression of the isolated antibody; and
b) isolating the isolated antibody from the culture and/or culture supernatant of the host cell.

12. A pharmaceutical composition comprising the isolated antibody according to any one of claims 1-4, and optionally a pharmaceutically acceptable carrier or excipient.

13. The pharmaceutical composition of claim 12, comprising the isolated antibody according to claim 3, and a pharmaceutically acceptable carrier or excipient.

14. A method of treating a disease in a subject, comprising administering to the subject an effective amount of the isolated antibody according to any one of claims 1-4.

15. The method of claim 14, comprising administering to the subject the effective amount of the isolated antibody according to claim 3, and a pharmaceutically acceptable carrier or excipient.

16. Use of the isolated antibody according to any one of claims 1-4 in the manufacture of a medicament for treating a disease in a subject.

17. The use of claim 16, wherein the isolated antibody is the isolated antibody according to claim 3.
